# EUROPEAN PATENT SPECIFICATION

(11) **EP 1 362 114 B1**
(45) Date of publication and mention of the grant of the patent: **09.05.2012**
(21) Application number: 02710733.3
(22) Date of filing: 07.02.2002
(51) Int. Cl.: C12N 15/90

(54) **REPLICATIVE IN VIVO GENE TARGETING**
REPLIKATIVES IN VIVO GEN-TARGETING
CIBLAGE GENIQUE IN VIVO REPLICATIF

(30) Priority: 08.02.2001 CA 2332186; 27.02.2001 US 271473 P
(43) Date of publication of application: 19.11.2003
(73) Proprietor: HER MAJESTY THE QUEEN IN RIGHT OF CANADA, as represented by the Minister of Agriculture and Agri-Food, Saskatoon, SK S7N 0X2 (CA)
(72) Inventor: ROZWADOWSKI, Kevin, L., Saskatoon, Saskatchewan S7N 3R1 (CA); LYDIATE, Derek, J., Saskatoon, Saskatchewan S7N 1E6 (CA)
(74) Representative: Michalík, Andrej
(86) International application number: PCT/CA2002/000136
(87) International publication number: WO 2002/062986

(56) References cited:
- US-A- 5 780 296
- SHCHERBAKOVA O G ET AL: "OVEREXPRESSION OF BACTERIAL RECA PROTEIN STIMULATES HOMOLOGOUS RECOMBINATION IN SOMATIC MAMMALIAN CELLS" MUTATION RESEARCH, AMSTERDAM, NL, vol. 459, no. 1, 16 February 2000 (2000-02-16), pages 65-71, XP001004532 ISSN: 0027-5107 cited in the application
- ABUIN A. AND BRADLEY A.: "Recycling selectable markers in mouse embryonic stem cells." MOLECULAR AND CELLULAR BIOLOGY, vol. 16, no. 4, April 1996 (1996-04), pages 1851-1856, XP002215308
- STORCK T ET AL: "Rapid construction in yeast of complex targeting vectors for gene manipulation in the mouse" NUCLEIC ACIDS RESEARCH, OXFORD UNIVERSITY PRESS, SURREY, GB, vol. 22, no. 24, 25 November 1996 (1996-11-25), pages 4594-4596, XP002076777 ISSN: 0305-1048
- YANEZ R J ET AL: "GENE TARGETING IS ENHANCED IN HUMAN CELLS OVEREXPRESSING HRAD51" GENE THERAPY, MACMILLAN PRESS LTD., BASINGSTOKE, GB, vol. 6, no. 7, July 1999 (1999-07), pages 1282-1290, XP001057105 ISSN: 0969-7128
- KHREBTUKOVA I. ET AL: "Utilization of microhomologous recombination in yeast to generate targeting constructs for mammalian genes" MUTATION RESEARCH, AMSTERDAM, NL, vol. 401, 1998, pages 11-25, XP002076783 ISSN: 0027-5107
- LANZOV V A: "GENE TARGETING FOR GENE THERAPY: PROSPECTS" MOLECULAR GENETICS AND METABOLISM, ACADEMIC PRESS, SAN DIEGO, CA, US, vol. 68, no. 2, October 1999 (1999-10), pages 276-282, XP000872438 ISSN: 1096-7192
- CHAI SUNGHEE ET AL: "The small subunit of the terminase enzyme of Bacillus subtilis bacteriophage SPP1 forms a specialized nucleoprotein complex with the packaging initiation region." JOURNAL OF MOLECULAR BIOLOGY, vol. 252, no. 4, 1995, pages 386-398, XP002215309 ISSN: 0022-2836

## Description

### FIELD OF THE INVENTION

The invention is in the field of recombinant nucleic acid technology, particularly methods for targeted gene modification by nucleic acid recombination and/or repair using various nucleic acid replication systems.

### BACKGROUND OF THE INVENTION

Gene targeting generally refers to the directed alteration of a specific DNA sequence in its genomic locus *in vivo*. This may involve the transfer of genetic information from a nucleic acid molecule, which may be referred to as a gene targeting substrate, to a specific locus (i.e. target) in the host cell genome. In current methods, the gene targeting substrate usually exists as an extrachromosomal nucleic acid molecule. The target locus may for example be present in the host cell's nuclear chromosomes or organellar chromosomes (e.g. mitochondria or plastids) or a cellular episome. The gene targeting substrate typically encodes sequences homologous to the target locus. However, the sequence of the gene targeting substrate is modified to encode changed genetic information, vis-a-vis the target genetic locus, through the insertion or deletion of one or more base pairs or by the substitution of one or more bases for other types of bases. As a result, the gene targeting substrate may encode, for example, a different gene product than the target locus or a nucleic acid sequence which is non-functional or functions differently than the target locus.

The process of gene targeting may involve the action of host nucleic acid recombination and/or repair functions [1;2]. The homology between the target locus and the gene targeting substrate, in combination with host cell functions, is thought to facilitate the process of the gene targeting substrate 'scanning' the host genome to find and associate with the target locus. Host nucleic acid recombination and/or repair functions may then act to transfer genetic information from the gene targeting substrate to the target locus by the processes of homologous recombination or gene conversion or nucleic acid repair. In this manner, the novel sequence of the gene targeting substrate is transferred into the host genome at the targeted locus, which may result in loss of the wild-type genetic information at this locus. The modified target locus may now be stably inherited through cell divisions and, if present in germ cells and gametes, to subsequent progeny resulting from sexual reproduction.

This ability to perform precise genetic modifications of a host cell's genome at defined loci is an extremely powerful technology for basic and applied biological research. A principal advantage of gene targeting over conventional transformation technologies, which results in integration of the exogenously supplied DNA cassettes at random sites in the host genome [3;4], is the maintenance of appropriate chromosomal context for the modified gene. In contrast, transformational integration of DNA cassettes into random sites of the host genome can have large negative effects on the host cell, for example by causing insertional inactivation of the resident gene where the DNA cassette integrates. In addition, integration at random sites can affect expression of the introduced gene encoded by the cassette [5]. Such 'position effects' may result from epigenetic control of gene expression relating to the regulation of chromatin conformation [6]. Thus transgenes which integrate at random sites in the genome may not be expressed in the correct fashion to accurately reflect the biological effect of the gene under basic study, or provide the desired phenotype in a biotechnology application [6]. Targeting of a transgene to its correct native site in the host genome may help to ensure correct regulation of its expression.

Gene targeting may enable the accurate analysis of the phenotypic effects of modified genes by simultaneously replacing the endogenous gene copy. In contrast, placement of a transgene encoding a modified version of an endogenous gene at random sites in the genome may not enable accurate analysis of the effect of this transgene because the endogenous gene copy is still functioning. Expression of the endogenous gene copy may compensate for or impair the action of the gene product encoded by the transgene. Through gene targeting, the endogenous gene copy may be replaced by the introduced modified gene. As a result, the endogenous gene copy will not be able to interfere with the action of the introduced modified gene and an accurate interpretation of the biological effects of the modified gene may be possible. This ability is very important for accurate assessment of gene function in basic studies, and is very important for biotechnology applications aimed at modifying the physiological, biochemical or developmental paths and responses of cells and organisms.

Through gene targeting a non-exclusive list of possible modifications or combinations of modifications to the host genome includes:
1. Gene replacement and gene addition: by replacing the targeted chromosomal gene or genes, or promoter or promoters, or portions of the aforementioned, with another gene or genes, or promoter or promoters, or portions of the aforementioned; or adding a gene or genes and regulatory components, or portions thereof, at a targeted chromosomal locus adjacent to resident endogenous loci.
2. Gene inactivation and gene deletion: Inactivating a targeted chromosomal gene through disruption of its functional transcription or translation by changing the sequence composition or by insertion or deletion of one or more base pairs.
   Deleting the coding region or regulatory components, or portions thereof, of a targeted chromosomal gene or genes.
   Using gene targeting, an absolute inactivation of specified target genes may be possible by, for example, creating insertion, deletion or substitution mutations in the target genes. Thus the phenotypic effects of the gene may be assessed by studying the engineered null-mutant. This null-mutant may also be genetically stable in subsequent generations ensuring the continued propagation of this line maintaining the same engineered phenotype. The modified line may also be isogenic to the original cell line or organism from which it is derived thus enabling reliable and accurate comparisons between the modified and original lines so that the effects of the modification may be accurately determined. Targeted gene inactivation may therefore have advantages over conventional means of gene silencing, such as antisense RNA and cosuppression, which may not provide absolute inactivation of the target gene and/or may not cause a stable and consistent level of inactivation through generations [8;9].
3. Allele modification: Changing the sequence of a targeted chromosomal gene to create a new allele which encodes a protein with a changed amino acid composition (i.e. protein engineering), or which has modified translatability or stability of the transcript.

Gene targeting has been demonstrated in several species including lower eukaryotes [10-12], invertebrate animals [13;14], mammals [15-19], lower plants [20] and higher plants [21-25]. Gene targeting substrates include single-stranded DNA (ssDNA) [11;24-27], double-stranded DNA (dsDNA) [10;15-18;27], or hybrid molecules with RNA and DNA constituents [21-23;28-30]. For some prior DNA-based gene targeting substrates, the amount of homology to the target locus present in the gene targeting substrate has varied from 10's of basepairs (bp) [12] to 10's of kilobasepairs (kb) [31], depending upon the nature of the target locus and the type of host cell or species and the efficiency of nucleic acid recombination and repair functions in that host cell or species. For RNA/DNA hybrid gene targeting substrates, the homology in some cases has been 10's of basepairs [21-23;28-30].

Successful gene targeting has been achieved by treatment of cultured cells [10,15-19;29], tissues [21-25;28] or organisms [13] with gene targeting substrate. This has resulted in modified target loci which are stable through cell divisions. To obtain modified target loci stably transmissible through sexual reproduction in mammals, specialized procedures employing specific embryonic stem cell lines may be employed [15;17]. In other animal systems, gene targeting substrates may be injected into gonads [13], or gene targeting substrate may be engineered to be present in the cells at early developmental stages to ensure modification of germ line cells [14]. Conversely, in some plants the totipotency of all cells may enable nearly any modified cell line to be regenerated into intact plants capable of transmitting the modified locus to progeny.

Application of gene targeting, especially in plants and mammals, may be inhibited by several limitations in conventional technology, which may be technically demanding, rely on tedious and expensive in vitro procedures, or successful only in specialized cell lines. These limitations may be compounded by a low frequency of gene targeting events [2;21-25;30] which may not be efficiently identifiable [2,6]. In some applications, only target loci which when modified result in selectable or easily screenable phenotypes may be employed, so that the rare gene targeting events may be identified.

Conventional strategies may rely on incorporation of a selectable marker at the target locus [15;17;24;25] resulting in insertional-inactivation mutants by interruption of the target gene with the selectable marker, an approach that may not enable more subtle modifications such as single base-pair changes. Current selection and enrichment procedures may also be ineffective if they select false-positives with high frequency [35].

A principal factor affecting the frequency of gene targeting with some conventional approaches may be the mechanism of delivering gene targeting substrate to the host cells. Current procedures may produce gene targeting substrate exogenously and may then rely on various means to get the gene targeting substrate into the host cell and nucleus, including chemical treatments [10;11;28;30;36-38], physical treatments [13;16;17;21-23;39-42], or biological vehicles [24;25;43].

Systems for production of dsDNA gene targeting substrates *in vivo* have been reported in yeast [44] and *Drosophila melanogaster* [14], in which a gene targeting cassette may be activated by an endonuclease. The action of the endonuclease in such systems appears to terminally modify the cassette so that the gene targeting cassette is not regenerated.

### SUMMARY OF THE INVENTION

In some embodiments, the invention provides methods using gene targeting systems that renew or regenerate a gene targeting cassette to enable repeated cycles of gene targeting substrate production as set out in the appended claims. Gene targeting cassettes may for example be regenerated by replication of the gene targeting substrate. In some embodiments, successive rounds of gene targeting cassette replication may allow the accumulation of multiple molecules of gene targeting substrate per cell or nucleus, so that the presence of more gene targeting substrate may promote the occurrence of gene targeting.

In alternative embodiments, inducible gene targeting systems of the invention may be used for production of gene targeting substrate at multiple time points, such as alternative (or multiple) points in a cell cycle, or in the life cycle of a cell, or in the development of an organism. The methods of the invention may therefore be adapted so that the gene targeting substrate is made available at a particular physiological or developmental stage, such as when gene targeting can occur at a desired frequency.

In some embodiments, the invention produces single-strand breaks in the host genome at replication primer recognition sequences flanking the gene targeting cassette, avoiding double-strand breaks that may result in deletion, rearrangement or mutation of genetic information and lead to cell growth inhibition or lethality [45;46].

In one aspect, the invention provides a method for integrating into a genome of a host, or a progenitor of the host, or into an ancestral genome of the host a gene targeting cassette comprised of recombinant nucleic acid sequences, such as DNA sequences, In alternative embodiments, the gene targeting cassette may be encoded on an extrachromosomal element present in a host cell or a progenitor of the host, or an ancestor of a host cell. The gene targeting cassette when integrated in the host genome or when encoded by an extrachromosomal element may comprise:
a) a replication initiator sequence recognized in the host, directly or indirectly, by one or more replication factor(s), such as DNA or RNA or protein molecules participating in the synthesis or action of a primer, so that the replication factor(s) mediate(s) nucleic acid replication in the host initiated at the replication initiator sequence;
b) a reproducible sequence operably linked to the replication initiator sequence so that nucleic acid replication initiated at the replication initiator sequence replicates the reproducible sequence creating a copy of at least one strand of the reproducible sequence, or portion thereof. The reproducible sequence may be operably linked to a replication terminator sequence, in the cassette or in the genome of the host, to terminate nucleic acid replication initiated at the replication initiator sequence in the host, to release a copy of at least one strand of the reproducible sequence, or a portion thereof.

Nucleic acid replication mediated by the replication initiator sequence and terminated at the replication terminator sequence, wherein at least some portion of the cassette has been replicated, may result in the regeneration of the gene targeting cassette, so that it is adapted for subsequent rounds of nucleic acid replication to produce multiple copies of at least some portion of the reproducible sequence (to act as a gene targeting substrate). At least one of the copies of the reproducible sequence, or a portion thereof, may then interact with a target sequence in the genome of the host to modify the target sequence to produce a heritable change, for example by the processes of homologous recombination, or gene conversion or nucleic acid repair. A portion of the reproducible sequence may have a high degree of identity to a portion of the target sequence, such that the sequence is sufficiently identical to facilitate homologous pairing with the target sequence. The relevant portion of the reproducible sequence may in some embodiments be 5, 10, 15, 20, 25 or more nucleotides in length, and the identity between the portions of the reproducible and target sequences may for example be 50%-100%, more than 60%, 70%, 80%, 90% or 95%. In some embodiments, the degree of homology and the length of the relevant portion of the reproducible sequence may be selected so that the reproducible sequence is homologous only to the target sequence in the genome, and not to other sequences in the genome. The relevant portion of the reproducible sequence may differ from the corresponding portion of the target sequence by having at least one nucleic acid deletion, substitution or addition.

In alternative embodiments, the primer may be acted upon by a nucleic acid polymerase, encoded by the host or heterologously expressed in the host, which has reduced fidelity in replicating the reproducible sequence of the gene targeting cassette. In such a case the gene targeting substrate produced may have random mutations as compared to the sequence encoded by the reproducible sequence encoding it. The gene targeting substrate produced in this manner may produce a variety of allelic variants when the mutated sequence integrates at the target locus. Libraries of cells or organisms bearing the mutated alleles may be selected for properties indicative of a desired phenotypic change or a desired property of the reproducible sequence.

### BRIEF DESCRIPTION OF THE DRAWING

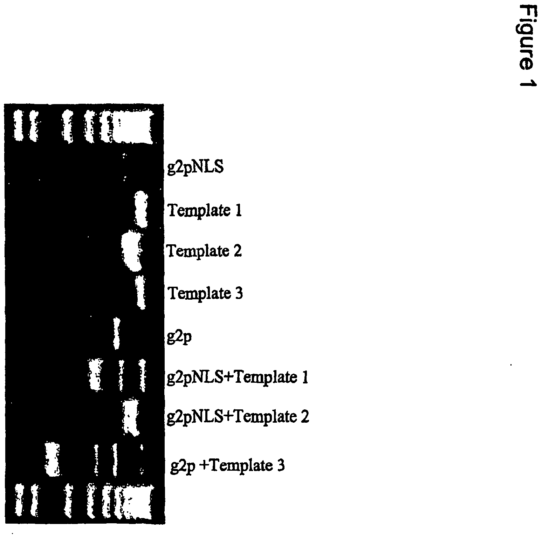

Figure 1 shows functionality of cloned rolling-circle replication components and engineered g2p. DNA was isolated from E. coli DH5alpha strains possessing plasmids encoding the cloned φfd initiator-terminator sequences plus intervening sequence (i.e. Template plasmids), or plasmids capable of expressing the nickase g2p or g2p-NLS, or combinations of Template plus nickase plasmids. Template 1 plasmid was pMW113. Template 2 plasmid was pMW114 which has the same intervening sequence as pMW113 but does not encode functional φfd initiator-terminator sequences. Template 3 plasmid was pRH24. g2p was encoded by pRH27. g2p-NLS was encoded by pAS 17. Note the novel DNA molecule produced by rolling-circle replication when both the nickase and template plasmids are combined. In this embodiment, production of this product is dependent on both the nickase and functional φfd initiator-terminator sequences. Outermost lanes are 1 kb ladder (Gibco BRL) DNA molecular size markers.

### DETAILED DESCRIPTION OF THE INVENTION

In various embodiments as set out in the appended claim, the invention provides processes for producing ssDNA or dsDNA substrates for gene targeting. In some embodiments, multiple copies of a gene targeting substrate may be produced *in vivo* or *in nucleo* of a target organism's cells. Production of gene targeting substrates *in vivo* and/or *in nucleo* may enable accumulation of the gene targeting substrate within the nucleus to a concentration which results in frequent gene targeting events.

In some embodiments, gene targeting methods of the invention may make use of endogenous or heterologous nucleic acid polymerases, a family of highly processive enzymes, and gene targeting substrates that may be many kilobases in length. Extensive regions of homology to the target locus may be engineered into the gene targeting cassette so as to increase the specificity and frequency of gene targeting events.

The degree of homology between sequences may be expressed as a percentage of identity when the sequences are optimally aligned, meaning the occurrence of exact matches between the sequences. Optimal alignment of sequences for comparisons of identity may be conducted using a variety of algorithms, such as the local homology algorithm of Smith and Waterman, 1981, Adv. Appl. Math 2: 482, the homology alignment algorithm of Needleman and Wunsch, 1970, J. Mol. Biol. 48:443, the search for similarity method of Pearson and Lipman, 1988, Proc. Natl. Acad. Sci. USA 85: 2444, and the computerised implementations of these algorithms (such as GAP, BESTFIT, FASTA and TFASTA in the Wisconsin Genetics Software Package, Genetics Computer Group, Madison, WI, U.S.A.). Sequence alignment may also be carried out using the BLAST algorithm, described in Altschul et al., 1990, J. Mol. Biol. 215:403-10 (using the published default settings). Software for performing BLAST analysis may be available through the National Center for Biotechnology Information (through the internet at http://www.ncbi.nlm.nih.gov/). The BLAST algorithm involves first identifying high scoring sequence pairs (HSPs) by identifying short words of length W in the query sequence that either match or satisfy some positive-valued threshold score T when aligned with a word of the same length in a database sequence. T is referred to as the neighbourhood word score threshold. Initial neighbourhood word hits act as seeds for initiating searches to find longer HSPs. The word hits are extended in both directions along each sequence for as far as the cumulative alignment score can be increased. Extension of the word hits in each direction is halted when the following parameters are met: the cumulative alignment score falls off by the quantity X from its maximum achieved value; the cumulative score goes to zero or below, due to the accumulation of one or more negative-scoring residue alignments; or the end of either sequence is reached The BLAST algorithm parameters W, T and X determine the sensitivity and speed of the alignment The BLAST programs may use as defaults a word length (W) of 11, the BLOSUM62 scoring matrix (Henikoff and Henikoff, 1992, Proc. Natl. Acad. Sci. USA 89: 10915-10919) alignments (B) of 50, expectation (E) of 10 (which may be changed in alternative embodiments to 1 or 0.1 or 0.01 or 0.001 or 0.0001; although E values much higher than 0.1 may not identify functionally similar sequences, it is useful to examine hits with lower significance, E values between 0.1 and 10, for short regions of similarity), M=5, N=4, for nucleic acids a comparison of both strands. For protein comparisons, BLASTP may be used with defaults as follows: G=11 (cost to open a gap); E=1 (cost to extend a gap); E=10 (expectation value, at this setting, 10 hits with scores equal to or better than the defined alignment score, S, are expected to occur by chance in a database of the same size as the one being searched; the E value can be increased or decreased to alter the stringency of the search.); and W=3 (word size, default is 11 for BLAST, 3 for other blast programs). The BLOSUM matrix assigns a probability score for each position in an alignment that is based on the frequency with which that substitution is known to occur among consensus blocks within related proteins. The BLOSUM62 (gap existence cost = 11; per residue gap cost = 1; lambda ratio = 0.85) substitution matrix is used by default in BLAST 2.0. A variety of other matrices may be used as alternatives to BLOSUM62, including: PAM30 (9,1,0.87); PAM70 (10,1,0.87) BLOSUM80 (10,1,0.87); BLOSUM62 (11,1,0.82) and BLOSUM45 (14,2,0.87). One measure of the statistical similarity between two sequences using the BLAST algorithm is the smallest sum probability (P(N)), which provides an indication of the probability by which a match between two nucleotide or amino acid sequences would occur by chance. In alternative embodiments of the invention, nucleotide or amino acid sequences are considered substantially identical if the smallest sum probability in a comparison of the test sequences is less than about 1, preferably less than about 0.1, more preferably less than about 0.01, and most preferably less than about 0.001.

Nucleic acid sequences to be used in the methods of the invention may in some embodiments be substantially identical, such as substantially identical gene targeting substrates and target sequences. The substantial identity of such sequences may be reflected in percentage of identity when optimally aligned that may for example be greater than 50%, 80% to 100%, at least 80%, at least 90% or at least 95%, which in the case of gene targeting substrates may refer to the identity of a portion of the gene targeting substrate with a portion of the target sequence, wherein the degree of identity may facilitate homologous pairing and recombination and/or repair. An alternative indication that two nucleic acid sequences are substantially identical is that the two sequences hybridize to each other under moderately stringent, or preferably stringent, conditions. Hybridization to filter-bound sequences under moderately stringent conditions may, for example, be performed in 0.5 M NaHPO₄, 7% sodium dodecyl sulfate (SDS), 1 mM EDTA at 65°C, and washing in 0.2 x SSC/0.1% SDS at 42°C (see Ausubel, et al. (eds), 1989, Current Protocols in Molecular Biology, Vol. 1, Green Publishing Associates, Inc., and John Wiley & Sons, Inc., New York, at p. 2.10.3). Alternatively, hybridization to filter-bound sequences under stringent conditions may, for example, be performed in 0.5 M NaHPO₄, 7% SDS, 1 mM EDTA at 65°C, and washing in 0.1 x SSC/0.1% SDS at 68°C (see Ausubel, *et al.* (eds), 1989, supra). Hybridization conditions may be modified in accordance with known methods depending on the sequence of interest (see Tijssen, 1993, Laboratory Techniques in Biochemistry and Molecular Biology -- Hybridization with Nucleic Acid Probes, Part I, Chapter 2 "Overview of principles of hybridization and the strategy of nucleic acid probe assays", Elsevier, New York). Generally, stringent conditions are selected to be about 5°C lower than the thermal melting point for the specific sequence at a defined ionic strength and pH.

In various aspects, the invention involves the specific replication of a reproducible nucleic acid sequence encoding the gene targeting substrate. To facilitate this, the system may include genetic elements and structural and enzymatic proteins involved in nucleic acid replication. The reproducible sequence encoding the gene targeting cassette may be flanked by specific nucleic acid sequences that mediate nucleic acid replication, so that replication may be initiated on one side of the reproducible sequence, by a replication initiator sequence, and terminated on the other side of the reproducible sequence by a replication terminator sequence, the replication terminator sequence being either part of the cassette or within the adjoining portion of the host genome. The terminator sequence need not be the same in each round of replication, and need not be a specific defined sequence within the host genome since in some embodiments the replication machinery may proceed though the reproducible sequence and then terminate at variable positions within the adjoining genome. In some embodiments, by the action of endogenous proteins or heterologous proteins expressed in an appropriate context in the cells of interest, a replication "primers" is formed and located at the replication initiator sequence. Such primers are components of the replication factors of the invention that, alone or in concert with endogenous or heterologous factors present in the host cell, mediate replication of the reproducible sequence. This replication primer may provide a hydroxyl group in the appropriate context to initiate nucleic acid replication by a polymerase. The primer may for example be derived from DNA, RNA or protein. The primer may for example be acted upon by endogenous or heterologous polymerases to replicate the reproducible sequence encoding a gene targeting substrate. The polymerase may proceed from the replication primer using one strand of the cassette as template to produce a new complementary strand while displacing the old strand of the reproducible sequence. In such embodiments, when the nucleic acid replication terminator site sequence is reached, such as when a sequence present in the host genome that can terminate replication is reached, the reproducible sequence will have been replicated. At this point, depending upon the mechanism used for priming nucleic acid synthesis at the initiator sequence, as discussed in the context of alternative embodiments, either the displaced "old" strand or the newly synthesized strand may be released. Thus one molecule of gene targeting substrate is produced as part of a reproduced sequence, and with each molecule of gene targeting substrate produced the dsDNA sequence of the gene targeting cassette is also resynthesized, so that the replication process can be repeated. Thus, with repeated cycles of gene targeting substrate synthesis and liberation, and concurrent regeneration of the coding sequence, multiple copies of gene targeting substrate may be produced *in vivo* as, so that set out in the appended claims the multiple copies may for example accumulate within a nucleus. *In nucleo* accumulation of multiple copies of the gene targeting substrate may facilitate a higher effective concentration of gene targeting substrate than would be attained by transformation with an exogenously supplied gene targeting substrate.

Depending upon the mechanism used to produce the gene targeting substrate, as used in the methods of the appended claims and described in the context of alternative embodiments, the gene targeting substrate may for example be a linear or covalently-closed ssDNA or dsDNA molecule. Both ssDNA and dsDNA molecules reportedly function as gene targeting substrate in prokaryotes and eukaryotes [10;11;15;17;18;24-27;31]. ssDNA gene targeting substrate may be converted to dsDNA in several fashions. A non-exclusive list of means that may be used to convert a ssDNA gene targeting substrate to a dsDNA gene targeting substrate includes:
1.) engineering the ssDNA to encode inverted repeat sequences which will anneal to one another in a hairpin fashion to create dsDNA;
2.) generating two forms of ssDNA which occur in opposite polarity (i.e. one in "sense" orientation and the other in the "antisense" orientation), so that the two molecules will be able to anneal/base-pair with one another to form a dsDNA molecule.

In alternative embodiments according to the methods of the appended claims, a gene targeting substrate may be synthesized so that it creates ssDNA or dsDNA gene targeting substrates. Nucleic acid molecules with cut or broken ends may also be provided as gene targeting substrates in alternative embodiments since such molecules may be efficient substrates for recombination and or repair [52-54]. In alternative embodiments, gene targeting substrates may be engineered to encode the recognition sites for enzymes or restriction enzymes that cleave ssDNA [55; 218] or dsDNA [56-59]. In such embodiments, production of gene targeting substrate *in vivo* may be coordinated with expression of the DNA cleaving enzyme, for example through use of appropriate promoters driving expression of the enzyme and a component of the replication system. The enzyme may then interact with its recognition sequence on the gene targeting substrate and cleave the DNA creating a linear molecule. This could then interact with host recombination and/or repair functions to facilitate the gene targeting event.

In some gene targeting methods of the invention, the gene targeting substrate may be produced by a combination of endogenous and heterologous protein and genetic elements required to initiate nucleic acid synthesis, catalyse nucleic acid polymerization and terminate nucleic acid synthesis. To produce the gene targeting substrate the required components may be placed into the host cell genome or be located on extrachromosomal elements, such as episomes or plasmids or viral genomes or artificial chromosomes, or any combination thereof.

In some embodiments of the appended claims, when expressing a protein in host cells or organisms, it may be desirable to use a protein-encoding polynucleotide that employs a codon distribution other than that found in the naturally occurring gene. Protein-encoding polynucleotides with alternative codons in the coding sequence may be used to optimize (e.g., increase) expression of the protein in hosts that have different preferential codon usage than the organism from which the gene is derived. Codon changes may also be used to facilitate manipulation of the polynucleotide of interest (e.g., by engineering useful tags or restriction sites into the coding sequence), and for other reasons. When the goal is to optimize expression (e.g., by increasing translational efficiency), tables of preferred codon usage, which are publicly available and are well known to those of skill in the art, may be used to design a suitable polynucleotide by "reverse translation" of the desired amino acid sequence. Alternatively, preferred codon usage may be determined for a particular organism or class of genes by comparison of published gene sequences for the target organism or gene class.

In alternative embodiments of the methods as set out in the appended claims, the initiator sequence and reproducible sequence may be flanked on each side by the recognition sequence for a site-specific recombinase such as, for example, FLP protein of the 2 micron element. Such embodiments may be adapted so that by the action of the recombinase on its respective recognition sequence the initiator sequence and reproducible sequence are excised (from the chromosomal locus or the extrachromosomal vector where they are integrated) as a circular dsDNA molecule. The action of replication factor(s) on the initiation sequence encoded by the excised molecule may produce a primer which can be acted upon by host enzymes resulting in replication of the reproducible sequence.

In various aspects the present invention relates to the modification of genes by gene targeting and the use of recombinant genes to synthesize gene targeting components in vivo. In this context, the term "gene" is used in accordance with its usual definition in the art, to mean an operatively linked group of nucleic acid sequences. The targeted modification of a gene in the context of the present invention (called gene targeting) may include the modification of any one of the various sequences that are operatively linked in the gene. By "operatively Linked" it is meant that the particular sequences interact either directly or indirectly to carry out their intended function, such as mediation or modulation of gene expression. The interaction of operatively linked sequences may for example be mediated by proteins that in turn interact with the sequences.

The expression of a gene will typically involve the creation of a polypeptide which is coded for by a portion of the gene. This process typically involves at least two steps: transcription of a coding sequence to form RNA, which may have a direct biological role itself or which may undergo translation of part of the mRNA into a polypeptide. Although the processes of transcription and translation are not fully understood, it is believed that the transcription of a DNA sequence into mRNA is controlled by several regions of DNA. Each region is a series of bases (i.e., a series of nucleotide residues comprising adenosine (A), thymidine (T), cytidine (C), and guanidine (G)) which are in a desired sequence.

Regions which are usually present in a gene include a promoter sequence with a region that causes RNA polymerase to associate with the promoter segment of DNA. The RNA polymerase normally travels along an intervening region of the promoter before initiating transcription at a transcription initiation sequence, that directs the RNA polymerase to begin synthesis of mRNA. The RNA polymerase is believed to begin the synthesis of mRNA an appropriate distance, such as about 20 to about 30 bases, beyond the transcription initiation sequence . The foregoing sequences are referred to collectively as the promoter region of the gene, which may include other elements that modify expression of the gene. For example, certain promoters present in bacteria contain regulatory sequences that are often referred to as "operators", and certain promoters in eukaryotes contain regulatory sequences that are often referred to as "enhancers". Such complex promoters may contain one or more sequences which are involved in induction or repression of the gene.

In the context of the present invention, "promoter" means a nucleotide sequence capable of mediating or modulating transcription of a nucleotide sequence of interest in the desired spatial and temporal pattern and to the desired extent, when the transcriptional regulatory region is operably linked to the sequence of interest. A transcriptional regulatory region and a sequence of interest are "operably linked" when the sequences are functionally connected so as to permit transcription of the sequence of interest to be mediated or modulated by the transcriptional regulatory region. In some embodiments, to be operably linked, a transcriptional regulatory region may be located on the same strand as the sequence of interest. The transcriptional regulatory region may in some embodiments be located 5' of the sequence of interest. In such embodiments, the transcriptional regulatory region may be directly 5' of the sequence of interest or there may be intervening sequences between these regions. Transcriptional regulatory sequences may in some embodiments be located 3' of the sequence of interest. The operable linkage of the transcriptional regulatory region and the sequence of interest may require appropriate molecules (such as transcriptional activator proteins) to be bound to the transcriptional regulatory region, the invention therefore encompasses embodiments in which such molecules are provided, either *in vitro* or *in vivo* as set out in the appended claims*.*

The sequence of DNA that is transcribed by RNA polymerase into messenger RNA generally begins with a sequence that is not translated into protein, referred to as a 5' non-translated end of a strand of mRNA, that may attach to a ribosome. In bacterial cells, this attachment may be facilitated by a sequence of bases called a "ribosome binding site" (RBS), mRNA molecules in eukaryotic cells may have functionally analogous sequence called internal ribosome entry sites (IRES). Regardless of whether an RBS or IRES exists in a strand of mRNA, the mRNA moves through the ribosome until a "start codon" is encountered. The start codon is usually the series of three bases, AUG; rarely, the codon GUG may cause the initiation of translation.

The next sequence of bases in a gene is usually called the coding sequence or the structural sequence. The start codon directs the ribosome to begin connecting a series of amino acids to each other by peptide bonds to form a polypeptide, starting with methionine, which forms the amino terminal end of the polypeptide (the methionine residue may be subsequently removed from the polypeptide by other enzymes). The bases which follow the AUG start codon are divided into sets of 3, each of which is a codon. The "reading frame," which specifies how the bases are grouped together into sets of 3, is determined by the start codon. Each codon codes for the addition of a specific amino acid to the polypeptide being formed. Three of the codons (UAA, UAG, and UGA) are typically "stop" codons; when a stop codon reaches the translation mechanism of a ribosome, the polypeptide that was being formed disengages from the ribosome, and the last preceding amino acid residue becomes the carboxyl terminal end of the polypeptide.

The region of mRNA which is located on the 3' side of a stop codon in a monocistronic gene is referred to as a 3' non-translated region. This region may be involved in the processing, stability, and/or transport of the mRNA after it is transcribed. This region may also include a polyadenylation signal which is recognized by an enzyme in the cell that adds a substantial number of adenosine residues to the mRNA molecule, to form a poly-A tail.

Various genes and nucleic acid sequences refered to in the context of the invention may be recombinant sequences. The term "recombinant" means that something has been recombined, so that when made in reference to a nucleic acid construct the term refers to a molecule that is comprised of nucleic acid sequences that are joined together or produced by means of molecular biological techniques. The term "recombinant" when made in reference to a protein or a polypeptide refers to a protein or polypeptide molecule which is expressed using a recombinant nucleic acid construct created by means of molecular biological techniques. The term "recombinant" when made in reference to genetic composition refers to a gamete or progeny or cell or genome with new combinations of alleles that did not occur in the parental genomes. Recombinant nucleic acid constructs may include a nucleotide sequence which is ligated to, or is manipulated to become ligated to, a nucleic acid sequence to which it is not ligated in nature, or to which it is ligated at a different location in nature. Referring to a nucleic acid construct as 'recombinant' therefore indicates that the nucleic acid molecule has been manipulated using genetic engineering, i.e. by human intervention. Recombinant nucleic acid constructs may for example be introduced into a host cell by transformation. Such recombinant nucleic acid constructs may include sequences derived from the same host cell species or from different host cell species, which have been isolated and reintroduced into cells of the host species. Recombinant nucleic acid construct sequences may become integrated into a host cell genome, either as a result of the original transformation of the host cells, or as the result of subsequent recombination and/or repair events.

In one aspect, the invention may provide methods using gene targeting cassettes for use in plants as set out in the appended claims. In this aspect of the invention, a plant transformation construct may be assembled in an appropriate vector to facilitate transfer of the gene targeting system components into the plant genome, for example by *Agrobacterium*[60] or biolistic delivery [61] or chemical treatment [37;38] or physical treatment [40-42]. The components included in the transformation cassette may optionally comprise one or more of the following components:
i.) A gene targeting cassette encoding the gene targeting substrate as part of a reproducible sequence, the gene targeting substrate having a sequence homologous to the target genomic locus that may encode a desired genetic change (i.e. one or more basepair insertions, deletions or changes) to be transferred to the target locus;
ii.) Replication initiator and terminator sequences flanking the reproducible sequence of the gene targeting cassette;
iii.) Gene(s) encoding specific replication (Rep) factor(s) (and alternatively further also encoding necessary accessory factors), such as protein(s) responsible for creation of a replication primer for nucleic acid synthesis at the initiator sequence which may be acted upon by a polymerase. Rep factor(s) may also participate in termination and release of the copy of gene targeting substrate when a polymerase traverses the terminator sequence;
iv.) Transcription promoter and terminator sequences for mediating expression of Rep factor(s); or
v.) Selectable marker(s) with appropriate gene expression elements to enable identification or selection of cells or regenerated plants that have the gene targeting components integrated into the genome.

Following transformation, a gene targeting cassette may be integrated into the host genome, and transformed cells may be selected from non-transformed cells using the appropriate selection agent corresponding to the selectable marker on the transformation cassette.

If, for example, the Rep factor(s) (with or without accessory factors) is(are) encoded by the gene targeting cassette adjacent to a constitutive promoter then immediately upon entry of the transformation cassette into the host cell or nucleus the Rep factor(s) may be functionally expressed to initiate production of gene targeting substrate. Alternatively, the host cell may naturally encode the Rep factor(s) or be previously modified to encode the Rep factor(s) so that entry of the gene targeting cassette can result in initiation of production of gene targeting substrate. Upon entry of the gene targeting cassette into the host cell or nucleus, Rep factor(s) (with or without accessory factors), alone or in concert with host nucleic acid replication machinery, may then initiate production of gene targeting substrate by acting on the initiator and terminator sequences, so that gene targeting substrate may be synthesized *in vivo* and accumulate in the host cell and/or *in nucleo.*

The gene targeting substrate may pair with the target genomic locus, in a process facilitated by virtue of the homology between the sequences. Host recombination, repair and/or replication processes may then act to transfer the genetic change encoded by the gene targeting substrate into the target locus by processes such as nucleic acid recombination or gene conversion or nucleic acid repair.

In alternative embodiments of the methods as set out in the claims, the gene targeting system of the invention may provide for repeated production of gene targeting substrate in cell generations subsequent to treatment of cells with the transformation cassette.

In some embodiments, the invention may provide for the temporal and/or spatial regulation of the production of gene targeting substrate during plant development.

For example, by using appropriate transcription and translation regulatory sequences, the functional expression of Rep factor(s) may be coordinated with particular points in the cell cycle or made to occur in particular tissues or during particular developmental stages so as to regulate the timing of gene targeting.

In alternative embodiments, the methods of the invention may provide for different types of expression of Rep factor(s) and/or gene targeting substrates, such as:
i) Constitutive
   Gene targeting substrate may be produced and be present in all cells and tissues and at all developmental and physiological stages. In some instances constitutive production of gene targeting substrate may be undesirable because of unwanted physiological or genetic load on the plant cells. Therefore, more specific expression may be advantageous in some situations.
ii) Cell cycle coordination
   Endogenous nucleic acid recombination and/or repair activities may be elevated during S-phase of the cell cycle [62]. Therefore, production of gene targeting substrate may be coordinated with S-phase so that endogenous nucleic acid recombination and/or repair enzymes may promote modification of the target locus by transfer of the genetic information from the gene targeting substrate to the target locus.

Synchronization of the production and presence of gene targeting substrate *in vivo* with selected points in the cell cycle may for example be achieved through the use of cell-cycle specific promoters to express Rep factor(s).
e.g. histone promoters: Histone genes are expressed coordinately with DNA replication to produce the abundant proteins required to package the newly synthesized DNA [64;65].
e.g. cyclins and cell division control genes are expressed at various points in the cell cycle to initiate and terminate passage through the different stages of the cell cycle [66].
Thus these two groups of promoters are listed as non-exclusive examples of promoters for use to coordinate expression of Rep factor(s) and production of gene targeting substrate with various stages of the cell cycle.

In alternative embodiments of the methods of the invention, coordination of the production of gene targeting substrate with cell division may allow the gene targeting substrate to be produced in dividing cells in the apical meristem. In plants, this may provide opportunities for a gene targeting event to occur in a cell which will, directly or indirectly, later give rise to the germ line, so that progeny plants may stably inherit the modified target locus.

In some embodiments of the methods of the invention gene targeting frequency may be increased by manipulating progression of the cell cycle. In multi-cellular organisms most cells are non-proliferating, differentiated cells in which DNA replication factors are absent because their genes are not being expressed or the factors are functionally inactive [329]. In cultured cells DNA replication factors may also be absent or inactive depending upon cellular origin or culture conditions like age and media composition. It has been established that in many biological systems expression and activity of cellular DNA recombination and repair processes are linked to the DNA replication process and that the activity of DNA recombination and repair machinery is naturally elevated during S-phase [240-244]. Accordingly, in some embodiments of the invention, the regulation of the cell cycle may be manipulated to control the activity level of cellular recombination and repair machinery and, thereby, influence or modulate the inherent potential of cells to promote homologous recombination and facilitate efficient gene targeting. In other embodiments, the invention may involve stimulation of S-phase onset and/or increasing the activity of related cellular machinery. These steps may be used to increase DNA synthesis (replication) of the reproducible sequence and to increase production of gene targeting substrate. Much of the cellular machinery (i.e. enzymatic, structural and regulatory proteins) responsible for DNA replication and regulation and progression of the cell cycle and cell growth is well conserved from yeast to animals, including humans, and plants [329;245]. Therefore many proteins may be potentially used to regulate the cell cycle and influence gene targeting frequency.

In one embodiment of the methods of the invention the regulation of the cell cycle may be achieved through manipulating the activity of members of the 'pocket family' of proteins, such as the retinoblastoma (Rb) tumour suppressor protein [329]. Rb is a central regulator of cell passage through the G1 phase and the G1-S transit of cell cycle by modulating the activity of the E2F-DP family of transcription factors [329;245]. Phosphorylation of Rb by CDK-cyclin complexes leads to release of Rb-bound E2F-DP transcription factors required to activate expression of genes required for the G1-S transition and S-phase progression [329]. Rb-like proteins are found in animal systems and plants where it is referred to as Rb-related (RBR) protein [329]. Many animal and plant viruses exploit the Rb-mediated control pathway to turn on the host DNA replication machinery and facilitate replication of the viral genomes. In such cases a viral encoded protein physically interacts with the Rb or RBR protein thereby impairing the ability of Rb or RBR to regulate the cell cycle [329]. As a result, the host cell moves into S-phase and the DNA replication process, as well as the coordinated DNA recombination and repair processes, are expressed and functional.

In some embodiments of the methods of the invention gene targeting frequency may be increased by controlling the activity of Rb or RBR or related proteins to control the onset and activity of S-phase functions, including recombination and repair processes. In some embodiments this control of Rb or RBR proteins may be mediated through controlling expression and function of viral proteins that interact with Rb or RBR. In some embodiments the influence on cell cycle progression and gene targeting frequency in animal cells may be mediated by proteins, such as the SV40 T-antigen [246], or the adenovirus E1A protein [247], or the papillomavirus E7 [248]. In some embodiments the influence on cell cycle progression and gene targeting frequency in plant cells may be mediated by proteins such as, for example, RepC1 of TYLCV, as described above, or the RepA proteins from maize streak virus [249], wheat dwarf virus [239], bean yellow dwarf virus [250], or tomato golden mosaic virus [251]. For example, for gene targeting applications in plants, a cell line or plant line can be developed where the RepC1- or RepA-like protein is expressed. Cells or tissues from these lines may thus possess increased potential for DNA replication and the coordinated recombination and repair functions. Gene targeting substrates delivered or produced in these cells or tissues may, therefore, have increased frequency of transferring genetic changes to target loci. In alternative embodiments, a gene construct for expressing RepC1- or RepA-like proteins may be introduced into plant cells or tissues coordinately with the delivery or production of gene targeting substrates in these cells or tissues. In such cases the RepC1- or RepA-like proteins may stimulate the onset of S-phase activities, and the concomitant increased activity level of recombination and repair processes, coordinately with the presence of the gene targeting substrate. This may result in increase frequency of transferring genetic changes to target loci.

### iii) Developmental stage coordination

Endogenous nucleic acid recombination and/or repair activities may be elevated during certain developmental stages, for example meiosis [67]. Therefore, production of gene targeting substrate may be coordinated with these developmental stages so as to exploit the elevated levels of endogenous nucleic acid recombination and/or repair activities to transfer the genetic information from the gene targeting substrate to the target locus . This may for example be achieved by expression of Rep factor(s) using promoters expressed during meiosis or meiosis-specific promoters. Numerous examples exist of genes which are expressed at this stage and whose promoters may be adapted for use in this invention [68-71].

### iv.) Tissue specific promoters

Specific tissues may have elevated endogenous nucleic acid recombination and/or repair activity and/or be more amenable for increased gene targeting frequency due to other biochemical, cellular, physiological or developmental states.

e.g. Developing embryos undergo rapid cell division and have active nucleic acid recombination and/or repair systems [72]. Therefore, production and accumulation of gene targeting substrate in embryos or embryonic tissues could lead to increased gene targeting frequency.

e.g. Developing and mature male and female gametophytes (i.e. pollen and egg cells) are haploid. Haploid cells may be more recombinogenic and amenable to gene targeting than diploid cells [20]. Therefore, expression of Rep factor(s) and production of gene targeting substrate in these cells and tissues using appropriate promoters may increase gene targeting frequency,

Tissue specific promoters could also be used if one desired gene targeting to only occur in a particular tissue so that other tissues will not possess the genetically modified target locus. Thus one may use a tissue or organ-specific promoter to create a chimeric plant or animal containing both unmodified and modified target genes, each being present in different tissues or organs.

Achieving gene targeting during meiosis and/or gametes may also have additional advantages in alternative embodiments of the methods claimed, including:
a) Embodiments as claimed adapted to generate homozygous lines with targeted changes. If the gene targeting event is adapted to occur at Meiosis I, then each of the resultant four gametes will contain the specified genetic change. With gene targeting substrate delivered to meiotic cells, such as in early stages of Meiosis I, large numbers of male and female gametes with the desired targeted genetic changes may result. In plants and other monoecious organisms where both male and female gametes are produced by the same individual, simply self-crossing the individual may result in a desired frequency of diploid progeny which are homozygous for the targeted genetic change. In alternative embodiments, in the case of plants, one may obtain individuals homozygous for the targeted genetic change by performing microspore culture after delivering gene targeting substrate to the meiotic cells. Microspores are haploid cells resulting from meiosis in the plant anther. These cells can in some cases be cultured to regenerate entire plants [73]. The plants can be chemically treated to create a diploid chromosome content and are thus homozygous for all genetic information. Therefore, microspores carrying the targeted genetic change as a result of treating meiotic cells or the microspores themselves with gene targeting substrate may be cultured and converted into plants that are homozygous for the targeted genetic change. Alternatively, where male and female gametes are produced by different individuals, the gene targeting process could be done in both a male and a female plant, and the two crossed.
b) Embodiments as claimed adapted for direct germ-line transmission of a targeted genetic change. Targeted genetic change generated in a gamete in accordance with the Invention may be heritable in the offspring. In contrast, gene targeting conducted in somatic cells will only be heritable if the somatic cell can directly or indirectly give rise to the germ-line from which gametes are derived.
c) Embodiments as claimed adapted to target changes to either maternal or paternal derived chromosomes. Targeted changes in either maternal or paternal chromosomes may for example be obtained with this invention by delivering gene targeting substrate specifically to either female or male reproductive organs.

### v) Environmentally Stimulated

In some embodiments, the methods of the invention may provide for activation of gene targeting by environmental stimuli, for example by linking expression of components of the gene targeting system of the invention to promoters that are responsive to environmental stimuli. Exposure of cells to different environmental conditions can elevate activity of endogenous nucleic acid recombination and/or repair processes [75-77]. Therefore, it may be beneficial to coordinate production of gene targeting substrate in response to these stimuli to take advantage of the elevated nucleic acid recombination and/or repair activity so as to transfer the genetic information from the gene targeting substrate to the target locus.
For example, the RAD51 gene encodes an enzyme involved in DNA recombination and repair that is induced in response to DNA damaging agents [78;79]. Rep factor(s) of the invention could be fused to the RAD51 promoter to coordinate induction and production of gene targeting substrate with endogenous nucleic acid recombination and/or repair functions in response to environmental stimuli.

### vi) Inducible

In alternative aspects of the invention as claimed, inducible promoters may be provided to drive expression of components of the gene targeting system. For example, a sequence encoding Rep factor(s) may be cloned behind an inducible or repressible promoter. The promoter may then be induced (or de-repressed) by appropriate external treatment of the organism when organismal development proceeds to a point when gene targeting is desired. Regulation of such promoters may be mediated by environmental conditions such as heat shock [80], or chemical stimulus. Examples of chemically regulatable promoters active in plants and animals include the ecdysone, dexamethasone, tetracycline and copper systems [81-86].

### vii) Bipartite Systems

In alternative embodiments of the invention as claimed, bipartite promoters may be used to express Rep factors). Bipartite systems may for example consist of 1) a minimal promoter containing a recognition sequence for 2) a specific transcription factor. The bipartite promoter is inactive unless it is bound by the transcription factor. The gene of interest may be placed behind the minimal promoter so that it is not expressed, and the transcription factor may be linked to a 'control promoter' which is, for example, a tissue-specific, developmental stage specific, or environmental stimuli responsive promoter. The transcription factor may be a naturally occurring protein or a hybrid protein composed of a DNA-binding domain and a transcription-activating domain. Because the activity of the minimal promoter is dependent upon binding of the transcription factor, the operably-linked coding sequence will not be expressed unless conditions are appropriate for expression by the `control promoter'. When such conditions are met, the 'control promoter' will be turned on facilitating expression of the transcription factor. The transcription factor will act in trans and bind to the DNA recognition sequence in the minimal promoter via the cognate DNA-binding domain. The activation domain of the transcription factor will then be in the appropriate context to aid recruitment of RNA polymerase and other components of the transcription machinery. This will cause transcription of the target gene. With this bipartite system, the gene of interest will only be expressed in cells where the 'control promoter' is expressed (i.e. the target gene will be expressed in a spatial and temporal pattern mirroring the 'control promoter' expressing the transcription factor). In addition, a bipartite system could be used to coordinate expression of more than one gene. Different genes could be placed behind individual minimal promoters all of which have the same recognition sequence for a specific transcription factor and whose expression, therefore, is reliant upon the presence of the transcription factor. The transcription factor is linked to a 'control promoter'. Therefore, when cells enter an appropriate stage where gene targeting is to be initiated, the control promoter expresses the transcription factor which then can coordinately activate expression of the suite of target genes. Use of a bipartite system may have the advantage that if expression of the target genes is no longer required in a particular plant or animal line, then the transcription factor may be bred out, so that without the transcription factor present, the target gene(s) will no longer be expressed in this line. If the target genes are desired to be expressed at a later stage, the promoter: transcription factor locus may be bred back into the line.

Minimal promoter elements in bipartite promoters may include, for example:
1) truncated CaMV 35S (nucleotides -59 to +48 relative to the transcription start site) [87];
2) DNA recognition sequences: *E. coli* lac operator [88;89], [89]yeast GAL4 upstream activator sequence [87]; TATA BOX, transcription start site, and may also include a ribosome recruitment sequence.

Bipartite promoters may for example include transcription factors such as: the yeast GAL4 DNA-binding domain fused to maize C1 transcription activator domain [87]; *E. coli* lac repressor fused to yeast GAL4 transcription activator domain [88]; or the *E. coli* lac repressor fused to herpes virus VP16 transcription activator domain [89].

In some situations, the 'control promoter', which is, for example, a tissue-specific, developmental stage specific, or environmental stimuli responsive promoter may promote transcription at too low of a level (i.e. weakly expressed) or at too high of a level (i.e. strongly expressed) to achieve the desired effect for gene targeting. Therefore, for example, a weak control promoter may be used in the bipartite system to express a transcription factor which can promote a high level of expression when it binds to the minimal promoter adjacent to the gene of interest. Thus while the gene of interest might only be expressed at a low level if it was directly fused to the 'control promoter', this promoter can indirectly facilitate high level expression of the gene of interest by expressing a very active transcription factor. The transcription factor may be present at low levels but because it is so effective at activating transcription at the minimal promoter fused to the gene of interest, a higher level of expression of the gene of interest will be achieved than if the gene was directly fused to the weak 'control promoter'. In addition, the transcription factor may also be engineered so that its mRNA transcript is more stable or is more readily translated, or that the protein itself is more stable. Conversely, if the "control promoter' is too strong for a desired application, it may be used to express a transcription factor with low ability to promote transcription at the minimal promoter adjacent to the target gene.

In alternative embodiments, a 'control promoter' may be used to express a heterologous RNA-polymerase which recognizes specific sequences not naturally present in the cell. For example, T7 RNA Polymerase may be used in eukaryotes to specifically promote transcription of a target gene linked to the T7 RNA Pol recruitment DNA sequence [90]. Components of the gene targeting system may then be regulated by the expression of T7 RNA Polymerase.

The embodiments of the invention as claimed relating to the control of expression of Rep factor(s) and coordinate production of gene targeting substrate as exemplified for plants may be applicable to animals as well as other eukaryotes (and prokaryotes), where there is conservation of processes and abilities to achieve gene expression, such as the foregoing types of expression control: i.) constitutive; or ii.) coordinated with cell-cycle, iii.) coordinated with development, iv.) tissue-specific, v.) responsive to environmental stimuli, vi.) inducible, or vii.) bipartite.

In some embodiments, genetic modification of target locus mediated by a gene targeting substrate as claimed in the methods of the invention may occur at any point from the initial transformation event, through all subsequent cell divisions, right up to the fully regenerated plant and production of gametes. Thus there are numerous opportunities for the gene targeting event to occur. When a cell that gives rise to the germ line has undergone the gene targeting event, the genetic change may be present in the gametes and stably passed on to the subsequent generation(s). If one allele of the target locus is altered by the gene targeting substrate in a diploid organism then up to 50% of the gametes from that particular germ line may be expected to carry the modified allele. However, if both alleles of the target locus are altered then all gametes from that germ line would be expected to carry the modified allele.

During meiosis normal chromosome recombination and reassortment may produce gametes which have the targeted change but no longer carry the initial transformation cassette. Thus self-crossing or out-crossing of a modified plant can lead to progeny that possess the modified target locus but not the initial transformation cassette. This is especially likely if the target locus has little or no genetic linkage to the genomic locus where the initial transformation cassette has inserted. In cases where the modified target locus is genetically linked to the initial transformation cassette then progeny from a segregating population may be evaluated to identify a recombinant where the modified target locus and the transformation cassette no longer cosegregate. Therefore, in this aspect of the invention, it may be possible to produce genetically changed plants which no longer have any undesired DNA sequences (e.g. the transformation cassette).

In accordance with some aspects of the invention creation of plants with specific genetic alterations at a target gene may involve a single tissue culture procedure: the initial transformation process where the gene targeting cassette is introduced to a plant cell. It may be possible for that cell or a progeny thereof to undergo the gene targeting during cell proliferation and regeneration into a plant. When this plant sexually reproduces, it may be possible for numerous progeny plants containing the genetic change resulting from gene targeting to be produced which may be derived from the initial single transformation event. Thus it may be possible in accordance with some aspects of the invention to minimize the number of tissue culture propagules required to be maintained in order to identify a gene targeting event, and to minimize tissue culture procedures which may be advantageous if it is desired to avoid the potential for genetic changes which may result from somaclonal variation during tissue culture [34]. In accordance with some aspects of the invention it may also be possible to use plant transformation procedures that require no tissue culture steps [91;92].

In alternative embodiments, specific changes of a target locus of interest may also be achieved with the invention if the gene targeting components are expressed from plant vectors that are not integrated in the plant genome. They may provide for methods of transiently transforming cells with gene targeting components.

In some embodiments, plant viruses may be used as vectors to carry and express foreign nucleic acid in plant cells [93] in conjunction with this invention. The components of the gene targeting system may for example be cloned into the viral vector. In one embodiment, cells or tissues are transformed with a gene targeting cassette carried by the viral vector. In such an embodiment, the Rep factor(s) (with or without accessory factors) may for example be expressed from the same viral vector encoding the replication initiator site and the reproducible sequence, or from a separate viral vector, in such a manner so that the Rep factor(s) act in concert with host functions so that a gene targeting substrate is produced *in vivo*. In alternative embodiments the host plant or plant cell may naturally express the Rep factor(s) or the host plant or plant cell may have been previously modified to express the Rep factor(s). If the viral vector is adapted to be localized and replicate in the plant cell nucleus, then the gene targeting substrate may accumulate *in nucleo.* If the viral vector is localized and replicates in the cytoplasm, movement of the gene targeting substrate into the nucleus may be enhanced, for example, by covalently or non-covalently linking the gene targeting substrate to protein(s) encoding a nuclear localization sequence. The gene targeting substrate may then facilitate the desired genetic change at the target genomic locus. Cells with the targeted genetic change can then be directly regenerated into a plant independently or as part of a chimera with cells not containing the targeted change. When the germ line of the regenerated plant is derived from a cell with the targeted genetic alteration, then the genetic change will be heritable.

In alternative embodiments, the targeted genomic change results in a selectable phenotype so that selection may be applied, resulting in enrichment for the survival and growth of only the cells with the targeted genetic alteration. Thus, the gene targeting events can be enriched and non-modified cells eliminated. The cells with the altered locus can then be regenerated into plants. Selecting for non-chimeric, genetically altered plants may increase the frequency of obtaining plants homozygous for the specified genetic change in the subsequent generation.

In other embodiments, the viral vector may have a conditional ability for propagation. Cells may be treated with such a vector and cultured under "permissive" conditions allowing viral vector replication to occur. Gene targeting events may then be induced to occur and screened or selected for. The cultured cells/tissues may then be placed under "stringent" conditions which disable the viral vector, so that plants with the specified genetic alteration can be regenerated which are free of the virus vector.

In other embodiments, intact plants are treated with a viral vector. In such embodiments, the gene targeting cassette may be produced and genetic alteration of the target locus may occur in random cells of the plant tissues. Tissues and/or cells are then collected from the treated plant and cultured appropriately to select or identify cells which have undergone the gene targeting event These cells may then be regenerated into plants which may pass the genetically modified locus to progeny.

In other embodiments, the components of the gene targeting method of the invention may be encoded by extrachromosomal elements such as episomes, plasmids or artificial chromosomes. In such cases, gene targeting could be achieved in accordance with the embodiments outlining the use of viral vectors as described above.

In some aspects, the gene targeting cassette may be present in the desired host on an extrachromosomal nucleic acid vector, such as an episome, plasmid, virus, or artificial chromosome. In some embodiments of the methods as claimed these extrachromosomal vectors may be capable of replicating in the host cell(s) by means of a nucleic acid origin of replication inherent to the vector, for example, as in a viral vector [222], or engineered into the vector, for example, as in a plasmid vector [232]. In some embodiments of the methods as claimed where the gene targeting cassette may be cloned into such vectors the gene targeting cassette may be replicated as a component of the vector so that the number of copies of the gene targeting cassette per cell may equal the number of vector molecules per cell. The gene targeting cassette, as in other embodiments of the methods as claimed, may encode a specific replication initiator sequence operably linked to a reproducible sequence. Activation of this replication initiator may depend on the action of a specific replication factor which may act independently of the origin of replication responsible for replication of the vector backbone. Thus the replication of the reproducible sequence may occur independently of the replication of the remainder of the vector. In this manner, the ratio of the number of copies per cell of the reproducible sequence to the number of copies per cell of the vector backbone encoding the reproducible sequence and other components of the gene targeting cassette may be different than one. The capability to alter this ratio may result in a desired frequency of gene targeting. The replication and release of the reproducible sequence from the vector backbone may also facilitate modification of a target locus in a fashion that reduces the chance of sequences other than those of the reproducible sequence, such as vector sequences, also being introduced into the target locus. Incorporation of vector sequences may occur with other systems. The presence of vector sequences in the target locus may be undesirable because, for example, these sequences may confer reduced genetic stability on the modified locus (due to nucleic acid recombination involving vector sequences), or they may incorporate undesirable genetic components into the host genome (such as selectable markers or viral sequences), or they may have undesirable effects on the expression and function of the target gene or other genes in the host chromosome (by the incorporation of additional promoter or enhancer sequences encoded by the vector).

In some embodiments of the claimed methods, transient expression of genes for components of the gene targeting system of the invention may be facilitated by introduction of DNA cassettes into plant cells by, for example, treatment of the cells with chemicals [37;38] or electrical current [40,41], or by biolistic introduction of particles coated with DNA [61], or by microinjection [42]. In such embodiments, gene targeting components can be transiently expressed to facilitate *in vivo* production of gene targeting substrate and consequent alteration of a specified genetic locus. In some embodiments the transient expression may not require replication of the vector backbone (encoding the gene targeting cassette) in the host cell. In alternative embodiments the vector backbone (encoding the gene targeting cassette) may replicate. Cells carrying the genetic alteration at the target genomic locus resulting from transient expression of the gene targeting system may then be propagated or regenerated into plants.

In some embodiments utilizing extrachromosomal elements such as viral or episomal vectors or artificial chromosomes, or transient expression of gene targeting components, where the components of the gene targeting system are maintained extrachromosomally on the vector, the host plants with the targeted genetic modification may not contain any undesired DNA sequences in their genome (having only the targeting change). The vector may be lost from cells encoding the targeted genetic modification as a result of missegregation of the extrachromosomal element(s) to daughter cells following mitotic or meiotic cell divisions whereby a daughter cell may result that no longer contains the extrachromosomal vector. Alternatively, loss of the vector may result from degradation of the vector by cellular processes. Subsequent daughter cells of a cell may be identified where the extrachromosomal vector is lost and they may thus also be free of undesired DNA sequences (e.g. the gene targeting components).

In alternative embodiments, the invention may be applied to animals and animal cells, in a variety of ways analogous to those described for plants. Cells and tissues from many animal species can be cultured in such embodiments, in accordance with methods known in the art, including procedures for the transfer of exogenous vector nucleic acid into animal cells to achieve transient or stable expression of vector-encoded genetic elements (with the vector remaining extrachromosomal or being integrated directly into the chromosome, respectively). In accordance with this aspect of the invention, vectors may be engineered to encode components of the gene targeting system of the invention, such as the gene targeting substrate flanked by the initiator and terminator sequences and the Rep factor(s) expressed by an appropriate promoter. In some embodiments of the claimed methods, the gene targeting transformation construct may be transferred into target cells by various chemical or physical means known in the art. As with plants, expression of Rep factor(s) in concert with host replication functions may result in production, release and accumulation of gene targeting cassette *in vivo* and *in nucleo* as in the appended claims, and gene targeting substrates may be acted upon by host nucleic acid recombination and/or repair functions to transfer the encoded information to the target genomic locus.

In various embodiments, alteration of one or both alleles in a diploid genome or multiple alleles in a polyploid genome may for example be achieved by the invention. Modified alleles may also be identified using various types of molecular markers as known in the art.

In animals, if it is desired for the modified target locus to be passed in whole organisms and heritable by sexual progeny then specialised cell types are generally initially used [15;17]. Stem cells can for example be transformed with the gene targeting construct and the target locus modified as described above. Stem cells with the modified target locus may then be used to create chimeric animals by adaptation of known procedures [15;17]. Some of these animals may then be able to transfer the modified target locus to their sexual progeny. Alternatively, procedures are known for the cloning of animals using somatic cells [94]. These somatic cells could have a target locus modified using the invention. The cells encoding the modified target locus could then be used for development of the cloned animal. Progeny from this animal could then encode the modified target locus and stably transfer it to sexual progeny or those progeny derived from repeating the cloning process.

Another mechanism for generating a heritable modified targeted genomic locus may be to perform the gene targeting in gametes or gonadal cells capable of differentiating into gametes. Gametes could be collected and treated *in vitro* with the gene targeting construct. The resultant production of gene targeting substrate *in vivo,* in concert with host functions, may result in genetic modification of the target locus. Such gametes could then be used in fertilization. The resultant zygote and organism may thus carry the modified locus in all of its cells and be capable of passing it to progeny. Gametes may also be modified *in situ* by using a gene targeting construct capable of systemic spread through the host and entry into host cells, particularly the germ-line and derivatives, or by direct application or injection of the gene targeting construct to gametes or gonadal cells differentiating into gametes. In such an embodiment, gametes or germ-line cells may take up the construct. The gene targeting substrate may then be produced *in vivo* to facilitate the desired change to the target locus in these cells. The gametes upon fertilization would thus result in an organism carrying the modified locus in all of its cells and may be capable of passing it to progeny. Methods of treatment of gonadal cells with exogenous gene targeting substrate may be adapted for use in alternative aspects of the present invention. The claimed methods of the invention may not apply for modifying the germ line identity of human beings. The claimed methods of the invention may be not be used for treatment of the human or animal body by surgery or therapy. In addittion to production of gene targeting substrates *in vivo* in the host cell or host organism which is to be modified, in alternative embodiments the invention may be adapted to produce gene targeting substrate in an heterologous system for use in the host cell or organism which is desired to be modified. For example, a gene targeting construct may first be created encoding the gene targeting cassette flanked by initiation and termination sequences. This construct may then be placed in a host expressing Rep factor(s), such as a bacterium like *E. coli.* In conjunction with host functions, the gene targeting substrate is thereby produced. This system may be adapted to provide a mechanism for producing small to large quantities of the gene targeting substrate. The gene targeting substrate may then be isolated, and if necessary, purified by standard techniques. The gene targeting substrate can then be transferred into desired plant, animal, or other eukaryotic or prokaryotic cells by various chemical or physical treatments known in the art to achieve a targeted genetic alteration in the host cells or organisms. In some embodiments, transfer of the gene targeting substrate to the nucleus may be enhanced by covalently or non-covalently binding a polypeptide sequence encoding a nuclear localization sequence to the gene targeting substrate. For example, a nuclear localization polypeptide may by added to the gene targeting substrate before applying it to the cells, or the polypeptide may be expressed within the host cells. Once in the nucleus the gene targeting substrate will, in conjunction with host nucleic acid recombination and/or repair functions, transfer the information to the target genomic locus.

Some embodiments of the invention involve adaptations of rolling-circle DNA replication (RCR), to replicate gene targeting substrates. Various forms of RCR occur in a variety of prokaryotic and eukaryotic genetic elements [95-103]. Two components common to a variety of RCR processes are: 1) a gene encoding a rolling circle replication protein; and 2) a DNA sequence (replication initiator sequence) encoding a rolling circle replication protein recognition and nicking site where DNA replication is initiated (a replication origin). Additional components of RCR may include DNA sequences in the replication initiator sequence that are recognized by accessory proteins which affect rolling circle replication protein function and may be encoded by the rolling circle replication element or the host cells [97;101;104]. Rolling circle replication protein can act to initiate and terminate DNA replication, as follows. Rolling circle replication protein first binds to a sequence within the replication initiator sequence and then catalyses nicking (i.e. cleavage) of a single strand of the dsDNA molecule. This activity may be defined as "nickase" activity (i.e. a protein that catalyzes nicking of a dsDNA molecule). Rolling circle replication proteins from various systems have motifs conserved with topoisomerases and these sequences are reportedly involved in the catalytic activities of this family of proteins[55], The nicking exposes a 3'-hydroxyl group on one strand of the DNA which can then act as a primer for DNA synthesis, which may for example be mediated by host cell factors. DNA synthesis proceeds using the non-nicked strand as template and this procession displaces the nicked strand. When one unit of a reproducible sequence has been replicated and the rolling circle replication protein recognition sequence is next encountered, acting as a replication terminator sequence, the rolling circle replication protein acts to cleave the displaced single-strand DNA (ssDNA). In addition, rolling circle replication protein may covalently join or ligate together the two ends of the released ssDNA copy of the reproduced sequence. Thus, in some embodiments, a closed circular ssDNA copy of a reproducible genetic element may be released while the dsDNA molecule is regenerated to undergo another cycle of RCR By concurrently regenerating the initial dsDNA molecule, numerous ssDNA copies of DNA sequence may be generated by subsequent cycles of RCR of a single copy of the dsDNA molecule. In some embodiments, the present invention utilizes this ability to amplify the number of copies of a DNA sequence from a single initial reproducible sequence, for producing gene targeting substrate.

In various embodiments of the claimed methods, a DNA cassette may be assembled which has two copies of the rolling circle replication protein recognition and nicking sequence, one acting as a replication initiator sequence and one acting as a replication terminator sequence, flanking each side of a reproducible DNA sequence that encodes a gene targeting substrate. The gene encoding rolling circle replication protein may also be cloned and placed between appropriate transcription and translation initiation and termination signals. Genes encoding accessory proteins deemed necessary for appropriate rolling circle replication protein function are also cloned and placed between appropriate transcription and translation initiation and termination signals. The system components, and genes encoding appropriate accessory proteins, as necessary, may then be cloned into a transformation vector which may either integrate into a host chromosome or remain extrachromosomal. Functional expression of rolling circle replication protein and necessary accessory protein(s) in the host cell may initiate production of gene targeting substrate. Rolling circle replication protein may cause a nick (i.e. cleave a single strand of a dsDNA molecule) within a replication initiator sequence. This will expose a 3'-hydroxy group which may act as a primer for DNA synthesis by host cell factors. DNA synthesis may displace a ssDNA copy of the reproducible sequence encoding the gene targeting substrate and may regenerate the dsDNA sequence encoding the gene targeting substrate. When DNA synthesis proceeds to the second rolling circle replication protein recognition/binding and nicking sites, rolling circle replication protein will act again and cleave the displaced ssDNA. Rolling circle replication protein may also covalently join the two ends of the released ssDNA molecule to create a closed circular ssDNA molecule. Thus a ssDNA copy of the reproducible sequence encoding the gene targeting substrate may be created and released, and the dsDNA form of that sequence may be regenerated Rolling circle replication protein may then again act to initiate replication of another ssDNA copy of the reproducible dsDNA sequence encoding the gene targeting substrate. This process of synthesis and regeneration may continue cycling thereby creating *in vivo* multiple copies of gene targeting substrate from the single initial copy. If the system components are in the cell nucleus, then multiple copies of then gene targeting substrate may be produced *in nucleo.* In various aspects, the methods of the invention may be adapted to work in plants, animals, lower eukaryotes, and prokaryotes.

In alternative embodiments of the claimed methods of the invention, a DNA cassette may be assembled as outlined above but having a single copy of the rolling circle replication protein recognition and nicking sequence adjacent to the reproducible sequence that encodes a gene targeting substrate. The genes encoding the rolling circle replication protein and accessory proteins, as necessary, are placed between appropriate transcription and translation initiation and termination sequences. The system components are cloned into a transformation vector which may integrate into a host chromosome or remain extrachromsomal. Functional expression of rolling circle replication protein and necessary accessory proteins may cause a nick within the replication initiation sequence. A 3'-hydroxyl may thus be exposed which may act as a primer for DNA synthesis. DNA synthesis may displace a ssDNA copy of the reproducible sequence encoding the gene targeting substrate and may regenerate the sequence encoding the gene targeting substrate into dsDNA. DNA synthesis may proceed until a sequence in the host chromosome, or in the extrachromosomal element encoding the gene targeting cassette, downstream from the reproducible sequence encoding the gene targeting substrate is encountered which may cause dissolution of the replication fork initiated at the rolling circle replication protein recognition and nicking sequence and may result in release of the displaced ssDNA strand. The ssDNA copy of the reproducible sequence and adjacent sequences encoded by the chromosome or extrachromosomal element may then act as a gene targeting substrate while the dsDNA form of that sequence may be regenerated. Rolling circle replication protein may then again act to initiate replication of another ssDNA copy of the reproducible dsDNA sequence encoding the gene targeting substrate. This process of synthesis and regeneration may continue cycling thereby creating *in vivo* multiple copies of gene targeting substrate from the single initial copy. If the system components are in the cell nucleus, then multiple copies of the gene targeting substrate will be produced *in nucleo.*

In alternative embodiments of the methods of the invention, the reproducible sequence encoding the gene targeting substrate may be flanked on one side by the recognition and nicking sequence for one type of rolling circle replication protein and flanked on the other side by the recognition and nicking sequence for another type of rolling circle replication protein. One of these recognition and nicking sequences is oriented for it to function as an initiator sequence and the other as a terminator sequence. The alternative types of rolling circle replication proteins may be mutant forms of the same protein or rolling circle replication proteins from different prokaryotic or eukaryotic genetic elements.

In alternative embodiments of the methods of the invention, two rolling circle replication proteins may be engineered to be encoded as a single polypeptide (i.e. a fusion protein) which may be able to bind and cleave DNA sequences which encode the recognition and nicking sequences for the two respective rolling circle replication protein constituents of the fusion protein. In some embodiments of the methods of the invention the genes encoding either of the two types of rolling circle replication proteins or the fusion protein encoding the functions of two types of rolling circle replication proteins are expressed in a cell containing the reproducible sequence encoding the gene targeting cassette flanked by the recognition and nicking sequences for the two types of rolling circle replication proteins (one recognition and nicking sequence is oriented to act as an initiator and the other as a terminator). The initiator sequence is recognized and nicked by one type of rolling circle replication protein or the respective domain of the fusion protein. This may expose a 3'-hydroxyl group which may act as a primer for DNA synthesis by host cell factors. DNA synthesis may displace a ssDNA copy of the reproducible sequence encoding the gene targeting substrate and may regenerate the dsDNA sequence encoding the gene targeting substrate. When DNA synthesis proceeds to the second rolling circle replication protein recognition and nicking sites, the second type of rolling circle replication protein or the second domain of the fusion protein may act to cleave the displaced ssDNA. Thus a ssDNA copy of the reproducible sequence encoding the gene targeting substrate may be created and released, and the dsDNA form of that sequence may be regenerated. Rolling circle replication protein may then again act to initiate replication of another ssDNA copy of the reproducible dsDNA sequence encoding the gene targeting substrate. This process of synthesis and regeneration may continue cycling thereby creating *in vivo* multiple copies of gene targeting substrate from the single initial copy. If the system components are in the cell nucleus, then multiple copies of the gene targeting substrate may be produced *in nucleo.*

In alternative embodiments of the methods of the invention, a rolling circle replication protein and accessory protein(s) may be engineered to be encoded as a single polypeptide (i.e. a fusion protein). The accessory protein(s) may enhance the activity of the rolling circle replication protein. The accessory protein(s) may be encoded by the genetic element encoding the rolling circle replication protein or be encoded by the host. RCR and related processes have been very well characterized in numerous systems and the essential components required to facilitate these types of DNA replication have been defined. Thus the invention may be achieved by employing various well characterized components from these systems, a non-exclusive list of which includes:
1) prokaryotic viruses including those with circular genomes such as filamentous phage including F-specific types like fd, fl, M13 [95], N-specific phage like Ike [95], and others including ZJ/2, Ec9, AE2, HR, Ifl, If2, X, v6, Pf3, Pf2 and Cf [95]; isometric ssDNA phage like φX174, S13, and G4 [96]; and others like St-1 [105], α-3 [105;106], G4 [107], G14 [106], U3 [106], and phasyl [108];
2) plant viruses including gemini viruses the three families of which are represented by Wheat Dwarf Virus, Maize Streak Virus (WDV; MSV; mastrevirus), Beet Curly Top Virus (BCTVcurtovirus), Tomato Yellow Leaf Curl Virus (TYLCV) and Tomato Leaf Curl Virus (TLCV; begomovirus)[99; 245]; and circoviruses or nanoviruses like banana bunchy top virus [109;110], subterranean clover virus [111] and coconut foliar decay virus [112];
3) Animal viruses including circoviruses like porcine circovirus [100], chicken anemia virus [113], psittacine beak and feather disease virus [114]; and parvoviruses [113] like adeno-associated virus [103;115;116], and minute virus of mice [102;117];
4) Plasmids including pC194 [118;119], pT181 [120;121], pUB110 [122], pCA2.4 [123], pE194 [124], pKYM [125;126], and others[97;127-129];
5) Conjugation DNA transfer systems including F-factor [130] and various broad-host range plasmids, such as those from the approximately twenty different incompatibility groups identified to date like IncW (R388; [131]), IncP (RP4, R751; [132;133]), IncQ (RSF1410; [134]), IncN (R46; [135]), IncF (ColB4, [136]), and IncI (R64; [137]) and other plasmids as reviewed by Pansegrau and Lanka (1996), as well as conjugative transposons like Tn4399 [138;139]. Some plasmids are mobilizable by conjugation with helper functions supplied in trans including ColE1 plasmids [140;141], CloDF13 [142] and pSC101 [143].

Of the prokaryotic viruses using RCR to amplify their genomes, two which have been extensively characterized are the filamentous phage group including fd, f1 and M13 [95;144], and the isometric ssDNA phage group including φX174 [96;145]. In, various aspects of the invention, such viruses may provide components that may be incorporated in alternative embodiments of the invention. In some embodiments, two components from these viruses may be required for their replication *in vitro* or in heterologous arrangements: rolling circle replication protein and origin (rolling circle replication protein recognition) sequence [146-148]. The filamentous phage rolling circle replication protein is encoded by viral gene II [96;146;147;149] and is referred to as g2p (gene II protein). φX174 rolling circle replication protein is encoded by viral gene A [96; 150] and is referred to as XpA. A derivative of XpA, XpA*, containing the carboxyl-terminal 341 amino acids of XpA has similar catalytic properties as XpA [151] and may also be used in alternative embodiments of the invention. These proteins have been characterized extensively for their enzymatic properties -148;152-159]. The respective rolling circle replication protein recognition (origin) sequences are encoded within an approximately 450 bp intergenic region of filamentous phage [160;161]and by 280-500 bp in φX174 [162;163], but minimal functional sequences have been defined as approximately 40 bp [164] and approximately 30 bp [156;162], respectively. Derivatives of origin sequences may still function effectively in facilitating RCR [150;165;166]. Such derivatives of origin sequences may be used in alternative embodiments of this tion as replication initiator sequences.

The viral components that may be used in the invention including rolling circle replication protein and the origin (replication initiator and terminator) sequence, may be used in heterologous systems like eukaryotic cells. Prokaryotic viral rolling circle replication protein and its cognate origin sequences may also be used in eukaryotes.

In alternative embodiments, proteins such as replication factors and accessory proteins may be adapted for use in the invention by addition of nuclear localization sequences. By promoting localization of the proteins to the eukaryotic nucleus the production of gene targeting substrate *in nucleo* may be enhanced. RCR is used by plant viruses as exemplified by the Geminidae family [99;104]. This family has three main groups known as Mastrevirus, Curtovirus, and Begomovirus, and may be represented here by WDV and MSV, BCTV, and TYLCV and TLCV, respectively[99; 245]. The rolling circle replication proteins of gemini viruses have been cloned and undergone extensive molecular and biochemical characterization [104;174-181]. Gemini virus rolling circle replication proteins share extensive functional and structural features [104] and have the conserved sequence motifs found in the topoisomerase-like rolling circle replication proteins and nickases of other types of replicons using RCR [55]. Despite the degree of conservation amongst Gemini virus rolling circle replication proteins, the proteins retain specificity regarding interactions with the origin sequences of their respective viral genomes [175;182]. However, hybrid rolling circle replication proteins can be engineered to have modified catalytic activity and substrate specificity [183], and such modified rolling circle replication proteins may also be used in alternative embodiments of the invention. Gemini virus rolling circle replication proteins may maintain their acitivity and specificity when expressed in heterologous organisms [110;174;176;177;180;184;185]. The rolling circle replication protein binding site in the gemini virus genome and the sequence that is nicked by rolling circle replication protein is found in the origin of RCR within a DNA sequence known as the intergenic region [104]. As little as 13 bp can act as a binding site for rolling circle replication protein [186] and minimal DNA sequences which are cleaved by rolling circle replication protein *in vitro* range from 23-66 nucleotides [110;174;176;179]. In vivo analysis to date has shown maximum origin function when the entire intergenic region is used [187], which, for example, in the case of WDV is approximately 410 bp [187;188], TYLCV is approximately 300 bp [183;189], and TLCV is approximately 340 bp [185;190]. Smaller fragments of the intergenic region may still function effectively in facilitating RCR [187], and such derivatives of the intergenic region may also be used in alternative embodiments of this invention.

RCR is also used by a family of viruses known as Circoviridae which includes examples of both animal and plant viruses [100]. Porcine circovirus (PCV) has been characterised extensively [100] and provides an example of the components of RCR that may be adapted for use in the invention. PCV encodes a rolling circle replication protein which has been cloned and found able to act in trans to catalyse initiation of DNA replication [191]. The origin sequence of PCV which encodes the rolling circle replication protein binding and cleavage/nicking sites has been cloned and defined as an 111 bp fragment [192], although alternative sized fragments may also function in initiating or terminating replication in accordance with alternative embodiments of the invention to facilitate replication in the context of heterologous DNA sequences to generate gene targeting substrate *in vivo.*

RCR plasmid replication systems are known in a wide variety of prokaryotes [97;127;128], as well as in eukaryotes including plants [193]. These plasmids may have the conserved features of other RCR systems, including a rolling circle replication protein which interacts with a specific recognition sequence in the cognate DNA molecule and catalyses formation of a nick [97;129]. Rolling circle replication proteins cloned and characterized from various plasmids [118;120;123;125] have many conserved features [97] and may have topoisomerase-like activity and nickase activity [120]. The corresponding DNA sequences which the rolling circle replication proteins bind and cleave/nick, to initiate and terminate RCR, have also been identified [97]. The size of functional origin sequences may vary between plasmids and has, for example, so far been delineated as 127 bp for pT181 [120], 55 bp for pC194 [194], and 173 bp for pKYM [126]. In alternative embodiments of the invention, reduced or enlarged sequences may for example be effective or optimal for replication initiator or replication terminator function in the context of heterologous DNA sequences when a reproducible DNA sequence is flanked by copies of an origin sequence, and the rolling circle replication protein is supplied in trans, so that the reproducible sequence is amplified and released as a gene targeting DNA substrate molecule.

In alternative embodiments, the action of proteins active in replication methods of the invention may be enhanced by addition of nuclear localization sequences. By promoting localization of the proteins to the eukaryotic nucleus the production of gene targeting substrate *in nucleo* may be enhanced.

RCR is also known to be involved in intercellular DNA transfer systems, such as conjugation, which facilitate transfer of genetic information between cells. Intercellular DNA transfer commonly occurs amongst bacterial cells of the same or different species [101;195]. Trans-kingdom transfer of genetic material may also occur between bacterial and eukaryotic cells including plants [196], animals [43] and fungi [197]. Conjugation-mediated DNA transfer processes typically rely on the presence of a rolling circle replication protein-like protein, known as a DNA-relaxase, and its cognate binding and cleavage sites within a DNA sequence, such as oriT [101;198]. In typical conjugation-mediated DNA transfer processes, relaxase binds a plasmid and cleaves a single-strand within oriT where the relaxase protein may become covalently linked to the 5'-end of the cleaved plasmid. This process may be assisted by plasmid encoded accessory proteins, which may also be used in alternative embodiments of the methods of the present invention. The revealed 3'-hydroxyl group may then act as a primer for DNA synthesis catalysed by host factors. DNA synthesis displaces the relaxase-bound strand and regenerates the dsDNA plasmid molecule [101;198], in a process that is analogous to RCR in the systems described above. In conjugation, by the action of a series of proteins and cell structures, the displaced strand is transferred into the recipient cell [101;195]. In conjugation, when DNA synthesis displaces an entire single-stranded copy of the DNA molecule located in the donor cell, relaxase cleaves the DNA at oriT and covalently joins the ends together creating and releasing a closed-circular ssDNA copy of the initial dsDNA molecule [101;198]. In some systems the ends of the ssDNA molecule transferred to the recipient cell may not be covalently joined. The conjugation DNA replication systems may be used in alternative embodiments of the invention in methods analogous to the methods employing RCR-like replication mechanisms, including components of the transfer systems, and may be used to achieve replication of a gene targeting substrates *in vivo* in accordance with the present invention as set out in the claims. A non-exclusive list of such DNA conjugation systems include: F-plasmid of *Escherichia coli*[130]; and broad-host range plasmids from the approximately twenty incompatibility groups identified to date like IncW (R388; [131]), IncP (RP4, R751; [132;133]), IncQ (RSF1010; [134]), IncN (R46; [135]), IncF (ColB4, [136]), and IncI (R64; [137]) and other plasmids as reviewed by Pansegrau and Lanka (1996), as well as conjugative transposons like Tn4399 [138;139], and some plasmids are mobilizable by conjugation with helper functions supplied in trans including ColE1 plasmids [140;141], CloDF13 [142] and pSC101 [143]. The rolling circle replication protein-like DNA-relaxase proteins from several DNA transfer systems have been cloned and extensively characterized [198] including: TrwC from R388 [199-202]; TraI from RP4 [132;203]; MobA from RSF1010 [204;205]; TraI from F-plasmid [206;207]; NikB from R64 [137] and MocA from Tn4399 [138]. The activity of DNA-relaxase proteins in binding and cleaving oriT sequences may be enhanced by accessory proteins including: TrwA and TrwB from R388 [208;209]; TraG, TraJ, Trash and TraK from RP4 [101;210]; MobB and MobC from RSF1010 [205]; TraY and TraM from F-plasmid [211]; NikA from R64 [137]; IHF [211], MocB from Tn4399 [138] and analogous proteins from other systems. The oriT sequences that may be used for initiating DNA synthesis in concert with DNA-relaxase function have been defined for conjugal transfer plasmids and correspond to approximately 402 bp for R388 [131], 350 bp for RP4 [133], 574 bp for R751 [133] and approximately 1 kb for F-plasmid [211]. In alternative embodiments of the invention, reduced or altered sequences may also function as origins, such as 50 bp for R388 [202], 200 bp for RP4 [133], and 38 bp for RSF1010 [212]. In alternative embodiments of the invention, oriT sequences from conjugal transfer systems may be used with a DNA-relaxase that is supplied in trans. In alternative embodiments, the action of conjugation system proteins in the invention may be enhanced by addition of nuclear localization sequences.

In alternative embodiments, transposition systems may be adapted for use as *in vivo* gene targeting substrate replication systems in the methods of the invention as claimed. Transposable elements are discrete segments of nucleic acid which can move from one locus to another in the host genomes or between different genomes [213-215; 224; 225]. They exist in both prokaryotes and eukaryotes and are common to most species. Transposable elements propagate by amplifying themselves and moving to other sites in the genome. They can then be dispersed to new cells and through a population by various means of horizontal or vertical transfer of genetic information which results in transfer of a fragment of DNA containing a copy of a transposable element to a new cell. The transposable element can then amplify and move to new sites in this cell.

The successful dispersal of a transposable element in a population partly relies on its ability to transpose or move to new sites in a genome. Transposable elements may be grouped on the basis of the mechanism used for transposition. One group uses conservative or cut-and-paste transposition whereby the transposon is excised from the donor site and reinserted into a target site without replication of itself [213;215]. This process may generally involve cleavage of both strands of the DNA strands at the end of the element and insertion at a target DNA site. Another group,of transposons uses replicative transposition whereby the transposon becomes copied resulting in a copy at the original site and a new copy at the new target DNA site [213;215]. This process typically involves nicking of only a single strand of the DNA at the end of the element and transfer to a second site in a way that creates a replication fork resulting in duplication of the element and resolving the two copies creating insertions at the first and new site. Another group of transposable elements called insertion sequences, including members of the IS91 family like IS1294 and IS801 [225], transpose using a rolling-circle replication mechanism. Another group of transposable elements called retrotransposons use an RNA intermediate during transposition [237].

Transposition typically results in integration of the element at random sites in the genome. This has important implications for the host genome and affects the fate of the host cell and, therefore, the transposable element itself by generating mutations which may be advantageous or detrimental for the host cell [215]. As a result, transposable elements have been used successfully to generate random mutations in prokaryotic and eukaryotic species to facilitate characterizing gene function, gene identification and gene cloning [215-217].

The success of dissemination of a transposable element in a population is typically linked to its integration at random sites in the genome, which may act to enhance the probability that some DNA fragment containing a copy of the transposon will be transferred to a new cell. Thus, transposable elements have evolved mechanisms to achieve random integration and to avoid homologous recombination. Random integration of transposons may be linked to the DNA affinity of the central enzyme mediating transposition, transposase (sometimes referred to as an integrase), and affiliated proteins also encoded by a transposable element [213-215; 225; 237]. Transposase enzymes generally have two functional domains: 1) a specific DNA-binding domain which recognizes and binds a specific sequence in the terminal repeat region of the transposable element which acts to correctly place transposase; and, 2) the catalytic domain which catalyses either a single-stranded nick or double-stranded cleavage, depending on the species of transposable element, of the DNA flanking the transposable element [215; 225]. Transposases may also have a third domain near the active site which has non-specific DNA-binding ability. Through this non-specific DNA binding, the transposase may facilitate transfer of the transposable element from the initial site to a random site in the host genome [215]. Alternatively, transposable elements may encode a transposase recruiting protein which is responsible for random integration acting in concert with transposase. This recruiting protein binds DNA at random sites in the genome and then physically interacts with (i.e. recruits) transposase to facilitate transfer of the transposable element into the site at which the recruiting protein is bound [214].

Perhaps because insertion of a transposable element into another copy of itself would be suicidal in the context of limiting propagation of the transposable element, many transposable elements have evolved molecular means to prevent integration into DNA homologous to itself. This process of "target immunity" has been well defined biochemically [214].

There have been reports that transposons have been successful for specifying integration of DNA fragments only near a desired target site [216]. In this process of transposable element "homing", a transposable element is engineered to contain a DNA fragment homologous to a target locus. When the engineered transposable element undergoes transposition its integration at a new genome location shows some preference for the target locus with which the engineered transposable element has homology. However, the target locus is not replaced by the transposable element or the homologous DNA carried by the element. Rather the engineered transposable element integrates adjacent to the target locus. In addition, the position of the integration varies with some integration sites being distributed over 200 kb around the target locus, and these integration sites may not be predictable [216]. At least in some cases, the enrichment of insertions is thought not to result from homologous pairing involving homologous recombination processes, but is rather thought to be a result of the DNA fragment contained in the engineered transposable element containing recognition sites for DNA-binding proteins [216], with interactions between DNA-binding proteins associated with recognition sequences in the genomic locus and the DNA fragment in the engineered transposable element being proposed to recruit the engineered transposable element and enrich for its integration adjacent to the target locus [216]. In summary, although transposable elements can amplify themselves *in vivo* and be engineered to carry foreign DNA, they are generally unsuitable for gene targeting because of their inherent nature to insert at random sites in the genome and have specific molecular mechanisms to inhibit integration and replacement of homologous sequences in the genome.

In alternative embodiments of the methods of the invention, components of transposition systems may be adapted for use in the invention. Transposases from various transposable elements are capable of catalysing single-stranded nicks to release a 3'-hydroxyl group which can be used to prime DNA synthesis. In addition, the transposase recognizes and binds specific DNA sequences before catalysing the adjacent nick. In one aspect of the invention, the recognition sequence for a transposase may be placed adjacent to the reproducible sequence encoding the gene targeting substrate, to act as a replication initiator sequence. Expression of the transposase may thus result in specific nicking adjacent to the reproducible sequence. The resultant 3'-hydroxyl group may act as a primer for DNA replication machinery which will then replicate the reproducible DNA sequence encoding the gene targeting substrate. The displaced replicated strand may then act as a gene targeting substrate. The gene targeting cassette may be regenerated so that by action of the transposase and replication machinery, another molecule of the gene targeting substrate may be produced. This series of events can be repeated through subsequent cycles to generate multiple copies of the gene targeting substrate *in vivo.* In alternative embodiments of the claimed methods of the invention the primer for initiating replication of the reproducible sequence encoding the gene targeting substrate may be an RNA molecule. RNA molecules are a natural component of DNA replication systems for a variety of genetic elements including eukaryotic and prokaryotic chromosomes, plasmids and viruses where the RNA molecule provides a 3'-hydroxyl group to prime DNA synthesis. In one aspect of the invention the RNA molecule is created by a primase. The primase may be recruited to a sequence adjacent to the reproducible sequence to create a RNA primer and initiate DNA replication of the reproducible sequence. In alternative embodiments a primase may be engineered to encode a domain with the capability of recognizing a specific DNA sequence. This recognition sequence may be encoded adjacent to the reproducible sequence. In this manner, the recognition sequence may recruit the primase to create a RNA primer adjacent to the reproducible sequence and initiate replication of the reproducible sequence. In alternative embodiments, the primase may be recruited to the reproducible sequence by interacting with a second 'recruitment' protein which encodes a DNA binding domain and is capable of protein-protein interactions with the primase or a primase complex. The DNA sequence recognized by the recruitment protein is encoded adjacent to the reproducible sequence so that it may place the primase in an appropriate context to create a primer and facilitate initiation of DNA replication of the reproducible sequence. In alternative embodiments, a primase which naturally encodes a domain with the capability of recognizing specific DNA sequence may be employed. A non-exclusive example of such a primase is the alpha protein of phage P4 [219]. The alpha protein recognition sequence may be encoded adjacent to the reproducible sequence so that it may place the alpha protein primase in an appropriate context to create a primer and facilitate initiation of DNA replication of the reproducible sequence.

In alternative embodiments of the claimed methods of the invention the primer for initiating replication of the reproducible sequence encoding the gene targeting substrate may be an RNA molecule resulting from transcription catalysed by RNA polymerase. This transcript binds to a specific DNA sequence adjacent to the reproducible sequence encoding the gene targeting cassette to act as a primer of DNA replication enabling production of the gene targeting substrate. RNA transcripts are known to act as primers of DNA replication in a number of biological systems including ori(34) and ori(uvsY) of bacteriophage T4, ColEl episome, and oriK of the E. coli chromosome [238]. In these systems an RNA transcript is synthesized by host RNA polymerase and then binds to a specific site on the replicon to form a persistent RNA-DNA hybrid. The RNA transcript within this hybrid can act as a primer for DNA polymerase to perform DNA synthesis at the 3'-end of the RNA transcript generated by RNA polymerase or by the action of RNase [238]. To apply these elements to develop a gene targeting system a DNA construct would be assembled whereby a cassette encoding the reproducible DNA sequence encoding the gene targeting substrate is linked to an adjacent initiator sequence. This initiator sequence may incorporate a DNA unwinding element (DUE) which is a DNA sequence that may act to promote the formation and/or stability of RNA-DNA hybrids [238]. This DNA construct may also encode a sequence comprising a promoter linked to a sequence encoding a primer. When this promoter is active it will transcribe the adjacent sequence to create an RNA molecule which can hybridise to the initiator sequence and form an RNA-DNA hybrid. In alternative embodiments the promoter and primer encoding sequence may be on a separate construct already present and expressed in the cell or genome of the cell to be modified by the gene targeting substrate. The transcript forming the RNA-DNA hybrid at the initiator sequence can act directly as a primer for the DNA replication machinery to replicate the adjacent sequence to produce copies of the gene targeting substrate. Alternatively, the RNA-DNA hybrid may be processed by host enzymes, for example RNase, to create an appropriate 3'-end of the RNA molecule to efficiently function as a primer for replication of the reproducible sequence to produce gene targeting substrate. This process may be repeated multiple times to produce multiple copies of the gene targeting substrate which can facilitate genetic alteration of the target locus in the host genomes. In alternative embodiments of the claimed methods of the invention the primer for initiating replication of the reproducible sequence encoding the gene targeting substrate may be a protein molecule. Placement of certain amino acid residues of a protein in appropriate context with reference to a nucleic acid molecule may facilitate priming of replication of the nucleic acid molecule [220]. In some aspects of the invention a protein encoding an amino acid residue which may act to prime DNA synthesis (i.e. a primer protein) is engineered to encode a DNA-binding domain. A DNA sequence to which this protein may bind may be encoded adjacent to the reproducible sequence encoding the gene targeting substrate. In this manner the recognition sequence may recruit the primer protein to facilitate initiation of DNA replication of the reproducible sequence. DNA replication may be facilitated by an endogenous or heterologous DNA polymerase. In alternative embodiments, the protein encoding the priming amino acid residue may be recruited to the reproducible sequence by interacting with a second 'recruitment' protein which encodes a DNA binding domain and is capable of protein-protein interactions with the primer protein. The DNA sequence recognized by the recruitment protein is encoded adjacent to the reproducible sequence so that it may place the primer protein in an appropriate context to facilitate initiation of DNA replication of the reproducible sequence. DNA replication may be facilitated by an endogenous or heterologous DNA polymerase.

In some embodiments of the claimed methods the efficiency of replicating the reproducible sequence encoding the gene targeting cassette may be increase by linking a DNA unwinding element (DUE) to the initiator sequence. DUE sequences have nucleotide compositions that confer an inherent ability to unwind the DNA double helix. DUE sequences are commonly associated with DNA replication origins functional in prokaryotic and eukaryotic organisms [238;252-254]. Because of the tendency to promote DNA unwinding, DUE elements may be important components of prokaryotic and eukaryotic replication origins to enable efficient initiation of DNA replication [238;252-254]. Several DUE sequences have been identified and characterised [238;252-254] and such seqeunces may be identified by computer analysis of DNA sequences [255]. In some embodiments a DUE sequence is linked to the initiator sequence of the reproducible sequence encoding the gene targeting substrate so as to increase the efficiency of replication of the reproducible sequence. An example of a DUE sequence well characterised and applicable to the invention is the ∼100 bp DUE sequence from the ARS307 (also know as ARS C2G1) replication origin from Saccharomyces cerevisiae [253]. This seqeunce may be amplified by PCR and cloned adjacent to the initiator sequence derived from, for example, φfd, φX174. or TYLCV embodied here to promote replication of the adjacent sequence encoding a gene targeting substrate. In other embodiments, computer or biochemical or physical analysis of prokaryotic or eukaryotic viral or genomic DNA sequences may provide DUE-like sequences that may be used to promote replication of the reproducible sequence encoding a gene targeting substrate. In further alternative embodiments, a transcriptional promoter may be operatively linked with the initiator sequence, so that transcription proceeds from the promoter through the replication initiator sequence. In some embodiments, this may enhance the accessability of the initiator sequence to replication factors. In further alternative embodiments, transcription factor recognition sites may be operatively linked with the initiator sequence, such that binding of such recognition sites by transcription factors may enhance the accessibility of the initiator sequence to replication factors. In further alternative embodiments, nucleosomes associated with the initiator site may be dissociated by the action of acetylating, methylating or phophorylating histones to enhance accessibility of the initiator sequence to replication factors.

### EXAMPLE 1

### Cloning and evaluation of genes

Genes and genetic elements of interest were cloned using specific oligonucleotides designed to prime DNA synthesis in a PCR reaction with either cDNA or genomic DNA (gDNA) from the appropriate species as template. The primers were designed to incorporate convenient restriction sites into the amplicon to facilitate initial cloning of the gene or genetic element and subsequent subcloning into various expression or analytical vectors. Genes and genetic elements cloned and the oligonucleotide primers used to achieve this are described in TABLE 1. PCR conditions were as described [256] or as recommended by the supplier of the thermostable DNA polymerase Pfu (Stratagene), Pfx (Gibco BRL) or Taq (Pharmacia) . PCR reactions were conducted using a thermocycler (Perkin-Elmer Model 9700). In some cases specific restriction fragments known to encode the gene or genetic element of interest, based on sequence information from genome databases, were directly cloned from complex mixtures of DNA fragments without any PCR amplification. In other cases, specific restriction fragments known to encode the gene or genetic element of interest based on restriction maps of plasmids encoding the desired components were subcloned into other vectors for various applications. DNA sequence of clones was determined at a commercial sequencing facility (National Research Council Plant Biotechnology Institute, Saskatoon, Canada).

**TABLE 1: Oligonucleotides for amplifying and modifying target genes**

| Oligo name | Target Gene | Sequence (5'-3') |
|---|---|---|
| fdg2-5'RI | g2p | GGGGAATTCATGATTGACATGCTAGTTTTACG |
| fdg2-5'Sma | g2p | ATCCCCGGGATTGACATGCTAGTTTTACGAT |
| fdg2-3'Pst | g2p | GAACTGCAGTTATTATGCGATTTTAAGAACTGG |
| Init-5'BamPme | φfd initiator | GTAGGATCCGTTTAAACGCGCCCTGTAGCGGCG |
| Init-3'SacPac | φfd initiator | GGGCCGCGGTTAATTAATTGTAAACGTTAATATT TTGTT |
| Term-5'AscRV | φfd terminator | GTAGGCGCGCCGATATCGCGCCCTGTAGCGGCGC A |
| Term-3' SalNot | φfd terminator | GGGGTCGACGCGGCCGCTGAGTGTTGTTCCAGTT TGG |
| g2-5'Sfo | g2p | ATCGGCGCCATTGACATGCTAGTTTTACG |
| NLS-FLAG-Gly-sense | SV40NLS | GATCCAAAAAAATGGCTCCTAAGAAGAAGAGAAA GGTTAACGGTGATTACAAGGATGATGATGATAAG CCCGGGGGTGGAGGTGGAGGTGGAGGTGGAGGTG GAGGC |
| NLS-FLAG-Gly-antisense | SV40 NLS | GCCTCCACCTCCACCTCCACCTCCACCTCCACCC CCGGGCTTATCATCATCATCCTTGTAATCACCGT TAACCTTTCTCTTCTTCTTAGGAGCCATTTTTTT G |
| XpA*-5' SmaSfo | XpA* | CCCGGGGGCGCCATGAAATCGCGTAGAGGC |
| XpA-3'HIIINot | XpA* | CTCGAGAAGCTTGCGGCCGCTTATCATTTTCCGC CAGCAGTC |
| g2p-3'FLAG-Pst | g2p | ATCCTGCAGTTATTACTTATCATCATCATCCTTG TAATCACCGTTAACCTCATCTCTCTCGCG |
| g2p-3'Gly-SmaPst | g2p | ATCCTGCAGTTATTACCCGGGTCCACCTCCACCT CCACCTCCACCGGCGCCTGCGATTTTAAGAACTG GC |
| g2p-3'NLS-HpaPst | g2p | ATCCTGCAGTTATTAGTTAACCTCATCTCTCTCG CGTTTGCGTTCACTCGGTTCTCCATCATCATCTT CACGCGGACGCTTTGAAAGCCCGGGTCCACCTCC ACC |
| 3'Xori-URA | URA3 | GGGGTCGACGCGGCCGCGTGGTCTATAGTGTTAT TAATATCAAGTTGGATATCGGCGCGCCCCCGGGT AATAACTGATATAATT |
| 5'Xori-URA | URA3 | GTAGGATCCGTTTAAACAACTTGATATTAATAAC ACTATAGACCACTTAATTAACCGCGGATCGATCG AATTATCATTGAAATC |
| XpA-3'HIIINotSacSfo | XpA | GGGAAGCTTGCGGCCGCCTAGAGCTCTCATCAGG CGCCTTTTCCGCCAGCAGTCCAC |
| XpA-5'Sal-RBS-BamSma | XpA | GATATCGTCGACAAGGAGGATCCCGGATGGTTC GTTCTTATTACC |
| | | |
| XpA-Bind-Sense-Cla | XpA | AACAATACGATCGATCATCGCCCCGAAGGGGACG |
| XpA-Bind-Anti- Cla | XpA | GGGGCGATGATCGATCGTATTGTTTATGTTCAGC TGGGGGAGCACATTGTA |
| | | |
| XpA-INIT- 5'BamPme | φX174 on | ATCGGATCCGTTTAAACCGGCCATAAGGCTGCTT C |
| XpA-INIT- 3'PacMscSac | φX174 ori | ATCGAGCTCTGGCCATTAATTAAAGGCCTCCAGC AATCTTG |
| XpA-TERM- 5'XhoAscRV | φX174 ori | GTACTCGAGGGCGCGCCGATATCCGGCCATAAGG CTGCTTC |
| XpA-TERM- 3'NotSal | φX174 ori | GTAGTCGACGCGGCCGCGGCCTCCAGCAATCTTG |
| Mor-INIT- 3'SacMscPac | TYLCV ori | GTAGAGCTCTGGCCATTAATTAAATTGATGGTTT TTTCAAAACTTAG |
| Mor-TERM- 5'XhoAscRV | TYLCV ori | GTACTCGAGGCGCGCCGATATCTTGGTCAATGG GTACCAATT |
| Mor-C1- 5'SalRBSBam | TYLCV RepC1 | GATATCGTCGACAAGGAGGATCCCGGGATGGCTC AGCCTAAGCGT |
| Mor-C1-5'Bam | TYLCV RepC1 | ATCGGATCCAAAAAAATGGCTCAGCCTAAGCGT |
| Mor-C1- 3'NotXho | TYLCV RepC1 | ATCGCGGCCGCCTCGAGCTACTACGCCTCACTTG TCTCTTC |
| Mor-INIT- 5'BamPme | TYLCV ori | ATCGGATCCGTTTAAACTTGGTCAATGGGTACCA ATT |
| Mor-TERM- 3'XbaNot | TYLCV ori | GTATCTAGAGCGGCCGCATTGATGGTTTTTTCAA AACTTAG |
| WD-C1-5'Sal- RBS-BamNco | WDV RepC1 | GATATCGTCGACAAGGAGGATCCATGGCCTCTTC ATCTGC |
| WD-C1-3'NotPst | WDV RepC1 | ATCCTGCAGGCGGCCGCTCATCACTGCGAAGCAG TGAC |
| WD-C1-5'Bam | WDV RepC1 | ATCGGATCCATGGCCTCTTCATCTGC |
| | | |
| WDV-C1-Cterm- 5'+25bp-span | WDV RepC1 | CTGGAAAAATGAACATCTCTACTCCGAGTCACCG GGGAGGCAT |
| WDV-C1-Nterm- 3"+25bp-span | WDV RepC1 | TGGACTTATGCCTCCCCGGTGACTCGGAGTAGAG ATGTTCATTTTTCC |
| WD-INIT- 3'PacMscSac | WDV ori | ATCGAGCTCTGGCCATTAATTAACGAGATGGGCT ACCACGC |
| WD-INIT- 5'BamPme | WDV ori | ATCGGATCCGTTTAAACGGTAGTGAACAGAAGTC CGG |
| WD-TERM- 5'XhoAscRV | WDV ori | GTACTCGAGGGCGCGCCGATATCGGTAGTGAACA GAAGTCCGG |
| WD-TERM- 3'NotSal | WDV ori | GTAGTCGACGCGGCCGCCGAGATGGGCTACCACG C |
| H4-Prom- 5'KpnSac | Histone H4 promoter | ATCGGTACCGAGCTCGAAATATGAGTCGAGGCAT GGATAC |
| H4-Prom- 3'BamXho | Histone H4 promoter | ATCGGATCCTCTCGAGAGAAATTGATGTCTGTAG AAG |
| H4-Prom-3'X | Histone H4 promoter | AATCGCAGGCTTGGTGATTC |
| AtR51-Prom- 3'EX | AtRAD51 promoter | TGGACAGCATTCTGGTTTCTA |
| AtR51-Prom- 3'Xho | AtRAD51 promoter | ATCCTCGAGTTCTCTCAATCAGAGCAGATTC |
| AtR51-Prom-5'X | AtRAD51 promoter | AATTCTTTAGCAAGTGAATATGTTTTTCTT |
| AtR51-Prom- 5'Sac (-1.7 kb) | AtRAD51 promoter | ATCGAGCTCTAAATAAGTAAACAATTGACTTGCT TATAT |
| AtR51-Prom- 5'Sac (-1 kb) | AtRAD51 promoter | ATCGAGCTCATATATTTGATTAACATTTAGCGTC TACTAG |
| AtR51-Prom- 5'Sac (-0.7 kb) | AtRAD51 promoter | ATCGAGCTCGAAAATTGACAAATTTTGTGATATT TG |
| AtDMC-Prom- 3'BamRVXho | AtDMC1 promoter | GTAGGATCCGATATCCTCGAGTTTCTCGCTCTAA GACTCTCTAAG |
| AtDMC-Intron2- 3'NcoRV | AtDMC1 promoter | GTACCATGGCGATATCACCTCCTTCTTCAGCTCT ATGAATCCGAAAC |
| REP-5'Sal-RBS- BamSma | EcREP helicase | GATATCGTCGACAAGGAGGATCCCGGGATGCGTC TAAACCCCGGC |
| REP- 3'NotXhoSfo | EcREP helicase | ATCGCGGCCGCCTCGAGTCATTAGGCGCCTTTCC CTCGTTTTGCCGCCAT |
| DMC-Prom-S1 (3765) | AtDMC1 promoter | TGAGTTGTGAAGTGCTCTTA |
| DMC-Prom-S2 (4229) | AtDMC1 promoter | TTGGTTAAACTCCCCAACTT |
| AtR51-Prom- A1(1226) | AtRAD51 promoter | ACCGCCGAGAACCACCACAA |
| AtR51-Prom- A2(749) | AtRAD51 promoter | AACTAGTAGACGCTAAATGTTAATC |
| yIntron-5'S | Yeast intron | AGCTTACGTATGTTAATATGGACTAAAGGAGGCT TTTCTGGTACCTGAGCT |
| yIntron-5'AS | Yeast intron | CAGGTACCAGAAAAGCCTCCTTTAGTCCATATTA ACATACGTA |
| yIntron-3'S | Yeast intron | CGAATTTTTACTAACAAATGGTATTATTTATAAC AGCTG |
| yIntron-3'AS | Yeast intron | AATTCAGCTGTTATAAATAATACCATTTGTTAGT AAAAATTCGAGCT |
| Ef1B-Intron- 3'RIPvu | AtEFlbeta intron | ATCGAATTCAGCTGTAAACATATATACATAGAGA GACAGAAGA |
| Ef1B-Intron- 5'HIIISna | AtEF1beta intron | GATATCAAGCTTACGTAAGTTAGAATCTGTTTTC TAATAGCTGTCT |
| ADH-5'-2kb-TY- X-INIT | AtADH | AACCTAGAACCTCTTAATCCGACAAGAAGGAAG CACCAGCCATGAAAAGGAGCTCTGGCCATTAATT AA |
| ADH-3'-2kb-TY-X-TERM | AtADH | CCCAAAAGCAGAAATCTTCGAAACAAGTCTTAAG TCTCTTGTCTTTGATCTCGAGGCGCGCCGATAT |
| PI-f1-delta | φfd ori | GAAATACCGCACAGATGCGTAAGGAGAAAATACC GCATCAGGGTGTAGGCTGGAGCTGCTTC |
| P4-f1-delta | φfd ori | GCCCTTCCCAACAGTTGCGCAGCCTGAATGGCGA ATGGCGCGATTCCGGGGATCCGTCGAC |
| ADH-Test- AS(+400) | AtADH | TACGTATCTAGAAGCTTCATGGCCGAAGATAC |
| ADH-Test-S(- 400) | AtADH | ATCGGCGTGACCATCAAGACTA |
| Gal10-S | yGAL10 promoter | TATGGTGGTAATGCCATGTAAT |
| CycD3-Prom-5'X | AtCycD3 promoter | TCAGCGATTGCTCCTTGTAA |
| CycD3-Prom- 5'KpnSac | AtCycD3 promoter | ATCGGTACCGAGCTCTGTAGATTCGCTGGAGAAG TA |
| CycD3-Prom- 3'Xho | AtCycD3 promoter | ATCCTCGAGTGTGGGGGACTAAACTCAAG |
| CycD3-Prom-3'X | AtCycD3 promoter | GAGCGTTGACTCTCAGAATC |
| XpA-3'-Y303H- XbaSph | XpA | ATCTCTAGAGCATGCTGTGACCATAAGGCCACGT ATTTTG |
| XpA-5'-Y303H- XbaSph | XpA | ATCTCTAGACACAGCATGCCCATCGCAGTTCGCT A |
| KanMX-OUT-S | R Km^{R} | CCAGGATCTTGCCATCCTAT |
| KanMX-OUT-AS | Km^{R} | ATAGATTGTCGCACCTGATTG |
| HO-L-Test(- 2820) | yHO | TGTACTGTTGCAAGGCTAAT |
| HO-R- Test(+1870) | yHO | CGTATTTCTACTCCAGCATTCT |
| yR51-5'Bam | yRAD51 | GGGGGATCCAAAAAAATGTCTCAAGTTCAAGAAC AAC |
| VR51-3'Pst | yRAD51 | GGGGGATCCAAAAAAATGTCTCAAGTTCAAGAAC AAC AACTGCAGTTACTACTCGTCTTCTTCTCTGGGG AAAGAATTCGTTTAAACATGGCGTTTTTAAGCTA TTTTG |
| yR52-5'Pme | ScRAD52 | AAAGAATTCGTTTAAACATGGCGTTTTTAAGCTA TTTTG |
| yR52-3'Not | ScRAD52 | ATCGCGGCCGCTCATCAAGTAGGCTTGCGTGCA |
| | | |
| DMC-Prom- 5'Kpn-S1268 | AtDMC1 promoter | ATCGGTACCTGTACCGGTTGATTCATGTG |
| DMC-Prom- AS5408 | AtDMC1 promoter | TCATGAGACCATTGCAGGTAT |
| DMC-Prom-Int2- NcoRV | AtDMC1 promoter | GTACCATGGCGATATCACCTCCTTCTTCAGCTCT ATGAATCCGAAAC |
| ADM-Prom- 5'Kpn | AtDMC1 promoter | GGGGTACCTAATCGGTGATTGCCAAC |
| AtDMC-Pro-Nde- A1 | AtDMC1 promoter | TGCCTCTCACTTCACATATGC |
| AtMSH4-3'Bam | AtMSH4 promoter | CGGGATCCTTTCGCTCCACAGATCAG |
| AtMSH4-5'I | AtMSH4 promoter | GTGAGCTGTGTGACGTTA |
| AtMSH4-5'X | AtMSH4 promoter | CGCATCATGTTCTTGTTGAG |
| SPO-1-PROM-5'EX | AtSPO11 promoter | TCACCGTAGCTCTCGTCGCTTATT |
| SPO-1-PROM-3'EX | AtSPO11 promoter | AGCCAGCGAAGTCATCGACTAGAA |
| SPO-1-PROM-5'KpnSac | AtSPO11 promoter | ATCGGTACCGAGCTCTTCGCACGCACCTCCGATC T |
| SPO-1-PROM-3'Xho | AtSPO11 promoter | ATCCTCGAGCTCTTTCGAGTTTCAAAACTGAAAA ATG |
| C1 | Cm^{R} cassette | TTATACGCAAGGCGACAAGG |
| C2 | Cm^{R} cassette | GATCTTCCGTCACAGGTAGG |
| ADH-5'-2kb-TY-X-INIT | AtADH | AACCTAGAACCTCTTAATCCGACAAGAAGGGAAG CACCAGCCATGAAAAGGAGCTCTGGCCATTAATT AA |
| ADH-3'-2kb-TY-X-TERM | AtADH | CCCAAAAGCAGAAATCTTCGAAACAAGTCTTAAG TCTCTTGTCTTTGATCTCGAGGGCGCGCCGATAT |
| TEV-3'NcoSnaBam | TEV | ATCCCATGGTACGTAGGATCCCTATCGTTCGTAA ATGGTGAAAAT |

### A. Cloning of genetic elements from φfd and related bacteriophage

Samples of φfd and φM13 were obtained from the American Type Culture Collection (Item # 15669-B2 and 15669-B1, respectively). φfd was obtained as a freeze-dried sample in skim milk powder. The phage was resuspended in 0.5 ml of TYS broth (per litre distilled water: 10 g Tryptone (Difco); 5 g yeast extract (Difco); 5 g NaCl (Sigma)). To propagate the phage, an overnight culture of E. coli XL1-Blue (Stratagene) was first prepared in TYS containing tetracycline (12 µg/ml) and 200 µl of these cells were mixed with 2 or 20 µl of the φfd suspension. The cell-phage mixture was added to 3 ml TYS top agarose (i.e. TYS medium plus agarose (0.5% w/v); Sigma) and then poured onto TYS plates (i.e. TYS medium plus agar (1.5% (w/v); Sigma)) before incubating overnight at 37° C. The top agarose was scraped from these plates and placed in centrifuge tubes before centrifugation at 1-2000 RPM for 25 minutes. The resulting supernatant was collected and represented the phage stock which was stored at 4° C.

To prepare DNA samples of the phage to act as template for amplifying components by PCR, 6 ml of TYS with tetracycline (12 µg/ml) in 50 ml Falcon tubes was inoculated with 60 µl of an overnight culture of E. coli XL1-Blue and 60 µl the phage stock as prepared above. After incubating 8 h at 37° C with shaking at 200 RPM, 1.5 ml aliquots of the culture were distributed to microfuge tubes. The cells were pelleted by centrifugation at 12,000 RPM in a standard mcirocentrifuge (Brinkman) and 1.25 ml of the supernatant was transferred to a fresh microfuge tube. To this 250 µl of PEG solution (30% (w/v) polyethylene glycol (PEG) 8000 Sigma; 1.6 M NaCl) was mixed in and the mixture was incubated 15 min at room temperature. The phage was pelleted from this mixture by microcentrifugation (12, 000 RPM) for 10 min at room temperature. The supernatant was completely removed and discarded and the phage pellet was resuspended in 200 µl TE (10 mM Tris-HCl, 1 mM EDTA, pH 8.0) and then extracted with 100 µl phenol as per standard procedures [256]. From the supernatant, 175 µl was transferred to a fresh microfuge tube and 20 µl 3 M sodium-acetate plus 400 µl ethanol were added to precipitate the phage DNA as per standard procedures [256]. The DNA pellet was then resuspended in 25 µl LTE (1 mM Tris-HCl, 0.1 mM EDTA, pH 8.0) and stored at 4°C.

A1. Cloning of g2p and derivatives . Template for amplifying g2p was φfd genomic DNA isolated as described above. PCR reactions were performed with approximately 1 µg of genomic DNA as template, 1.0 pmol each of primers fdg2-5'RI and fdg2-3'Pst, 0.2mM dNTP's, 2.5 U Pfu (Stratagene) and Pfu buffer constituents provided by the manufacturer in a volume of 50 µl. The PCR conditions were 5 min @ 94 C, followed by 25 cycles of 30 s @ 94 C, 30 s @ 58 C and 2.5 min @ 72 C, followed by 10 min @ 72 C and storage at 4 C or -20 C. After completion of the cycling, two reactions were pooled and DNA fragments were resolved by agarose electrophoresis using a 1% gel and following standard procedures [256]. A DNA fragment of ∼1.2 kilobase pair (kb) expected to correspond to φfd g2p was excised and the DNA recovered from the agarose using the Qiaquick Gel Extraction Kit (Qiagen) following the protocol supplied by the manufacturer. DNA was digested with EcoRI and PstI following standard procedures [256]. The plasmid cloning vector pBluescript II SK-(Stratagene) was digested with EcoRI and PstI. The amplicon and vector DNA were purified by agarose electrophoresis and recovered as described above. Amplicon and vector DNA were then mixed in the presence of T4 DNA ligase (Gibco-BRL) to covalently link the two molecules following standard procedures [256] in a final volume of 25 µl. After incubating the ligation reaction as described [256], 1 µl of glycogen (20 mg/ml) was added to the ligation mixture made up to 100 µl with distilled water. After precipitation with ethanol [256], the DNA was resuspended in 4 µl of distilled water. An appropriate E. *coli* strain (e.g. DH5α (Gibco-BRL)) was transformed with 2.5 µl of the concentrated ligation following standard procedures [256] and plated on sterile TYS medium containing ampicillin (100 µg/ml). Putative clones were propagated in liquid TYS (i.e. without agar) and ampicillin (100 µg/ml). Plasmid DNA was isolated by standard alkaline-lysis "mini-prep" procedure [256]. The DNA sequence of the resultant clone, pRH12, was determined at a commercial sequencing facility (National Research Council-Plant Biotechnology Institute, Saskatoon, Canada) to confirm it encoded g2p. Cloning of all other genes and genetic elements described in this invention followed the same principles as for pRH12 with noted exceptions.

A second version of g2p was cloned wherein the ATG start codon was replaced with a SmaI site as one way of enabling translational fusion of g2p with other proteins or peptides. Template for amplifying g2pΔATG was φfd genomic DNA isolated as described above. PCR reactions were performed with approximately 1 µg of genomic DNA as template, 1.0 pmol each of primers fdg2-5'SmaI and fdg2-3'Pst, 0.2 mM dNTP's, 2.5 U Pfu (Stratagene) and Pfu buffer constituents recommended by the manufacturer in a volume of 50 µl. The PCR conditions were 5 min @ 94 C, followed by 25 cycles of 30 s @ 94 C, 30 s @ 58 C and 2.5 min @ 72 C, followed by 10 min @ 72 C and storage at 4 C or -20 C. After completion of the cycling, two reactions were pooled and DNA was digested with SmaI and Pst. The plasmid cloning vector pBluescript II KS- (Stratagene) was digested with SmaI and Pst. DNA fragments of interest corresponding to g2pΔATG (~1.2 kb) and the vector (~3 kb) were purified by agarose gel electrophoresis and recovered from the agarose as described above. The fragments were ligated together, transformed into *E. coli* and putative clones of the gene identified as described above. The DNA sequence of the resultant clone, pRH14, was determined to confirm it encoded g2pΔATG.

A third version of g2p was cloned so that the resultant protein would encode a nuclear localization sequence (NLS) at the N-terminus of the protein (i.e. NLS-g2p). A synthetic oligonucleotide was created which encoded the nuclear localization sequence corresponding to that found in simian virus 40 T-antigen [257]. The nucleotide sequence (GGATCCAAAAAAAATGGCTCAAGAAGAAG-AGAAAGGTTGGAGGAGGACCCGGG) encodes a BamHI site, in-frame start codon, and SmaI site (underlined). A plasmid containing this cloned NLS sequence and derived from pBluescript II KS- (Stratagene) was digested with SmaI and PstI and the DNA fragment corresponding to the vector (~3 kb) was gel purified. pH14 was also digested with SmaI and PstI and the DNA fragment corresponding to the g2p gene (~1.2 kb) was also gel purified. The DNA fragments were recovered from agarose, ligated together, transformed into *E. coli* and putative clones of the NLS-g2p gene identified as described above. The DNA sequence of the resultant clone, pRH36, was determined to confirm it encoded NLS-g2p.

A fourth version of g2p was cloned so that the resultant protein would encode a nuclear localization sequence (NLS) at the C-terminus of the protein (i.e. g2p-NLS). Synthetic oligonucleotides were created to attach to g2p the NLS that is found in the VirD2 protein of *Agrobacterium tumefaciens* which has been shown to function in plants and other eukaryotes [258;259]. The NLS was attached to the g2p gene in a multi-step process using PCR to attach sequences to g2p including the NLS, a series of glycine residues between g2p and the NLS to promote flexibility between g2p and the C-terminal additions, and the FLAG peptide [260] which enables detection of the fusion protein using commercially available antibodies (Sigma). A primary PCR reaction was performed with ~500 ng of pRH12 as template, 1.0 pmol each of primers fdg2-5'RI and g2p-3'Gly-SmaPst, 0.2 mM dNTP's, 2.5 U Pfu (Stratagene) and Pfu buffer constituents recommended by the manufacturer in a volume of 50 µl. The PCR conditions were 5 min @ 94 C, followed by 25 cycles of 30 s @ 94 C, 30 s @ 58°C and 2.5 min @ 72 C, followed by 10 min @ 72 C and storage at 4 C or -20 C. The PCR products were resolved by agarose gel electrophoresis and the ~1.2 kb fragment corresponding to g2p plus the poly-glycine encoding sequence was excised from the gel and purified from the agarose as outlined above. A secondary PCR reaction was then performed using 10 µl of this DNA fragment as template 1.0 pmol each of primers fdg2-5'RI and g2p-3'NLS-HpaPst, 0.2 mM dNTP's, 2.5 U Pfu (Stratagene) and Pfu buffer constituents recommended by the manufacturer in a volume of 50 µL. The PCR conditions were 5 min @ 94 C, followed by 35 cycles of 30 s @ 94 C, 30 s @ 64°C and 2.5 min @ 72 C, followed by 10 min @ 72 C and storage at 4 C or -20 C. The PCR products were resolved by agarose gel electrophoresis and the ~1.2 kb fragment corresponding to g2p plus the poly-glycine and NLS encoding sequences was excised from the gel and purified from the agarose as outlined above. A fraction of this PCR product was digested with EcoRI and PstI and the plasmid cloning vector pBluescript II SK- (Stratagene) was also digested with EcoRI and Pst. DNA fragments of interest corresponding to g2p+Gly+NLS (~1.2 kb) and the vector (~3 kb) were purified by agarose gel electrophoresis and recovered from the agarose as described above. The fragments were ligated together, transformed into *E. coli* and putative clones of the gene identified as described above. The DNA sequence of the resultant clone, pAS3, was determined to confirm it encoded g2p fused at the C-terminus to a glycine tract followed by the NLS from VirD2. A tertiary PCR reaction was then performed using 10 µl of the DNA fragment purified from the secondary PCR as template, 1.0 pmol each of primers fdg2-5'RI and g2p-3'FLAG-Pst, 0.2 mM dNTP's, 2.5 U Pfu (Stratagene) and Pfu buffer constituents recommended by the manufacturer in a volume of 50 µl. The PCR conditions were 5 min @ 94 C, followed by 25 cycles of 30 s @ 94 C, 30 s @ 64°C and 2.5 min @ 72 C, followed by 10 min @ 72 C and storage at 4 C or -20 C. The PCR products were resolved by agarose gel electrophoresis and the ~1.3 kb fragment corresponding to g2p plus the poly-glycine and NLS and FLAG encoding sequences was excised from the gel and purified from the agarose as outlined above. The DNA was digested with EcoRI and Pst. The plasmid cloning vector pBluescript II SK- (Stratagene) was digested with EcoRI and Pst. DNA fragments of interest corresponding to g2p+Gly+NLS+FLAG (~1.3 kb) and the vector (~3 kb) were purified by agarose gel electrophoresis and recovered from the agarose as described above. The fragments were ligated together, transformed into *E. coli* and putative clones of the gene identified as described above. The DNA sequence of the resultant clone, pAS4, was determined to confirm it encoded g2p fused at the C-terminus to a glycine tract followed by the NLS from VirD2 followed by the FLAG peptide. This gene assembly encoded by pAS4 will henceforth be referred to as g2p-NLS.

### A2. g2p expression constructs

Plasmid constructs were assembled to facilitate expression of g2p and its variants in E. coli by the tac promoter [261] which is regulatable by the gratuitous inducer IPTG. g2p was cloned into the expression vector pDK5 [262] by first digesting the vector with EcoRI and PstI. pRH12 was also digested with EcoRI and PstI. DNA fragments of interest corresponding to g2p (~1.2 kb) and pDK5 (~4.3 kb) were purified by agarose gel electrophoresis and recovered from the agarose as described above. The fragments were ligated together, transformed into *E. coli* and putative clones of the gene in the expression vector were identified. The resultant clone of g2p in pDK5 was denoted pRH27.

NLS-g2p was assembled in a derivative of the expression vector pDK5 [262]which encodes the NLS described for pRH36 fused to the EcoRI site of pDK5 and having a SmaI site at the 3' end of the sequence encoding the NLS (i.e. pDK5+NLS). This pDK5+NLS was digested with SmaI and PstI. pH14 was also digested with SmaI and PstI. DNA fragments of interest corresponding to g2pΔATG (~1.2 kb) and pDK5+NLS (~4.3 kb) were purified by agarose gel electrophoresis and recovered from the agarose as described above. The fragments were ligated together, transformed into *E. coli* and putative clones of the gene in the expression vector were identified. The resultant clone of NLS-g2p in pDK5 was denoted pRH28.

For expression of g2p-NLS, the gene was first cloned into pENTR11 (Gibco BRL). pAS4 encoding g2p-NLS was first cut with EcoRI and treated with Klenow polymerase (Gibco BRL) following standard procedures [256] to make the end of the DNA fragment blunt before a subsequent digestion with NotI. pENTR11 was digested with XmnI and NotI. DNA fragments of interest corresponding to g2p-NLS (~1.3 kb) and pENTR11 (~2.3 kb) were purified by agarose gel electrophoresis and recovered from the agarose as described above. The fragments were ligated together, transformed into *E. coli,* selected in the presence of kanamycin (50 µg/ml), and putative clones of the gene in the vector were identified. The resultant clone of g2p-NLS in pENTR11 was denoted pAS12. The g2p-NLS gene was then transferred into an E. coli expression vector, pMW137, using the Clonase (Gibco BRL) reaction following the directions supplied by the manufacturer, resulting in pAS 17 which is selectable with chloramphenicol (20 µg/ml). pMW137 is a derivative of pACYC184 [263] encoding the *tac* promoter and rrnB terminator from pKK223-3 [264]. pMW137 was constructed by first ligating the ~1.2 kb BamHI-PvuI fragment encoding the *tac* promoter and rrnB terminator from pKK223-3 to the ~3.6 kb HindIII-SalI fragment of pACYC184 using a combination of blunting ends with T4 polymerase (New England BioLabs) and restriction site linkers, as per standard procedures [256]. This assembly was then digested with SmaI and HindIII followed by treatment with T4 polymerase and ligation to the Destination-A cassette (Gibco BRL) resulting in pMW137.

Plasmid constructs were assembled to facilitate expression of g2p and its variants in eukaryotic yeast using an expression system developed by Gari et al., (1997) [265]. Briefly, the transcription promoters on these plasmids are a hybrid system developed by Gari et al. (1997) which permits suppression or induction of gene expression by varying growth medium constituents. This transcription control system employs components of the regulatory system controlling expression of tetracycline resistance in prokaryotes [265]. As a result, in the presence of tetracycline or doxycycline, an analogue of tetracycline, transcription of the target gene is suppressed. Conversely, when tetracycline or doxycycline is absent efficient transcription of the target gene can occur. By varying the number of *tetO* sites in the promoter from two (i.e. Tet2x promoter) to seven (i.e. Tet7x promoter), the promoter strength can be increased ~2-fold [265]. The combination of vector copy number (i.e. CEN-type vs. 2u-type with copy numbers of 1-2 plasmids per cell or up to 40 plasmids per cell, respectively [266]) and promoter strength allows gene expression to be varied ~5-fold [265]. Yeast expression plasmids using this system of gene regulation include pCM188, pCM189 and pCM190 as described by Gari et al., (1997) as well as derivatives thereof. These derivatives were based on the plasmids described by Geitz et al., (1997) and were created by subcloning an EcoRI-HindIII fragment encoding either the Tet2x (~2.6 kb) or Tet7x (~2.8 kb) promoter elements from pCM188 or pCM190, respectively, into the EcoRI-HindIII site of YEplac112 (i.e. creating YEplac112-Tet7x), or YCplac22 (i.e. creating YCplac22-Tet2x), or YEplac181 (i.e. creating YEplac181-Tet2x). In addition, derivatives of these plasmids were created which contained the Destination cassette (Gibco BRL). pCM188 and pCM190 were each digested with BamHI and PstI and then treated with T4 polymerase to make the DNA ends blunt before ligation to the Destination-C cassette (Gibco BRL) to create pAS13 (i.e. pCM188-DEST) and pAS14 (i.e. pCM190-DEST). Restriction enzyme analysis demonstrated that the Destination-C cassette in these vectors was in a sense orientation with regard to the promoter so that genes transferred into the Destination cassette would be functionally expressed. pAS 13 and pAS 14 were then each digested with XhoI and HindIII to release fragments encoding the Tet2x and Tet7x promoters, respectively, plus the attached Destination-C cassette. These fragments were then ligated to either YCplac22-Tet2x to create pAS22 (i.e. YCplac22-Tet2x-DEST) or YEplac1 12-Tet7x to create pAS23 (i.e. YEplac112-Tet7x-DEST).

g2p was cloned into the expression vector YEplac 112-Tet7x by first digesting the vector with PmeI and PstI. pRH12 was digested with EcoRV and PstI. DNA fragments of interest corresponding to g2p (~1.2 kb) and YEplac1 12-Tet7x (~7.8 kb) were purified by agarose gel electrophoresis and recovered from the agarose as described above. The fragments were ligated together, transformed into *E. coli* and putative clones of the gene in the expression vector were identified. The resultant clone of g2p in YEplac112-Tet7x was denoted pRH35.

g2p was cloned into the expression vector YCplac22-Tet2x by first digesting the vector with PmeI and PstI. pH12 was digested with EcoRV and PstI. DNA fragments of interest corresponding to g2p (~1.2 kb) and YCplac22-Tet2x (~7.4 kb) were purified by agarose gel electrophoresis and recovered from the agarose as described above. The fragments were ligated together, transformed into *E. coli* and putative clones of the gene in the expression vector were identified. The resultant clone of g2p in YCplac22-Tet2x was denoted pRH38.

NLS-g2p was cloned into the expression vector YEplac112-Tet7x by first digesting the vector with BamHI and PstI. pRH12 was also digested with BamHI and PstI. DNA fragments of interest corresponding to g2p (~1.2 kb) and YEplac1 12-Tet7x (~7.8 kb) were purified by agarose gel electrophoresis and recovered from the agarose as described above. The fragments were ligated together, transformed into *E. coli* and putative clones of the gene in the expression vector were identified. The resultant clone of NLS-g2p in YEplac112-Tet7x was denoted pRH37.

g2p-NLS was cloned into the expression vector YCplac22-Tet2x-DEST by using the Clonase (Gibco BRL) reaction, following the directions supplied by the manufacturer, to transfer the gene from pAS12. The resultant clone of g2p-NLS in YCplac22-Tet2x-DESTwas denoted pAS26.

g2p-NLS was cloned into the expression vector YEplac 112-Tet7x-DEST by using the Clonase (Gibco BRL) reaction, following the directions supplied by the manufacturer, to transfer the gene from pAS12. The resultant clone of g2p-NLS in YEplac112-Tet7x-DESTwas denoted pAS27.

g2p-NLS can also be cloned into vectors to enable integration into the chromosome of eukaryotic yeast cells. To enable integration of and expression of g2p-NLS from the yeast chromosome, pAS26 or pAS27 can be digested with EcoRI and HindIII and the resulting fragments encoding the Tet2x or Tet7x promoters linked to g2p-NLS, respectively, (i.e. ~3.8 kb and ~4 kb, respectively) purified. These fragments may then be treated with T4 polymerase to make the DNA ends blunt Alternatively, the promoter plus g2p-NLS fragments may be isolated by digestion of pAS26 or pAS27 with PvuII. pHO-poly-KanMX4-HO [267] may then be digested with SmaI and treated with calf intestinal phosphatase following standard procedures [256]. The resulting DNA fragments encoding g2p-NLS plus associated promoter from pAS26 or pAS27 and the ~6.1 kb fragment from pHO-poly-KanMX4-HO can then be purified by agarose gel electrophoresis and recovered from the agarose as described above. The fragments may be ligated together, transformed into *E. coli* and putative clones of the assembly identified as described above. The resultant clone of g2p-NLS plus either the Tet2x or Tet7x promoter cloned into the chromosomal integrating vector pHO-poly-KanMX4-HO may then be transferred into the yeast chromosome following established procedures [267]. Using appropriate restriction enzyme combinations, g2p plus Tet2x or Tet7x promoter assemblies can also be placed into an integrating vector like YIplac128 [268].

Using the Gateway (Gibco BRL) cloning system genes encoding g2p, and variants thereof, may be transferred to vectors for expression in eukaryotic yeast, plant or animal cells or prokaryotic cells like E. coli. For example, g2p, NLS-g2p or g2p-NLS may be transferred to YCplac22-Tet2X::DEST or YEplac112-Tet7x::DEST for expression in eukaryotic yeast cells or to vectors possessing a Destination cassette (Gibco BRL) appropriately arranged with an appropriate promoter to facilitate expression of the gene in plant or animal cells. Versions of g2p with or without NLS sequences or intervening introns or altered sequences described here may also be transferred to vectors for expression in eukaryotic yeast, plant or animal cells in a similar fashion as used for the variants described here employing either restriction enzymes alone or restriction enzymes in concert with the Gateway (Gibco BRL) or other cloning approach.

### A3. Cloning of φfd origin elements and derivatives

A sequence corresponding to the φfd origin of replication which may be used to initiate DNA replication as part of a gene targeting system was cloned after amplification by PCR. Template for amplifying φfd-initiator was φfd genomic DNA isolated as described above. PCR reactions were performed with approximately 0.5 µg of genomic DNA as template, 1.0 pmol each of primers Init-5'BamPme and Init-3'SacPac, 0.2 mM dNTP's, 2.5 U Pfu (Stratagene) and Pfu buffer constituents recommended by the manufacturer in a volume of 50 µl. The PCR conditions were 5 min @ 94 C, followed by 35 cycles of 30 s @ 94 C, 30 s @ 58 C and 1 min @ 72 C, followed by 10 min @ 72 C and storage at 4 C or -20 C. After completion of the cycling, the DNA was digested with SacII. The plasmid cloning vector pBluescript II SK- (Stratagene) was digested with SmaI and SacII. DNA fragments of interest corresponding to φfd-initiator (~460 bp) and the vector (~3 kb) were purified by agarose gel electrophoresis and recovered from the agarose as described above. The fragments were ligated together, transformed into *E. coli* and putative clones of the gene identified as described above. The DNA sequence of the resultant clone, pH5, was determined to confirm it encoded φfd-initiator.

A sequence corresponding to the φfd origin of replication which may act to terminate DNA replication as part of a gene targeting system was cloned after amplification by PCR. Template for amplifying φfd-terminator was φfd genomic DNA isolated as described above. PCR reactions were performed with approximately 0.5 µg of genomic DNA as template, 1.0 pmol each of primers Term-5'AscRV and Term-3'SalNot, 0.2 mM dNTP's, 2.5 U Pfu (Stratagene) and Pfu buffer constituents recommended by the manufacturer in a volume of 50 µl. The PCR conditions were 5 min @ 94 C, followed by 35 cycles of 30 s @ 94 C, 30 s @ 58 C and 1 min @ 72 C, followed by 10 min @ 72 C and storage at 4 C or -20 C. After completion of the cycling, the DNA was digested with SalI. The plasmid cloning vector pBluescript II SK- (Stratagene) was digested with SmaI and SalI. DNA fragments of interest corresponding to φfd-terminator (~330 bp) and the vector (~3 kb) were purified by agarose gel electrophoresis and recovered from the agarose as described above. The fragments were ligated together, transformed into *E. coli* and putative clones of the gene identified as described above. The DNA sequence of the resultant clone, pRH9, was determined to confirm it encoded φfd-terminator.

The φfl origin (Genbank Accession # V00606) and φfd origin (Genbank Accession # V00602) regions share 98% identity within the 457 bp sequence bound by conserved RsaI and DraI sites. One of the diverted nucleotides results in the absence of a BamHI site within the φfl origin region vs. the φfd origin region. The φfl origin is encoded by pTZ19 [269], pEMBL8 [270], and many other cloning vectors. To clone sequences corresponding to the φfl origin of replication which may be used to initiate or terminate DNA replication the same PCR conditions, primers and cloning procedures as indicated for cloning the φfd origin regions were used except that pTZ19 was used as template for the PCR reaction. The DNA sequence of the resultant clones, pRH10 and pRH11, was determined to confirm they encoded the φfl-initiator and φfl-terminator, respectively.

The φfd-initiator and φfd-terminator sequences were linked together by first preparing the cloned DNA fragment encoding the φfd-initiator such that one end cleaved with SacI was made blunt with T4 polymerase and the other end was cleaved with HindIII. The cloned DNA fragment encoding the φfd-terminator was prepared so that one end was cleaved with EcoRI and made blunt with Klenow polymerase and the other end was cleaved with SalI. The ~460 bp and ~330 bp fragments encoding the φfd-initiator and φfd-terminator sequences, respectively, were then ligated to pSPORT2 (Gibco BRL) digested with HindIII and SalI. The resultant clone of the linked φfd-initiator and φfd-terminator sequences in pSPORT2 was denoted pRH20. The φfd-initiator and φfd-terminator can be linked with an adjoining or intervening sequence to facilitate replication and amplification of this sequence in conjunction with the action of the g2p protein or derivatives thereof.

The φfl-initiator and φfl-terminator sequences were linked together by first preparing the cloned DNA fragment encoding the φfl-initiator such that one end cleaved with SacI was made blunt with T4 polymerase and the other end was cleaved with HindIII. The cloned DNA fragment encoding the φfl-terminator was prepared so that one end was cleaved with EcoRI and made blunt with Klenow polymerase and the other end was cleaved with SalI. The ~460 bp and ~330 bp fragments encoding the φfl-initiator and φfl-terminator sequences, respectively, were then ligated to pSPORT2 (Gibco BRL) cleaved with HindIII and SalI. The resultant clone of the linked φfl-initiator and φfl-terminator sequences was denoted pH21. The φfl-initiator and φfl-terminator can be linked with an adjoining or intervening sequence to facilitate replication and amplification of this sequence in conjunction with the action of the g2p protein or derivatives thereof.

### A4. Constructs for assaying g2p and its variants

To assay g2p and its variants in E. coli, the φfd-initiator and φfd-terminator sequences, with and without an intervening sequence to be replicated, and the various forms of g2p were cloned on separate plasmids which could be cotransformed into E. coli. The linked φfd-initiator and φfd-terminator sequences were cloned into pACYC184 by digesting both this vector and pRH20 with HindIII and SalI. The resulting ~3.6 kb DNA fragment from pACYC184 and the ~800 bp fragment from pRH20 encoding the φfd-initiator and φfd-terminator sequences were purified by agarose gel electrophoresis and recovered from the agarose as described above. The fragments were ligated together, transformed into *E. coli* and putative clones of the assembly identified as described above. The resultant clone of the linked φfd-initiator and φfd-terminator sequences in pACYC184 was denoted pRH26.

A version of the linked φfd-initiator and φfd-terminator sequences containing an intervening sequence to be replicated was also cloned into pACYC184. pZeoSVLacZ (InVitrogen) was digested with ScaI and SacII to release a ~3.3 kb fragment encoding the E. coli LacZ gene. pRH20 was digested with PacI and treated with T4 polymerase to make this end blunt, and then digested with SacII. The resulting ~3.3 kb DNA fragment from pZeoSVLacZ and the ~5.1 kb fragment from pRH20 encoding the ~fd-initiator and ~fd-terminator sequences in pSPORT2 (Gibco BRL) were purified by agarose gel electrophoresis and recovered from the agarose as described above. The fragments were ligated together, transformed into *E. coli* and putative clones of the assembly identified as described above. The resultant clone of the φfd-initiator and φfd-terminator sequences linked with the ~3.3 kb intervening sequence in pSPORT2 (Gibco BRL) was denoted pRH22. pRH22 and pACYC184 were then digested with SalI and HindIII. The resulting ~3.6 kb DNA fragment from pACYC184 and the ~4.1 kb fragment from pRH22 encoding the φfd-initiator and φfd-terminator sequences with the ~3.3 kb intervening sequence were purified by agarose gel electrophoresis and recovered from the agarose as described above. The fragments were ligated together, transformed into *E. coli* and putative clones of the assembly identified as described above. The resultant clone of the linked φfd-initiator and φfd-terminator sequences with a ~3.3 kb intervening sequence in pACYC184 was denoted pRH24.

To assay g2p and its variants in eukaryotes, the φfd-initiator and φfd-terminator sequences, with and without an intervening sequence to be replicated, and the various forms of g2p were cloned to enable their cotransformation into yeast. As an example of sequences to be replicated using the invention, the URA3 gene from *Saccharomyces cerevisiae* was used. Lambda clone PM-6150 encoding this gene and flanking genomic regions was obtained from the American Type Culture Collection (Item #70772). The lambda clone was propagated and DNA isolated following standard procedures [256]. The lambda clone DNA was digested with ClaI and SmaI and a ~1.85 kb fragment was purified by agarose gel electrophoresis and recovered from the agarose as described above. Based on the published genomic sequence of S. cerevisiae this fragment will encode the URA3 gene. The cloning vector pQuantox (Quantum Biotechnologies) was also digested with ClaI and SmaI and the DNA fragment corresponding to this vector (~5.3 kb) was purified. The two fragments were ligated together, transformed into *E. coli* and putative clones of the assembly identified as described above. The resultant clone of the ~1.85 kb fragment encoding URA3 was denoted pMW41. Variants of the URA3 gene were also created after first subcloning this ~1.85 kb fragment into pBluescript II KS- by digesting both pMW41 and the recipient vector with NotI and XhoI, purifying the respective fragments and ligating them together. The resultant clone of the ~1.85 kb fragment encoding URA3 in pBluescript II KS- was denoted pMW107. pMW107 was digested with EcoRV and NcoI to delete ~16 bp within the open reading frame of URA3 and the resulting DNA ends were made blunt by treatment with T4 DNA polymerase before the ~4.8 kb fragment was purified by agarose gel electrophoresis. This fragment was self-ligated, transformed into *E. coli* and putative clones of the assembly identified as described above. The resultant clone of the ura3ΔEcoRV-NcoI allele in pBluescript II KS- was denoted pMW105. Another URA3 allele was created by digesting pMW107 with PstI and EcoRV to delete ~205 bp encompassing the start codon of the URA3 gene. The DNA ends resulting after this digestion were made blunt by treatment with T4 DNA polymerase before the ~4.6 kb fragment was purified by agarose gel electrophoresis. This fragment was self-ligated, transformed into *E. coli* and putative clones of the assembly identified as described above. The resultant clone of the ura3ΔPstI-EcoRV allele in pBluescript II KS- was denoted pMW180. Another URA3 allele was created by digesting pMW41 with SmaI and StuI to delete ~450 bp encompassing approximately the 3' half of the URA3 gene. The ~6.7 kb fragment was purified by agarose gel electrophoresis, self-ligated, transformed into *E. coli* and putative clones of the assembly identified as described above. The resultant clone of the ura3ΔStuI-SmaI allele in pQuantox was denoted pRH29.

The URA3 alleles described above were linked to φfd-initiator and φfd-terminator sequences and cloning into shuttle vectors for introduction into eukaryotic yeast cells. To transfer the ura3ΔStuI-SmaI into a yeast shuttle vector, pRH29 was first digested with SalI, and the DNA ends made blunt by treatment with Klenow polymerase, and then digested with SacII to release a ~1.4 kb fragment. pRH20 was digested with PacI, the DNA ends made blunt by treatment with T4 polymerase, and then digested with SacII. The resulting ~5.1 kb DNA fragment from pRH20 and the ~1.4 kb fragment from pRH29 were purified by agarose gel electrophoresis and recovered from the agarose as described above. The fragments were ligated together, transformed into *E. coli* and putative clones of the assembly identified as described above. The resultant clone of the φfd-initiator and φfd-terminator sequences with a ~1.4 kb ura3ΔStuI-SmaI intervening sequence in pSPORT2 was denoted pRH30. In a similar fashion the ~1.4 kb ura3ΔStuI-SmaI fragment was cloned to intervene the φfd-initiator and φfd-terminator sequences in the opposite orientation as in pRH30. To achieve this, pRH20 was digested with AscI, the DNA ends made blunt by treatment with Klenow polymerase, and then digested with SacII. The resulting ~5.1 kb DNA fragment from pRH20 and the ~1.4 kb fragment from pRH29 were purified by agarose gel electrophoresis and recovered from the agarose as described above. The fragments were ligated together, transformed into *E. coli* and putative clones of the assembly identified as described above. The resultant clone of the φfd-initiator and φfd-terminator sequences with a ~1.4 kb ura3ΔStuI-SmaI intervening sequence in pSPORT2 was denoted pH31. To transfer these two φfd-initiator and φfd-terminator::ura3ΔStuI-SmaI assemblies as well as the φfd-initiator and φfd-terminator sequences without an intervening sequence to yeast vectors, pRH30, pRH31, pRH20 and YCplac111[268] were first digested with SalI and SphI. The resulting ~2.2 kb fragments from pRH30 and pRH31, the ~0.8 kb fragment from pRH20 and ~6.1 kb fragment from YCplac1 11 were purified by agarose gel electrophoresis and recovered from the agarose as described above. The insert and vector fragments were ligated pairwise together, transformed into *E. coli* and putative clones of the assemblies identified as described above. The resultant clone of φfd-initiator and φfd-terminator::ura3ΔStuI-SmaI assembly from pRH30 in YCplac1 11 was denoted pRH32. The resultant clone of φfd-initiator and φfd-terminator::ura3ΔStuI-SmaI assembly from pRH31 in YCplac111 was denoted pRH33. The resultant clone of φfd-initiator and φfd-terminator assembly from pRH20 in YCplac1 11 was denoted pRH34.

The URA3 alleles described and linked to φfd-initiator and φfd-terminator sequences were also cloned into vectors for integration into the chromosome of eukaryotic yeast cells. To enable integration of the φfd-initiator and φfd-terminator::ura3ΔStuI-SmaI and φfd-initiator and φfd-terminator (i.e. without an intervening sequence) assemblies into a chromosome, pRH20, pRH30 and YIplac128 [268] were first digested with SalI and SphI. The resulting ~2.2 kb fragments from pRH30, the ~0.8 kb fragment from pRH20 and ~4.3 kb fragment from YIplac128 were purified by agarose gel electrophoresis and recovered from the agarose as described above. The insert and vector fragments were ligated pairwise together, transformed into *E. coli* and putative clones of the assemblies identified as described above. The resultant clone of φfd-initiator and φfd-terminator::ura3ΔStuI-SmaI assembly from pRH30 in YIplac128 was denoted pRH40. The resultant clone of φfd-initiator and φfd-terminator assembly (i.e. without an intervening sequence) from pRH20 in YIplac128 [268] was denoted pRH39.

To transfer the ura3ΔNcoI-EcoRV linked to φfd-initiator and φfd-terminator sequences into a yeast shuttle vector, pMW105 was first digested with XhoI, and the DNA ends made blunt by treatment with T4 polymerase, and then digested with SacII to release ~1.8 kb fragment. pRH34 was digested with PacI, the DNA ends made blunt by treatment with T4 polymerase, and then digested with SacII. The resulting ~6.9 kb DNA fragment from pRH34 and the ~1.8 kb fragment from pMW105 were purified by agarose gel electrophoresis and recovered from the agarose as described above. The fragments were ligated together, transformed into *E. coli* and putative clones of the assembly identified as described above. The resultant clone of the φfd-initiator and φfd-terminator sequences with a ~1.8 kb ura3ΔNcoI-EcoRV intervening sequence in YCplac111 [268] was denoted pMW113. In a similar fashion the ~1.8 kb ura3ΔNcoI-EcoRV fragment was cloned to intervene the φfd-initiator and φfd-terminator sequences in the opposite orientation as in pMW113. To achieve this, pRH34 was digested with AscI, the DNA ends made blunt by treatment with T4 polymerase, and then digested with SacII. A DNA fragment from pRH34 and the ~1.8 kb fragment as described above from pMW105 were purified by agarose gel electrophoresis and recovered from the agarose as described above. The fragments were ligated together, transformed into *E. coli* and putative clones of the assembly identified as described above. The resultant clone, denoted pMW1 14, in YCplac 111 [268] encoded the ura3ΔNcoI-EcoRV fragment, however, the φfd-initiator and φfd-terminator sequences were made defective by an undefined cause during the cloning procedure.

The ura3ΔNcoI-EcoRV allele linked to φfd-initiator and φfd-terminator sequences was also cloned into vectors to enable integration into the chromosome of eukaryotic yeast cells. To enable integration of the φfd-initiator and φfd-terminator.:ura30NcoI-EcoRVassembly into a chromosome, pMW105 was first digested with XhoI, and the DNA ends made blunt by treatment with T4 polymerase, and then digested with SacII to release ~1.8 kb fragment. pRH39 was digested with AscI, the DNA ends made blunt by treatment with T4 polymerase, and then digested with SacII. The resulting ~5.1 kb DNA fragment from pRH39 and the ~1.8 kb fragment from pMW105 were purified by agarose gel electrophoresis and recovered from the agarose as described above. The fragments were ligated together, transformed into *E. coli* and putative clones of the assembly identified as described above. The resultant clone of the φfd-initiator and φfd-terminator sequences with a ~1.8 kb ura3ΔNcoI-EcoRV intervening sequence in YIplac128 [268] was denoted pMW108.

To transfer the ura3ΔPstI-EcoRV linked to φfd-initiator and φfd-terminator sequences into yeast shuttle vectors, pMW180 was first digested with KpnI, and the DNA ends made blunt by treatment with T4 polymerase, and then digested with SacII to release ~1.6 kb fragment. pRH34 was digested with AscI, the DNA ends made blunt by treatment with T4 polymerase, and then digested with SacII. The resulting ~6.9 kb DNA fragment from pRH34 and the ~1.6 kb fragment from pMW180 were purified by agarose gel electrophoresis and recovered from the agarose as described above. The fragments were ligated together, transformed into *E. coli* and putative clones of the assembly identified as described above. The resultant clone of the φfd-initiator and φfd-terminator sequences with a ~1.6 kb ura3ΔPstI-EcoRV intervening sequence in YEplac181 [268] was denoted pMW183. pMW183 was then digested with PmeI and EcoRI to release a ~2.4 kb fragment encoding φfd-initiator and φfd-terminator::ura3ΔPstI-EcoRV which was treated with T4 polymerase to make the DNA ends blunt and purified by agarose gel electrophoresis and recovered from the agarose as described above. YEplac181-Tet2x was digested with PmeI and treated with calf-intestinal phosphatase. These two fragments were ligated together, transformed into *E. coli* and putative clones of the assembly identified as described above. The resultant clone of the φfd-initiator and φfd-terminator sequences with a ~1.6 kb ura3ΔPstI-EcoRV intervening sequence in YEplac181-Tet2x was denoted pNML18.

The ura3ΔPstI-EcoRV allele linked to φfd-initiator and φfd-terminator sequences was also cloned for integration into the chromosome of eukaryotic yeast cells. To enable integration of the φfd-initiator and φfd-terminator::ura3ΔPstI-EcoRV into a chromosome, pMW180 was first digested with NdeI and SmaI, to release ~0.9 kb fragment. pRH32 was digested with SacI, the DNA ends made blunt by treatment with T4 polymerase, and then digested with NdeI. The resulting ~6 kb DNA fragment from pRH32 and the ~0.9 kb fragment from pMW180 were purified by agarose gel electrophoresis and recovered from the agarose as described above. The fragments were ligated together, transformed into *E. coli* and putative clones of the assembly identified as described above. The resultant clone of the φfd-initiator and φfd-terminator sequences with a ~1.6 kb ura3ΔPstI-EcoRV intervening sequence in YCplac1 11 [268] was denoted pMW241. pMW241 was then digested with PmeI and NotI as was YEplac181-Tet2x. The resulting ~2.6 kb DNA fragment from pMW241 and the ~8.3 kb fragment from YEplac1 81-Tet2x were purified by agarose gel electrophoresis and recovered from the agarose as described above. The fragments were ligated together, transformed into *E. coli* and putative clones of the assembly identified as described above. The resultant clone of the φfd-initiator and φfd-terminator sequences with a ~1.6 kb ura3ΔPstI-EcoRV intervening sequence in YEplac181-Tet2x was denoted pMW242. pMW242 was then digested with EcoRI and NotI and the DNA ends made blunt by treatment with T4 polymerase. Alternatively, PvuII digestion of pMW242 enables purification of a ~5.1 kb DNA fragment with blunt ends. pHO-poly-KanMX4-HO [267] was digested with SmaI and treated with calf intestinal phosphatase following standard procedures [256]. The resulting ~5.5 kb DNA fragment from pMW242 and the ~6.1 kb fragment from pHO-poly-KanMX4-HO were purified by agarose gel electrophoresis and recovered from the agarose as described above. The fragments were ligated together, transformed into *E. coli* and putative clones of the assembly identified as described above. The resultant clone of the φfd-initiator and φfd-terminator sequences with a ~1.6 kb ura3ΔPstI-EcoRV intervening sequence in the chromosomal integrating vector pHO-poly-KanMX4-HO was denoted pMW245. Using appropriate restriction enzyme combinations, the φfd-initiator and φfd-terminator sequences with a ~1.6 kb ura3ΔPstI-EcoRV allele intervening sequence from pMW241 can also be placed in YIplac128 [268].

### B. Cloning of φX174 components

### B1. cloning ofXpA and derivatives

Template for amplifying φX174 components was φX174 viral RF I DNA (New England BioLabs). To clone the XpA* gene PCR reactions were performed with approximately 1 µg of viral DNA as template, 1.0 pmol each of primers XpA*-5'SmaSfo and XpA*-3'HIIINotI, 0.2 mM dNTP's, 2.5 U Pfu (Stratagene) and Pfu buffer constituents recommended by the manufacturer in a volume of 50 µl. The PCR conditions were 5 min @ 94 C, followed by 25 cycles of 30 s @ 94 C, 30 s @ 58 C and 2.5 min @ 72 C, followed by 10 min @ 72 C and storage at 4 C or -20 C. After completion of the cycling the DNA was digested with HindIII. The plasmid cloning vector pBluescript II KS- (Stratagene) was digested with SmaI and HindIII. DNA fragments of interest corresponding to XpA* (~1 kb) and the vector (~3 kb) were purified by agarose gel electrophoresis and recovered from the agarose as described above. The fragments were ligated together, transformed into *E. coli* and putative clones of the gene identified as described above. The DNA sequence of the resultant clone, pAS5, was determined to confirm it encoded XpA*.

The gene encoding XpA was cloned using approximately 1 µg of viral DNA as template in a PCR reaction containing 1.0 pmol each of primers XpA-5'Sal-RBS-BamSma and XpA-3'HIIINotISacSfo, 0.2 mM dNTP's, 2.5 U Pfx (Gibco BRL) and Pfx buffer constituents recommended by the manufacturer in a volume of 50 µl. The PCR conditions were 5 min @ 94 C, followed by 25 cycles of 30 s @ 94 C, 30 s @ 60 C and 2 min @ 68 C, followed by 10 min @ 72 C and storage at 4 C or -20 C. After completion of the cycling the DNA was digested with NotI. The plasmid cloning vector pBluescript II SK+ (Stratagene) was digested with EcoRV and NotI. DNA fragments of interest corresponding to XpA (~1.5 kb) and the vector (~3 kb) were purified by agarose gel electrophoresis and recovered from the agarose as described above. The fragments were ligated together, transformed into *E. coli* and putative clones of the gene identified as described above. The DNA sequence of the resultant clone, pNML7-8, was determined to confirm it encoded XpA.

A second version of XpA* was cloned so that the resultant protein would encode a nuclear localization sequence (NLS) at the N-terminus of the protein (i.e. NLS-XpA*). The NLS is followed by a sequence encoding the FLAG peptide [260], which enables detection of the fusion protein using commercially available antibodies (Sigma), and a tract of glycine residues to promote flexibility between XpA* and the N-terminal additions. A pair of synthetic oligonucleotides were created which, when annealed together, can form a double-stranded DNA molecule which encodes the nuclear localization sequence corresponding to that found in simian virus 40 T-antigen [257], the FLAG peptide and the glycine tract. The nucleotide sequence encoding these components were: NLS-FLAG-Gly-sense (5'-GATCCAAAAAAATGGCTCCTAAGAAGAAGAGAAAGGTTAACGOTGATTA CAAGGATGATGATGATAAGCCCGGGGGTGGAGGTGGAGGTGGAGGTGGA GGTGGAGGC-3'); and NLS-FLAG-Gly-antisense (5'-GCCTCCACCTCCACCTCCACCTCCACCTCCACCCCCGGGCTTATCATCATC ATCCTTGTAATCACCGTTAACCTTTCTCTTCTTCITAGGAGCCATTTTTTTG-3'). These oligonucleotides when annealed together forming a cohesive end at the 5' end corresponding to the BamHI site and a cohesive end at the 3' end corresponding to the SfoI site. The two oligonucleotides were annealed together as per instructions supplied by the supplier (National Research Council-Plant Biotechnology Institute). pAS5 was digested with BamHI and SfoI and the resulting ~4 kb fragment was purified by agarose gel electrophoresis and recovered from the agarose as described above. The pAS5 fragment and the annealed oligonucleotide were ligated together, transformed into *E. coli* and putative clones of the assembly identified as described above. The DNA sequence of the resultant clone, pSCK5, was determined to confirm it encoded XpA* fused at the N-terminus to the NLS from SV40 T-antigen, followed by the FLAG peptide and a glycine tract. This gene assembly encoded by pSCK5 will henceforth be referred to as NLS-XpA*.

A second version of XpA was cloned to as an example of a means to promote stability of constructs possessing this gene in E. coli. Evidence in the literature points to the XpA and derived XpA* gene having toxic effects when propagated in E. coli [271;272]. To reduce possible antagonistic activity of XpA in E. coli two exemplary 80 approaches include changing amino acid residue #303 from a tyrosine to a histidine [271] or placing an intron or other intervening sequence in the open reading frame of the gene which cannot be excised in E. coli thereby inhibiting functional expression of the XpA gene in E. coli. These two examples may also be applied to promote stability in E. coli of constructs possessing XpA*. Other approaches may also be used for effective applications of XpA or XpA*, and derivatives thereof, in eukaryotic and prokaryotic cells without employing the insertions in the gene or residue changes outlined here. To achieve the amino acid residue change PCR primers XpA-5'Sal-RBS-BamSma and XpA-3'-Y303H-XbaSph are combined, and XpA-5'-Y303H-XbaSph and XpA-3'HIIINotISacSfo are combined in separate PCR reactions with XpA as template. The fragments are digested with SphI and ligated together into a cloning vector. The resulting resynthesized XpA gene has the Y303H mutation and will be less antagonistic to E. coli viability [271]. The second approach involves cloning an intron into the XpA gene which cannot be spliced out in E. coli and produces frame-shift or non-sense mutations which cause non-functional translation protein products to result from this assembly if expressed in E. coli. An intron which could be spliced out of the XpA gene, or variants thereof, when expressed in eukaryotic yeast cells was created in a manner as described by Yoshimatsu and Nagawa (1989) [273]. To achieve this, oligonucleotides yIntron-5'S and yIntron-5'AS were annealed together in one reaction, as per instructions supplied by the supplier (InVitrogen), and yIntron-3'S and yIntron-3' AS were similarly annealed together. This results in two double-stranded DNA molecules which share a common SacI cohesive end and have unique respective HindIII and EcoRI sites. This combined ~100 bp fragment encoding the yeast intron was cloned into the HindIII and EcoRI site of pUC18 [274] resulting in pNML13. pNML13 was then digested with SnaBI and PvuI. pNML7-8 was digested with StuI and treated with calf intestinal phosphatase as per standard procedures [256]. The resulting ~110 bp DNA fragment from pNML13 and the ~4.5 kb fragment from pNML7-8 were purified by agarose gel electrophoresis and recovered from the agarose as described above. The fragments were ligated together, transformed into *E. coli* and putative clones of the assembly identified as described above. The resultant clone with the yeast intron in the StuI site of XpA in a sense orientation with respect to the gene (i.e. XpA::yIntron) was denoted pMW244. The intron may also be placed at other sites in the XpA gene, or variants thereof, such as the BsaAI site to achieve a similar effect.

An intron which could be spliced out of the XpA gene, or variants thereof, when expressed in eukaryotic plant cells was also created. To achieve this, oligonucleotides EF 1 B-Intron-5'HIIISna and Efl B-Intron-3'RIPvu were used in a PCR reaction to amplify the first intron of the eEF-1β gene cloned from *Arabidopsis thaliana.* The amplified ~120 bp fragment can then be digested with SnaBI and PvuII to create blunt ends on the intron which may then be ligated into the XpA gene, or variants thereof, digested, for example, with a restriction enzyme that also creates blunt ends. Resultant clones can then be analysed to identify ones where the intron is in the sense orientation with respect to the XpA gene so that the intron may be effectively spliced out when the gene is expressed in plant cells.

A third version of the XpA gene was cloned so that the resultant protein would encode a nuclear localization sequence (NLS) at the N-terminus of the protein (i.e. NLS-XpA) followed by the FLAG peptide [260], which enables detection of the fusion protein using commercially available antibodies (Sigma), and a tract of glycine residues to promote flexibility between XpA and the N-terminal additions. pMW244 was digested with SmaI and NotI. pSCK10, which encodes NLS-XpA* from pSCK5 adjacent to a ribosome binding site in pENTR1A, was digested with SfoI and NotI. DNA fragments of interest corresponding to XpA::yIntron (~1.6 kb) and the NLS and pENTRIA fragment of pSCK10 (~2.4 kb) were purified by agarose gel electrophoresis and recovered from the agarose as described above. The fragments were ligated together, transformed into *E. coli* and putative clones of the gene in the vector identified. The NLS-XpA::yIntron may then be transferred to yeast expression vectors (e.g. YCplac22-Tet2x-DEST or YEplac112-Tet7x-DEST) via the Clonase (Gibco BRL) reaction.

The XpA gene naturally encodes the recognition sequence for the nicking activity of the XpA protein ~320 bp 3' of the start codon [275]. In some embodiments, the XpA gene is modified so that the XpA nickase recognition sequence is modified so that this DNA is no longer efficiently nicked by XpA. As an example of how to change the nickase recognition sequence, PCR may be used to generate a new version of the XpA gene no longer encoding the native nickase recognition sequence. Two separate PCR reactions may be done with either φX174 viral RF I DNA (New England BioLabs) or pNML7-8 as template with oligonucleotide primers XpA-5'Sal-RBS-BamSma combined with XpA-Bind-Anti-Cla and XpA-3'HIIINotSacSfo combined with XpA-Bind-Sense-Cla. The ~340 bp fragment resulting from amplification with XpA-5'Sal-RBS-BamSma combined with XpA-Bind-And-Cla and the ~1.2 kb fragment resulting from amplification with XpA-3'HIIINotSacSfo combined with XpA-Bind-Sense-Cla are purified, cleaved with ClaI and ligated together into a vector. The primers XpA-Bind-Anti-Cla and XpA-Bind-Sense-Cla incorporate nucleotide changes that maintain the amino acid sequence of the XpA gene but reduce the function of the nickase recognition sequence. This modified XpA gene may then be expressed in this form or be engineered to encode a NLS at the N-terminus or C-terminus or within the interior of the protein.

### B2. Expression constructs for XpA and its variants

As one means to achieve expression of XpA*, the gene was first cloned into pENTR11 (Gibco BRL). pAS5 encoding XpA* was first cut with SfoI and NotI and pENTR11 was digested with XmnI and NotI. DNA fragments of interest corresponding to XpA* (~1.1 kb) and pENTR11 (~2.3 kb) were purified by agarose gel electrophoresis and recovered from the agarose as described above. The fragments were ligated together, transformed into *E. coli* and putative clones of the gene in the vector were identified. The resultant clone of XpA* in PENT11 was denoted pAS 10.

The gene encoding NLS-XpA* was cloned into pENTR1A (Gibco BRL). pSCK5 encoding NLS-XpA* was first cut with BamHI and XhoI and pNML6, a derivative of pENTRIA encoding a ribosome binding site 3' of the SalI site and 5' of the BamHI site in the multiple-cloning site of the vector, was digested with BamHI and XhoI. DNA fragments of interest corresponding to NLS-XpA* (~1.2 kb) and pENTRIA (~2.3 kb) were purified by agarose gel electrophoresis and recovered from the agarose as described above. The fragments were ligated together, transformed into E. *coli* and putative clones of the gene in the vector were identified. The resultant clone of NLS-XpA* linked to a ribosome binding site in pENTR1A was denoted pSCK10.

The gene encoding NLS-XpA::yIntron may also be cloned into pENTR1A (Gibco BRL) in a similar manner as described for pSCK10 above.

Using the Gateway (Gibco BRL) cloning system genes encoding XpA or XpA*, and variants thereof, may be transferred to vectors for expression in eukaryotic yeast, plant or animal cells or prokaryotic cells like E. coli. For example, NLS-XpA* or NLS-XpA may be transferred to YCplac22-Tet2X::DEST or YEplac112-Tet7x::DEST for expression in eukaryotic yeast cells or plant or animal cell vectors possessing a Destination cassette (Gibco BRL) appropriately arranged with an appropriate promoter to facilitate expression of the gene. Versions of XpA and XpA* with or without NLS sequences or intervening introns or altered sequences described here may also be transferred to vectors for expression in eukaryotic yeast, plant or animal cells in a similar fashion as used for the variants described here employing either restriction enzymes alone or restriction enzymes in concert with the Gateway (Gibco BRL) or other cloning approach.

### B3. Cloning of φX174 origin elements and derivatives

Sequences corresponding to the φX174 origin of replication which may be used to initiate or terminate DNA replication as part of a gene targeting system were cloned after amplification by PCR. Template for amplifying φX174-initiator was φX174 viral RF I DNA (New England BioLabs). PCR reactions were performed with approximately 1 µg of viral DNA as template, 1.0 pmol each of primers XpA-INIT-5'BamPme and XpA-INIT-3'PacMscSac, 0.2 mM dNTP's, 2.5 U Pfx (Gibco BRL) and Pfx buffer constituents recommended by the manufacturer in a volume of 50 µl. Template for amplifying φX174-terminator was φX174 viral RF I DNA (New England BioLabs). PCR reactions were performed with approximately 1 µg of viral DNA as template, 1.0 pmol each of primers XpA-TERM-5'XhoAscRV and XpA-TERM-3'NotSal, 0.2 mM dNTP's, 2.5 U Pfu (Stratagene) and Pfu buffer constituents recommended by the manufacturer in a volume of 50 µl. The PCR conditions were 5 min @ 94 C, followed by 35 cycles of 30 s @ 94 C, 30 s @ 60 C and 30s @ 68 C, followed by 10 min @ 72 C and storage at 4 C or -20 C. After completion of the cycling, the DNA from the reaction to amplify the φX174-initiator was digested with BamHI and the DNA from the reaction to amplify the φX174-terminator was digested with SalI. The plasmid cloning vector YEplac181 [268] was digested with BamHI and SalI. DNA fragments of interest corresponding to φX174-initiator (∼0.3 kb), φX174-terminator (∼0.3 kb), and the YEplac181 vector (∼5.8 kb) were purified by agarose gel electrophoresis and recovered from the agarose as described above. The fragments were ligated together, transformed into E. *coli* and putative clones of the gene identified as described above. The DNA sequence of the resultant clone, pNML1, was determined to confirm it encoded φX174-initiator::terminator. The φX174-initiator and φX174-terminator can be linked with an adjoining or intervening sequence to facilitate amplification of this sequence in conjunction with the action of the XpA protein or derivatives thereof.

Sequences corresponding to the φX174 origin of replication which may be used to initiate or terminate DNA replication were also cloned by incorporation of the recognition sequence for XpAinto oligonucleotides used in a PCR amplification. PCR reactions were performed with approximately 1 µg of pMW105 (encoding the ura3ΔEcoRV-NcoI allele) as template, 1.0 pmol each of primers 5'Xori-URA and 3'Xori-URA, 0.2 mM dNTP's, 2.5 U Taq (Pharmacia) and Opti-Prime Buffer 4 (Stratagene) buffer constituents recommended by the manufacturer in a volume of 50 µl. The PCR conditions were 5 min @ 94 C, followed by 35 cycles of 30 s @ 94 C, 30 s @ 60 C and 2 min @ 72 C, followed by 10 min @ 72 C and storage at 4 C or -20 C. After completion of the cycling, the DNA from the reaction to amplify the ΔX174-initiator::terminator with the intervening ura3ΔEcoRV-NcoI allele was digested with BamHI and SalI. The plasmid cloning vector pSPORT2 (Gibco BRL) was digested with BamHI and SalI. DNA fragments of interest corresponding to φX174-initiator::terminator with the intervening ura3ΔEcoRV-NcoI allele (∼2 kp), and the pSPORT2vector (∼4.3 kb) were purified by agarose gel electrophoresis and recovered from the agarose as described above. The fragments were ligated together, transformed into *E*. *coli* and putative clones of the gene identified as described above. The DNA sequence of the resultant clone, pAS6, was determined to confirm it encoded φX174-initiator::terminator with the intervening ura3ΔEcoRV-NcoI allele.

### B4. Constructs for assaying XpA and its variants

To assay XpA or XpA* and their variants in eukaryotes, the φX174-initiator and φX174-terminator sequences, with and without an intervening sequence to be replicated, and the various forms of XpA or XpA* were cloned to enable contransformation of different combinations of these elements into yeast. As an example of sequences to be replicated using the invention, the URA3 gene from *Saccharomyces cerevisiae* was used.

The URA3 alleles described above were linked to φX174-initiator and φX174-terminator sequences and cloned into shuttle vectors for introduction into eukaryotic yeast cells. To transfer the ura3ΔPstI-EcoRV allele into a yeast shuttle vector, pMW180 was digested with SmaI and XhoI. pNML1 was digested with MscI and XhoI. The resulting ∼6.5 kb DNA fragment from pNML1 and the ∼1.6 kb fragment from pMW180 were purified by agarose gel electrophoresis and recovered from the agarose as described above. The fragments were ligated together, transformed into *E*. *coli* and putative clones of the assembly identified as described above. The resultant clone of the φX174-initiator and φX174-terminator sequences with a ∼1.6 kb ura3ΔPstI-F-coRV allele intervening sequence in YEplac181 [268] was denoted pMW188. pMW188 and YEplac181-Tet2x were digested with BamHI andNotI. The resulting ∼2.2 kb DNA fragment from pMW188 and the ∼8.3kb) fragment from YEplac181-Tet2x were purified by agarose gel electrophoresis and recovered from the agarose as described above. The fragments were ligated together, transformed into *E*. *coli* and putative clones of the assembly identified as described above. The resultant clone of the φX174-initiator and φX174-terminator sequences with a ∼1.6 kb ura3ΔPstI-EcoRV allele intervening sequence in YEplac181-Tet2x was denoted pMW240

The ura3ΔPstI-EcoRV allele linked to φX174-initiator and φX174-terminator sequences was also cloned for integration into the chromosome of eukaryotic yeast cells. To enable integration of the φX174-initiator and φX174-terminator::ura3ΔPstI-EcoRV assembly into a chromosome, digestion of pMW240 with EcoRI and NotI followed by treatment of the DNA ends with T4 polymerase releases a ∼4.5 kb DNA fragment with blunt ends. Alternatively, PvuII digestion of pMW240 enables purification of a ∼5.1 kb DNA fragment with blunt ends. pHO-poly-KanMX4-HO [267] is digested with SmaI and treated with calf intestinal phosphatase following standard procedures [256]. The resulting DNA fragment from pMW240 and the ∼6.1 kb fragment from pHO-poly-KanMX4-HO are purified by agarose gel electrophoresis and recovered from the agarose as described above. The fragments are ligated together, transformed into *E*. *coli* and putative clones of the assembly identified as described above. The resultant clone of the φX174-initiator and φX174-terminator-sequences with a ∼1.6 kb ura3ΔPstI-EcoRV intervening sequence in the chromosomal integrating vector pHO-poly-KanMX4-HO is thus created. Using appropriate restriction enzyme combinations, the φX174-initiator and φX174-terminator sequences with a ∼1.6 kb ura3ΔPstI-EcoRV allele intervening sequence from pMW188 can also be placed in YIplac128 [268].

### C. Cloning of genetic elements from TYLCV

### C1. Cloning of RepC1 and derivatives and expression constructs

Template for amplifying TYLCV (Tomato Yellow Leaf Curl Virus) components was clone (pSP98) of the TYLCV bigeminivirus strain Sar Isolate M obtained from the American Type Culture Collection (Item # PVMC-25). To clone the RepC1 gene PCR reactions were performed with approximately 1 µg of pSP98 as template, 1.0 pmol each of primers Mor-C1-5'Bam and Mor-C1-3'NotXho, 0.2 mM dNTP's, 2.5 U Pfx (Gibco BRL) and Pfx buffer constituents recommended by the manufacturer in a volume of 50 µl. The PCR conditions were 5 min @ 94 C, followed by 25 cycles of 30 s @ 94 C, 30 s @ 58 C and 1 min @ 68 C, followed by 10 min @ 72 C and storage at 4 C or -20 C. After completion of the cycling the DNA was digested with BamHI and NotI. The plasmid cloning vector pENT3C (Gibco BRL) was digested with BamHI and NotI. DNA fragments of interest corresponding to RepC1 (∼1.1 kb) and the vector (∼2.2 kb) were purified by agarose gel electrophoresis and recovered from the agarose as described above. The fragments were ligated together, transformed into *E*. *coli* and putative clones of the gene identified as described above. The DNA sequence of the resultant clone, pNML2, was determined to confirm it encoded RepC1 from TYLCV.

A second version of the RepC1 gene was cloned whereby a ribosome binding site was placed upstream of the RepC1 open reading frame. PCR reactions were performed using an aliquot of the primary PCR reaction used to create pNML2 (i.e. with Mor-C1-5'Bam and Mor-C1-3'NotXho primers and pSP98 as template) in a secondary PCR reaction with 1.0 pmol each of primers Mor-C1-5'Sal-RBS-Bam and Mor-C1-3'NotXho, 0.2 mM dNTP's, 2.5 U Pfx (Gibco BRL) and Pfx buffer constituents recommended by the manufacturer in a volume of 50 µl. The PCR conditions were 5 min @ 94 C, followed by 25 cycles of 30 s @ 94 C, 30 s @ 58 C and 1 min @ 68 C, followed by 10 min @ 72 C and storage at 4 C or-20 C. After completion of the cycling the DNA was digested with NotI. The plasmid cloning vector pENT1A (Gibco BRL) was digested with DraI and NotI. DNA fragments of interest corresponding to RepC1 (∼1.1 kb) and the vector (∼2.2 kb) were purified by agarose gel electrophoresis and recovered from the agarose as described above. The fragments were ligated together, transformed into *E*. *coli* and putative clones of the gene identified as described above. The DNA sequence of the resultant clone, pNML9, was determined to confirm it encoded RepC1 from TYLCV.

Plasmid constructs were assembled to facilitate expression of RepC1 and its variants in eukaryotic yeast RepC1 was cloned into the expression vector YCplac22-Tet2x by using the DNA fragment encoding RepC1 generated in a PCR reaction as described to create pNML2 (i.e. with Mor-C1-5'Bam and Mor-C1-3'NotXho primers and pSP98 as template). This DNA fragment and the vector YCplac22-Tet2x were both digested with BamHI and NotI. The resulting ∼1.1 kb fragment encoding RepC1 and the ∼7.4 kb DNA fragment from YCplac22-Tet2x were purified by agarose gel electrophoresis and recovered from the agarose as described above. The fragments were ligated together, transformed into *E*. *coli* and putative clones of the assembly identified as described above. The resultant clone of the TYLCV RepC1 in YCplac22-Tet2x was denoted pNML4. RepC1 was also cloned into the expression vector YEplac112-Tet7x by using the DNA fragment encoding RepC1 generated in a PCR reaction as described to create pNML2 (i.e. with Mor-C1-5'Bam and Mor-C1-3'NotXho primers and pSP98 as template). This DNA fragment and the vector YEplac112-Tet7x were both digested with BamHI and NotI. The resulting ∼1.1 kb fragment encoding RepC1 and the ∼7.8 kb DNA fragment from YEplac112-Tet7x were purified by agarose gel electrophoresis and recovered from the agarose as described above. The fragments were ligated together, transformed into *E*. *coli* and putative clones of the assembly identified as described above. The resultant clone of the TYLCV RepC1 in YEplac112-Tet7x was denoted pNML3.

Using the Gateway (Gibco BRL) cloning system genes encoding RepC1, and variants thereof, may be transferred to vectors for expression in eukaryotic yeast, plant or animal cells or prokaryotic cells like E. coli. For example, RepC1 may be transferred to vectors possessing a Destination cassette (Gibco BRL) appropriately arranged with an appropriate promoter to facilitate expression of the gene in plant cells or animal cells or yeast cells or prokaryotic cells. Versions of RepC1 with or without NLS sequences or intervening introns or altered sequences described here may also be transferred to vectors for expression in eukaryotic yeast, plant or animal cells in a similar fashion as used for the variants described here employing either restriction enzymes alone or restriction enzymes in concert with the Gateway (Gibco BRL) or other cloning approach.

### C2. Cloning of TYLCV origin elements and derivatives

Sequences corresponding to the TYLCV origin of replication which may be used to initiate or terminate DNA replication as part of a gene targeting system were cloned after amplification by PCR. Template for amplifying TYLCV-initiator was pSP98 encoding the TYLCV bigeminivirus strain Sar Isolate M obtained from the American Type Culture Collection (Item # PVMC-25). PCR reactions were performed with approximately 1 µg of pSP98 DNA as template, 1.0 pmol each of primers Mor-INIT-5'BamPme and Mor-INIT-3'SacMscPac, 0.2 mM dNTP's, 2.5 U Pfu (Stratagene) and Pfu buffer constituents recommended by the manufacturer in a volume of 50 µl. Template for amplifying TYLCV-terminator was also was pSP98. PCR reactions were performed with approximately 1 µg of viral DNA as template, 1.0 pmol each of primers Mor-TERM-5'XhoAscRV and Mor-TERM-3'XbaNot, 0.2 mM dNTP's, 2.5 U Pfx (Gibco BRL) and Pfx buffer constituents recommended by the manufacturer in a volume of 50 µl. The PCR conditions were 5 min @ 94 C, followed by 25 cycles of 30 s @ 94 C, 30 s @ 60 C and 30 main @ 68 C, followed by 10 min @ 72 C and storage at 4 C or -20 C. After completion of the cycling, the DNA from the reaction to amplify the TYLCV-initiator was digested with BamHI and the DNA from the reaction to amplify the TYLCV-terminator was digested with XbaI. The plasmid cloning vector YEplac181 [268] was digested with BamHI and XbaI. DNA fragments of interest corresponding to TYLCV-initiator (∼0.3 kb), TYLCV-terminator (∼0.3 kb), and the YEplac181 vector (∼5.8 kb) were purified by agarose gel electrophoresis and recovered from the agarose as described above. The fragments were ligated together, transformed into *E*. *coli* and putative clones of the gene identified as described above. The DNA sequence of the resultant clone, pNML5, was determined to confirm it encoded TYLCV-initiator::terminator. The TYLCV-initiator and TYLCV-terminator can be linked with an adjoining or intervening sequence to facilitate amplification of this sequence in conjunction with the action of the TYLCV-RepC1 protein.

### C3. Constructs for assaying RepC1 and its variants

To assay RepC1 and variants thereof in eukaryotes, the TYLCV-initiator::terminator sequences, with and without an intervening sequence to be replicated, and the various forms of RepC1 were cloned to enable cotransformation of different combinations of these elements into yeast. As an example of reproducible sequences to be replicated using the invention, the URA3 gene from *Saccharomyces cerevisiae* was used.

The URA3 alleles described above were linked to TYLCV-initiator::terminator sequences and cloned into shuttle vectors for introduction into eukaryotic yeast cells. To transfer the ura3ΔPstI-EcoRV allele into a yeast shuttle vector, pMW180 was digested with SmaI and XhoI. pNML5 was digested with MscI and XhoI. The resulting ∼6.5 kb DNA fragment from pNML5 and the ∼1.6 kb fragment from pMW180 were purified by agarose gel electrophoresis and recovered from the agarose as described above. The fragments were ligated together, transformed into *E*. *coli* and putative clones of the assembly identified as described above. The resultant clone of the TYLCV-initiator and TYLCV-terminator sequences with a ∼1.6 kb ura3ΔPstI-EcoRV allele intervening sequence in YEplac181 [268] was denoted pMW201. pMW201 and YEplac181-Tet2x were digested with BamHI and NotI. The resulting ∼2.2 kb DNA fragment from pMW201 and the ∼8.3kb) fragment from YEplac181-Tet2x were purified by agarose gel electrophoresis and recovered from the agarose as described above. The fragments were ligated together, transformed into *E*. *coli* and putative clones of the assembly identified as described above. The resultant clone of the TYLCV-initiator and TYLCV-terminator sequences with a ∼1.6 kb ura3ΔPstI-EcoRV allele intervening sequence in YEplac181-Tet2x was denoted pNML17.

The ura3ΔPstI-EcoRV allele linked to TYLCV-initiator and TYLCV-terminator sequences was also cloned for integration into the chromosome of eukaryotic yeast cells. To enable integration of the TYLCV-initiator and TYLCV-terminator::ura3ΔPstI-EcoRV assembly into a chromosome, digestion of pNML17 with EcoRI and NotI followed by treatment of the DNA ends with T4 polymerase releases a ∼4.5 kb DNA fragment with blunt ends. Alternatively, PvuII digestion of pNML17 enables purification of a ∼5.1 kb DNA fragment with blunt ends. pHO-poly-KanMX4-HO [267] is digested with SmaI and treated with calf intestinal phosphatase following standard procedures [256]. The resulting DNA fragment from pNML17 and the ∼6.1 kb fragment from pHO-poly-KanMX4-HO are purified by agarose gel electrophoresis and recovered from the agarose as described above. The fragments are ligated together, transformed into *E*. *coli* and putative clones of the assembly identified as described above. The resultant clone of the TYLCV-initiator and TYLCV-terminator sequences with a ∼1.6 kb ura3ΔPstI-EcoRV intervening sequence in the chromosomal integrating vector pHO-poly-KanMX4-HO is thus created. Using appropriate restriction enzyme combinations, the TYLCV-initiator and TYLCV-terminator sequences with a ∼1.6 kb ura3ΔPstI-EcoRV allele intervening sequence from pNML17 can also be placed in YIplac128 [268].

In a similar fashion as to the cloning and application of components from begomovirus-type viruses like, for example, TYLCV, components from mastrevirus-type viruses like, for example, Wheat Dwarf Virus (WDV) may be cloned and used. WDV elements may be more functional in monocotyledonous plant species than elements from viral isolates which normally infect dicotyledonous species. An isolate of the WDV was obtained from the American Type Culture Collection (Item # 45046) as the clone pspT19WDV1. Based on the sequence of the WDV genome as determined by Woolston et al., (1988) [276] oligonucleotide primers were designed to enable amplification and cloning of the nickase and replication origin from this virus. The RepC1-like gene, as is common in many gemini virus strains which infect monocotyledonous plants, is encoded by a transcript which encodes two different proteins in two distinct but overlapping open reading frames [277]. Expression of the full-length open reading frame requires splicing of an intron-like sequence within the WDV genome region coding for RepC1-Iike protein. The WDV-RepC1-like gene may thus be cloned by creating cDNA from mRNA isolated from WDV-infected plant tissues, as per standard procedures [256], as part of a RT-PCR reaction with the oligonucleotide primers WD-C1-5'Bam and WD-C1-3'NotPst. Alternatively, the WDV-RepC1-like gene may be amplified from the cloned WDV genome in a plasmid vector. In this approach, two separate primary PCR reactions would be done using pspT19WDV1 as template with WD-C1-5'Bam and WDV-C1-Nterm-3"+25bp- span as primers in one reaction and WD-C1-3'NotPst and WDV-C1-Cterm-5'+25bp-span as primers in a second reaction. The primers WDV-C1-Nterm-3"+25bp-span and WDV-C1-Cterm-5'+25bp-span share 25 bp of complementarity so that the ends of the two fragments produced in the primary PCR reactions will be able to anneal with each other in a secondary PCR reaction. By adding only WD-C1-5'Bam and WD-C1-3'NotPst as primers in this secondary PCR reaction, the full-length open reading frame encoding WDV-RepC1-like protein may be amplified.

Sequences corresponding to the WDV origin of replication which may be used to initiate or terminate DNA replication may also be cloned after amplification by PCR. Using the cloned WDV genome as template in PCR reactions with WD-INIT-5'BamPme and WD-INIT-3'PacMscSac as primers will amplify a ∼410 bp fragment encoding the WDV-initiator. Using the cloned WDV genome as template in PCR reactions with WD-TERM-5'XhoAscRV and WD-TERM-3'NotSal as primers will amplify a ∼410 bp fragment encoding the WDV-terminator. These two fragments can be linked with an adjoining or intervening sequence to facilitate its amplification in conjunction with the action of the WDV-RepC1-like protein.

### D. Cloning of a helicase

The action of nickases, for example g2p, XpA and RepC1, to promote DNA replication at their cognate recognition sequences may be enhanced by helicases [278]. As an example of a helicase which might be used to enhance nickase function the REP helicase of E. coli [279] was cloned. Alternative proteins from eukaryotic, prokaryotic or viral genomes may also be applied to enhancing the action of nickases to promote DNA replication at specific recognition sequences. Such proteins may for example be identified by protein-protein interaction assays, such as the yeast two-hybrid system [330]. To provide template DNA for use in a PCR reaction to amplify the REP gene, genomic DNA was purified from E. coli JM101 [280] following standard procedures [256]. To clone the REP gene PCR reactions were performed with approximately 1 µg of JM101 genomic DNA as template, 1.0 pmol each of primers REP-5'Sal-RBS-BamSma and REP-3'NotXhoSfo, 0.2 mM dNTP's, 2.5 U Pfx (Gibco BRL) and Pfx buffer constituents recommended by the manufacturer in a volume of 50 µl. The PCR conditions were 5 min @ 94 C, followed by 25 cycles of 30 s @ 94 C, 30 s @ 58 C and 2 min @ 68 C, followed by 10 min @ 72 C and storage at 4 C or -20 C. After completion of the cycling the DNA was digested with SalI and NotI. The plasmid cloning vector pENT1A (Gibco BRL) was digested with SalI and NotI. DNA fragments of interest corresponding to REP (∼1.9 kb) and the vector (∼2.2 kb) were purified by agarose gel electrophoresis and recovered from the agarose as described above. The fragments were ligated together, transformed into E. *coli* and putative clones of the gene identified as described above. The DNA sequence of the resultant clone, pNML210, was determined to confirm it encoded REP from E. coli.

The arrangement of the SmaI and SfoI restriction sites at the respective 5' and 3' end of the cloned REP gene enables linking of the REP gene to DNA fragments encoding NLS sequences, such as those described for pSCK5 and pAS4, at the N-terminus or C-terminus of the REP protein. The function of REP in promoting DNA replication in eukaryotic cells may be enhanced if it is attached to a NLS since the large size of REP protein might reduce its ability to localize and function in the eukaryotic nucleus. To engineer the REP protein so that it encodes an NLS on the C-terminus pNML10 was digested with BamHI and SfoI and pAS4 was digested with SfoI and XbaI. The yeast expression vector pESC-TRP (Stratagene) was digested with BamHI and NheI. The cohesive end at the 3' end of the C-terminal NLS fragment created by digestion with XbaI is compatible with the cohesive end of pESC-TRP created by digestion with NheI. DNA fragments of interest corresponding to REP (∼1.9 kb), C-terminal NLS (∼150 bp), and the pESC-TRP vector (∼6.5 kb) were purified by agarose gel electrophoresis and recovered from the agarose as described above. The fragments were ligated together, transformed into *E*. *coli* and putative clones of the gene identified as described above. The resultant clone of the E. coli REP helicase engineered to encode a NLS at its C-terminus (i.e. referred to as REP-NLS) was denoted pNML24. REP helicase could also be engineered to encode a NLS at its N-terminus or within the interior of the protein. To clone an NLS at the N-terminus of REP, pSCK5 or pSCK10 may be digested with SfoI and NotI and the corresponding vector fragment encoding the NLS be isolated. pNML10 may be digested with SmaI and NotI and ligated to the isolated vector plus NLS sequence. This would result in a clone of the E. coli REP helicase engineered to encode a NLS at its N-terminus (i.e. referred to as NLS-REP).

pESC-TRP (Stratagene), the vector backbone for pNML24, encodes an φf1 origin of replication within the vector backbone. To delete the φf1 origin sequences in the vector backbone of pESC-TRP recombinogenic cloning was employed [281] was applied. The kanamycin marker in pKD 13 [282] was amplified in a PCR reaction using the oligonucleotides P1-f1-delta and P4-f1-delta. The amplicon was purified and either co-transformed with pNML24 into E. coli EL250 [281] or the amplicon was transformed into an EL250 derived strain that already carried pNML24. Following the recombinogenic cloning procedure [281] clones of pNML24 were isolated which had the φf1 origin sequence of pESC-TRP replaced by the kanamycin marker of pKD13. In some clones the recombinogenic cloning procedure [281] was continued so as to eliminate the kanamycin marker from the vector by the action of FLP recombinase.

The various forms of E. coli REP helicase were cloned into various E. coli, yeast and plant expression vectors for further analysis. REP was cloned into the expression vector pMW137 by using the Clonase (Gibco BRL) reaction, following the directions supplied by the manufacturer, to transfer the gene from pNML20. The resultant clone of REP in pMW137 was denoted pNML29. REP-NLS was cloned into the expression vector pMW137 by first cloning the REP-NLS encoding DNA fragment into pENTR1A encoding a ribosome binding site. pNML10 was digested with XhoI and the ends of the DNA then made blunt by treatment with Klenow polymerase, as per standard procedures [256], followed by digestion with BamHI. pNML24 was digested with PstI and the ends of the DNA then made blunt by treatment with Klenow polymerase, as per standard procedures [256], followed by digestion with BamHI. The resulting ∼2.2 kb DNA fragment from pNML10 and the ∼2.1kb, fragment from pNML24 were purified by agarose gel electrophoresis and recovered from the agarose as described above. The fragments were ligated together, transformed into *E*. *coli* and putative clones of the assembly identified as described above. The resultant clone of REP-NLS in pENT1A was denoted pNML27. REP-NLS was then cloned into the expression vector pMW 137 by using the Clonase (Gibco BRL) reaction, following the directions supplied by the manufacturer, to transfer the gene from pNML27. The resultant clone of REP-NLS in pMW137 was denoted pNML30.

REP-NLS was cloned into the expression vector YCplac22-Tet2x and YEplac112-Tet7x. pNML24 was digested with BamHI and PstI. YCplac22-Tet2x and YEplac112-Tet7x were each digested with BamHI and PstI. The resulting ∼2.1kb, DNA fragment from pNML24 and ∼7.4 kb DNA fragment from YCplac22-Tet2x and the ∼7.8 kb DNA fragment from YEplac 112-Tet7x were purified by agarose gel electrophoresis and recovered from the agarose as described above. The fragments were ligated together in two separate reactions, transformed into *E*. *coli* and putative clones of the assembly identified as described above. The resultant clone of REP-NLS in YCplac22-Tet2x was denoted pNML35. The resultant clone of REP-NLS in YEplac112-Tet7x was denoted pNML34.

Using the Gateway (Gibco BRL) cloning system genes encoding REP, and variants thereof, may be transferred to vectors for expression in eukaryotic yeast, plant or animal cells or prokaryotic cells like E. coli. For example, REP, NLS-REP or REP-NLS may be transferred to vectors possessing a Destination cassette (Gibco BRL) appropriately arranged with an appropriate promoter to facilitate expression of the gene in plant or animal cells. Versions of REP with or without NLS sequences or intervening introns or altered sequences described here may also be transferred to vectors for expression in eukaryotic yeast, plant or animal cells in a similar fashion as used for the variants described here employing either restriction enzymes alone or restriction enzymes in concert with the Gateway (Gibco BRL) or other cloning approach.

### E. Effect of Recombination Proteins

In other embodiments, the efficiency of gene targeting using the invention may be enhanced by increasing the inherent potential of a cell to catalyse homologous recombination events. This potential may be increased through elevated expression or activity of catalytic or structural proteins participating in facilitating homologous recombination events. Conversely, the frequency of homologous recombination events may be increased by decreasing the function of processes which compete with homologous recombination processes and which may promote non-homologous recombination events. Two examples of protein which may be used to promote homologous recombination are RAD51 and RAD52 which are functionally conserved amongst eukaryotes and prokaryotes [283-290]. To evaluate the effect of RAD51 and RAD52, yeast was used as a model eukaryote.

The yeast RAD51 (yRAD51) gene was cloned after amplification by PCR. Template for amplifying yRAD51 was genomic DNA from *Saccharomyces cerevisiae* strain AB972 [291] isolated by standard procedure [256]. Two PCR reactions were performed with approximately 1 µg of genomic DNA, 1.0 µmol yR51-5'Bam oligonucleotide and 1.0 pmol yR51-3'Pst oligonucleotide, 0.2 mM dNTP's, 2.5 U Pfu (Stratagene) and Pfu buffer constituents provided by the manufacturer in a volume of 50 µl. The PCR conditions were 5 min @ 94 C, followed by 25 cycles of 30 s @ 94 C, 30 s @ 58 C and 2.5 min @ 72 C, followed by 10 min @ 72 C and storage at 4 C or -20 C. The two reactions were pooled and DNA was digested with BamHI and PstI. The plasmid cloning vector pBluescript II KS- (Stratagene) was digested with BamHI and PstI. DNA fragments of interest corresponding to yRAD51 (∼1.2 kb) and the vector (∼3 kb) were purified by agarose gel electrophoresis and recovered from the agarose as described above. The fragments were ligated together, transformed into *E*. *coli* and putative clones of the gene identified as described above. The DNA sequence of the resultant clone, pMW35, was determined to confirm it encoded yRAD51.

The yeast RAD52 (yRAD52) gene was cloned after amplification by PCR. Template for amplifying yRAD52 was genomic DNA from *Saccharomyces cerevisiae* strain AB972 [291] isolated by standard procedure [256]. Two PCR reactions were performed with approximately 1 µg of genomic DNA, 1.0 pmol yR52-5'Pme oligonucleotide and 1.0 pmol yR52-3'Not oligonucleotide, 0.2 mM dNTP's, 2.5 U Pfu (Stratagene) and Pfu buffer constituents recommended by the manufacturer in a volume of 50 µl. The PCR conditions were 5 min @ 94 C, followed by 25 cycles of 30 s @ 94 C, 30 s @ 60 C and 2 min @ 72 C, followed by 10 min @ 72 C and storage at 4 C or -20 C. The two reactions were pooled and DNA was digested with EcoRI and NotI. The plasmid cloning vector pBluescript II SK- (Stratagene) was digested with EcoRI and NotI. DNA fragments of interest corresponding to yRAD52 (∼1.5 kb) and the vector (∼3 kb) were purified by agarose gel electrophoresis and recovered from the agarose as described above. The fragments were ligated together, transformed into *E*. *coli* and putative clones of the gene identified as described above. The DNA sequence of the resultant clone, pTK50, was determined to confirm it encoded yRAD52.

The yRAD51 gene was cloned into an expression vector. pMW35 and pESC-TRP (Stratagene) were each digested with BamHI and SalI. The resulting ∼1.2 kb DNA fragment from pMW35 and ∼6.5 kb DNA fragment from pESC-TRP were purified by agarose gel electrophoresis and recovered from the agarose as described above. The fragments were ligated together, transformed into *E*. *coli* and putative clones of the assembly identified as described above. This construct was then digested with NotI and the DNA ends made blunt by treatment with T4 DNA polymerase. To this the Destination cassette (Gibco BRL) was ligated. As a result, other genes like nickase genes like g2p-NLS, or REP-NLS helicase, may be cloned into this construct using the Clonase reaction (Gibco BRL).

The yRAD52 gene was cloned into an expression vector. pTK50 and pESC-TRP (Stratagene) were each digested with EcoRI and NotI. The resulting ∼1.5 kb DNA fragment from pTK50 and ∼6.5 kb DNA fragment from pESC-TRP were purified by agarose gel electrophoresis and recovered from the agarose as described above. The fragments were ligated together, transformed into *E*. *coli* and putative clones of the assembly identified as described above. The resultant clone of yRAD52 in pESC-TRP was denoted pNML26. This construct was then digested with ApaI and the DNA ends made blunt by treatment with T4 DNA polymerase. To this the Destination B cassette (Gibco BRL) was ligated resulting in pNML19. As a result, other genes like nickase genes like g2p-NLS, or REP-NLS helicase, may be cloned into this construct using the Clonase reaction (Gibco BRL).

### F. Plant Promoters

In some embodiments of the method of the invention, the invention enables production of gene targeting substrates during S-phase of the cell cycle. In some embodiments this is facilitated by linking the expression of Rep factor(s) to a transcription promoter that is expressed during S-phase. Two examples of such promoters are those facilitating transcription of the H4 histone and cyclin-D genes. H4 histone gene expression has been characterised in plants and analysis of the promoter indicates it is primarily active in dividing cells [292]. Expression of the cyclin-D family of genes has also been investigated by evaluating RNA levels [292-294]. Of the members of the Cyclin-D gene family in Arabidopsis, CycD3 appears to be expressed at the G1/S boundary [294).

A DNA sequence encoding a region of the promoter from the H4 histone gene of *Arabidopsis thaliana* was cloned. Template for amplifying the AtH4 promoter by PCR was genomic DNA from *Arabidopsis thaliana* ecotype Columbia isolated by standard procedure [256]. PCR reactions were performed with approximately 1 µg of genomic DNA, 1.0 pmol H4-Prom-5'KpnSac oligonucleotide and 1.0 pmol H4-Prom-3'BamXho oligonucleotide , 0.2 mM dNTP's, 2.5 U Pfx (Gibco BRL) and Pfx buffer constituents provided by the manufacturer in a volume of 50 µl. The PCR conditions were 5 min @ 94 C, followed by 25 cycles of 30 s @ 94 C, 30 s @ 58 C and 1 min @ 68 C, followed by 10 min @ 72 C and storage at 4 C or -20 C. The DNA was digested with KpnI and NcoI. pAVA393, a plasmid cloning vector derived from pBluescript II SK+ [295] was digested with KpnI and NcoI. DNA fragments of interest corresponding to AtH4 promoter (∼0.9 kb) and the vector (∼4 kb) were purified by agarose gel electrophoresis and recovered from the agarose as described above. The fragments were ligated together, transformed into *E*. *coli* and putative clones of the gene identified as described above. The DNA sequence of the resultant clone, pNML8, was determined to confirm it encoded the promoter region from the Arabidopsis H4 histone gene. pNML8 was digested with SstI and PstI and the ∼0.9 kb fragment encoding the AtH4 promoter was cloned into the SstI and PstI site of the plant transformation vector pCB302 [296] resulting in the clone denoted pNML12 which enabled analysis and application of the AtH4 promoter in plants. pNML8 was modified by PCR to incorporate additional restriction sites for BamHI, SnaBI and NcoI to the 3' end of the TEV translational enhancer sequence encoded by pAVA393 adjacent to the AtH4 promoter. pNML8 was used as template in a standard PCR reaction, as described above, with the oligonucleotide primers H4-Prom-5'KpnSac and TEV-3'NcoSnaBam. The DNA was digested with KpnI and NcoI as was pAVA393. DNA fragments of interest corresponding to AtH4 promoter plus TEV sequence (∼1 kb) and the vector (∼4 kb) were purified by agarose gel electrophoresis, recovered from the agarose, ligated together and transformed into E. coli, as described above. The resultant clone was denoted pNML11.

A DNA sequence encoding a region of the promoter from the cyclin-D3 (i.e. AtCycD3) of *Arabidopsis thaliana.* Template for amplifying the AtCycD3 promoter by PCR was genomic DNA from *Arabidopsis thaliana* ecotype Columbia isolated by standard procedure [256]. PCR reactions were performed with approximately 1 µg of genomic DNA, 1.0 pmol CycD3-Prom-5'KpnSac oligonucleotide and 1.0 pmol CycD3-Prom-3'Xho oligonucleotide , 0.2 mM dNTP's, 2.5 U Pfu Turbo (Stratagene) and buffer constituents provided by the manufacturer in a volume of 50 µl. The PCR conditions were 5 min @ 94 C, followed by 30 cycles of 30 s @ 94 C, 30 s @ 55 C and 2.5 min @ 72 C, followed by 10 min @ 72 C and storage at 4 C or -20 C. The DNA was digested with KpnI and NcoI. pAVA393, a plasmid cloning vector derived from pBluescript II SK+ [295] was digested with KpnI and NcoI. Alternatively, a primary PCR reaction may be done using the CycD3-Prom-5'X oligonucleotide and CycD3-Prom-3'X oligonucleotide with Arabidopsis ecotype Columbia genomic DNA as template. An aliquot of this reaction may then be used in a secondary PCR reaction with CycD3-Prom-5'KpnSac oligonucleotide and CycD3-Prom-3'Xho oligonucleotide. DNA fragments of interest corresponding to AtCycD3 promoter (∼1.1 kb) and the vector (∼4.1 kb) were purified by agarose gel electrophoresis and recovered from the agarose as described above. The fragments were ligated together, transformed into *E*. *coli* and putative clones of the gene identified and sequenced as described above. The resultant clone of the promoter region from the Arabidopsis AtCycD3 gene was denoted pTK159. The DNA fragment encoding the AtCycD3 promoter may then be cloned into a plant transformation vector like pCB302 [296] enabling analysis and application of the AtCycD3 promoter in plants.

In some embodiments, the invention enables production of gene targeting substrates coordinately with the expression of endogenous proteins facilitating recombination in mitotic and meiotic cells. In some embodiments this is facilitated by linking the expression of the Rep factor(s) to a transcription promoter that expresses a gene involved in homologous recombination. An example of such a promoter is that facilitating transcription of the RAD51 gene. RAD51 gene expression has been characterised in plants and analysis of the promoter indicates it is expressed in vegetative cells, particularly in response to exposure to DNA damaging agents, in reproductive tissues and in tissues undergoing cell division [297]. This pattern of expression is conserved in other eukaryotic species [298]. Template for amplifying the AtRAD51 promoter by PCR was genomic DNA from *Arabidopsis thaliana* ecotype Lansberg isolated by standard procedure [256]. A primary PCR reaction was performed with approximately 1 µg of genomic DNA as template, 1.0 pmol AtR51-Prom-5'X oligonucleotide and 1.0 pmol AtR51-Prom-3'EX oligonucleotide , 0.2 mM dNTP's, 2.5 U Pfx (Gibco BRL) and Pfx buffer constituents provided by the manufacturer in a volume of 50 µl. The PCR conditions were 5 min @ 94 C, followed by 35 cycles of 30 s @ 94 C, 30 s @ 56 C and 2 min @ 72 C, followed by 10 min @ 72 C and storage at 4 C or -20 C. An aliquot of this primary reaction was then used in a secondary PCR reaction with the oligonucleotide combination of AtR51-Prom-5'Sac and AtR51-Prom-3'Xho and Pfx polymerase and reaction conditions as described for the primary reaction. The DNA was digested with xhoI. pAVA393 [295] was digested with ApaI, treated with T4 polymerase to make the DNA ends blunt, and then digested with XhoI. DNA fragments of interest corresponding to AtRAD51 promoter (∼1.7 kb) and the vector (∼4.1 kb) were purified by agarose gel electrophoresis and recovered from the agarose as described above. The fragments were ligated together, transformed into *E. coli* and putative clones of the gene identified as described above. The DNA sequence of the resultant clone, pTK114, was determined to confirm it encoded ∼1.7 kb of the promoter region from the Arabidopsis AtRAD51 gene. In a similar fashion, smaller segments of the AtRAD51 promoter region were cloned using the oligonucleotides AtR51-Prom-5'Sac (-1 kb) and AtR51-Prom-5'Sac (-0.7 kb) to result in the clones pTK126 encoding ∼1.0 kb of the promoter region from the Arabidopsis AtRAD51 gene, and pTK127 encoding ∼0.7 kb of the promoter region from the Arabidopsis AtRAD51 gene. To enable analysis and application of the AtRAD51 promoter in plants, the cloned promoter fragments were transferred to plant transformation vectors. The DNA fragment encoding the AtRAD51 promoter from pTK114, pTK126 and pTK127 was isolated by digestion of the plasmids with SmaI and SacI. These fragments were then individually ligated to the plant transformation vector pCB302 [296] also digested with Smal and SacI resulting in the clones pTK139 (encoding the AtRAD51 promoter fragment as in pTK127), pTK140 (encoding the Trad5 promoter fragment as in pTK126), and pTK141 (encoding the AtRAD51 promoter fragment as in pTK114).

In some embodiments of the methods of the invention, the invention enables production of gene targeting substrates coordinately with the expression of endogenous proteins facilitating recombination in meiotic cells. In some embodiments this is facilitated by linking the expression of the Rep factor(s) to a transcription promoter that expresses a gene involved in homologous recombination in meiotic cells. Examples of such a promoter are those sequences facilitating transcription of the DMC1, MSH4 or SPO11 gene. The pattern of expression of these genes is conserved in eukaryotic species [299-301].

A DNA sequence encoding a region of the promoter from the DMC1 gene of *Arabidopsis thaliana* was cloned. Template for amplifying the AtDMC1 promoter by PCR was genomic DNA from *Arabidopsis thaliana* ecotype Lansberg isolated following standard procedures [256]. A primary PCR reaction was performed with approximately 1 µg of genomic DNA as template, 1.0 pmol DMC-Prom-5'Kpn-S1268 oligonucleotide and 1.0 pmol DMC-Prom-AS5408 oligonucleotide, 0.2 mM dNTP's, 2.5 U Pfx (Gibco BRL) and Pfx buffer constituents provided by the manufacturer in a volume of 50 µl. The PCR conditions were 5 min @ 94 C, followed by 35 cycles of 30 s @ 94 C, 30 s @ 63 C and 2 min @ 72 C, followed by 10 min @ 72 C and storage at 4 C or -20 C. An aliquot of this primary reaction was then used in a secondary PCR reaction with the oligonucleotide combination of DMC-Prom-5'Kpn-S1268 and DMC-Prom-Int2-NcoRV and Pfx polymerase and reaction conditions as described for the primary reaction except with an annealing temperature of 53 C. The amplified DNA was digested with KpnI. pBluescript II SK- (Stratagene) was digested with KpnI and EcoRV. DNA fragments of interest corresponding to AtDMC1 promoter (∼1.7 kb) and the vector (∼3 kb) were purified by agarose gel electrophoresis and recovered from the agarose as described above. The fragments were ligated together, transformed into *E*. *coli* and putative clones of the gene identified as described above. The DNA sequence of the resultant clone, pTK111, was determined to confirm it encoded ∼1.7 kb of the promoter region from the Arabidopsis AtDMC1 gene. A region 5' of the promoter sequence represented in pTK111 was also cloned. A PCR reaction was performed with approximately 1 µg of genomic DNA from *A*. *thaliana* ecotype Columbia, isolated as described above, was used as template, 1.0 pmol ADM-Prom-5'Kpn oligonucleotide and 1.0 pmol AtDMC-Pro-Nde-A1 oligonucleotide, 0.2 mM dNTP's, 2.5 U Pfu (Gibco BRL) and Pfu buffer constituents provided by the manufacturer in a volume of 50 µl. The PCR conditions were 5 min @ 94 C, followed by 30 cycles of 30 s @ 94 C, 30 s @ 55 C and 2 min @ 72 C, followed by 10 min @ 72 C and storage at 4 C or -20 C. The amplified DNA was digested with KpnI. pBluescript II SK- (Stratagene) was digested with KpnI and EcoRV. DNA fragments of interest corresponding to this upstream region of the AtDMC1 promoter (∼1.4 kb) and the vector (∼3 kb) were purified by agarose gel electrophoresis and recovered from the agarose as described above. The resultant clone was denoted pTK136. The cloned Arabidopsis DNA fragments of pTK111 and pTK136 could then be linked, as necessary, to create a ∼3 kb fragment encoding the promoter region of the AtDMC1 gene.

A derivative of the AtDMC1 promoter fragment encoded by pTK111 was created to remove the first intron of the AtDMC1 gene. pTK111 was used as template in a PCR reaction with oligonucleotides Universal Primer (Gibco BRL) and AtDMC-Prom-3'BamRVXho in a standard PCR reaction as described above using PfuTurbo (Stratagene) as a polymerase and annealing temperature of 55 C with extension time of 2.5 min for 30 cycles. The resulting DNA was digested with KpnI and XhoI and the ∼1.2 kb fragment purified. pNML14 was also digested with KpnI and XhoI and the vector portion purified. The vector and amplified fragment were ligated together and the resultant clone was denoted pTK138. The upstream fragment of the AtDMC1 promoter encoded by pTK136 was subcloned into pTK138 using KpnI and NdeI to isolate the respective fragments. The resultant clone was denoted pTK142.

A DNA sequence encoding a region of the promoter from the MSH4 gene of *Arabidopsis thaliana* was cloned. Template for amplifying the AtMSH4 promoter by PCR was genomic DNA from *Arabidopsis thaliana* ecotype Columbia isolated following standard procedure [256]. A PCR reaction was performed with approximately 1 µg of genomic DNA as template, 1.0 pmol AtMSH4-5'X oligonucleotide and 1.0 pmol AtMSH4-3'Bam oligonucleotide, 0.2 mM dNTP's, 2.5 U Pfu (Stratagene) and Pfu buffer constituents provided by the manufacturer in a volume of 50 µl. The PCR conditions were 5 min @ 94 C, followed by 35 cycles of 30 s @ 94 C, 30 s @ 60 C and 4 min @ 72 C, followed by 10 min @ 72 C and storage at 4 C or -20 C. The amplified DNA was digested with BamHI and KpnI. pBluescript II SK (Stratagene) was digested with BamHI and KpnI. DNA fragments of interest corresponding to AtMSH4 promoter (∼2 kb) and the vector (∼3 kb) were purified by agarose gel electrophoresis and recovered from the agarose as described above. The fragments were ligated together, transformed into *E*. *coli* and putative clones of the gene identified as described above. The DNA sequence of the resultant clone, pTK65, was determined to confirm it encoded ∼2 kb of the promoter region from the Arabidopsis AtMSH4 gene. To enable analysis and application of the AtMSH4 promoter in plants, the cloned promoter fragment was transferred to plant transformation vectors. The DNA fragment encoding the AtMSH4 promoter from pTK65 was isolated by digestion of the plasmid with KpnI, followed by treatment with T4 polymerase to make the DNA ends blunt, and digested with BamHI. This fragment was then ligated to the plant transformation vector pCB308 [296] digested with XbaI, treated with Klenow polymerase to make the DNA ends blunt, and then digested with BamHI. The insert and vector fragments were purified and ligated together, as outlined above, resulting in the clone pTK93.

A DNA sequence encoding a region of the promoter from a SPO11 gene of *Arabidopsis thaliana* was cloned. Template for amplifying the AtSPO11 promoter by PCR was genomic DNA from *Arabidopsis thaliana* ecotype Columbia isolated following standard procedure [256]. A PCR reaction was performed with approximately 1 µg of genomic DNA as template, 1.0 pmol SPO-1-PROM-5 'KpnSac oligonucleotide and 1.0 pmol SPO-1-PROM-3'Xho oligonucleotide, 0.2 mM dNTP's, 2.5 U Pfu (Stratagene) and Pfu buffer constituents provided by the manufacturer in a volume of 50 µl. The PCR conditions were 5 min @ 94 C, followed by 35 cycles of 30 s @ 94 C, 30 s @ 60 C and 4 min @ 72 C, followed by 10 min @ 72 C and storage at 4 C or -20 C. The amplified DNA was digested with KpnI and XhoI and the ∼1.2 kb fragment purified. pNML24 was also digested with KpnI and XhoI and the vector portion purified. The vector and amplified fragment were ligated together and the resultant clone of the AtSPO11 promoter region was denoted pJD1. This fragment can then be cloned into a plant transformation vector like pCB302 [296] for analysis and applications in plants.

In some embodiments of the methods of the invention, the invention enables production of gene targeting substrates in essentially all tissues throughout essentially all developmental stages, during essentially all stages of the cell cycle and in mitotic and meiotic cells through use of a constitutive promoter. Alternatively, constitutive promoters with differential expression amongst tissues, developmental stages, cell cycle stage, or mitotic or meiotic cells may also be used. In some embodiments gene expression patterns as desired is facilitated by linking the expression of the Rep factor(s) to a constitutive promoter. Examples of constitutive promoters applicable to the invention and applied in different embodiments of the invention are cryptic promoters [302], viral promoters [303], prokaryote-derived promoters [304] or promoters transcribing various cellular constituents [305-307].

### G. Plant Target Gene Assemblies and applications in plants

In some embodiments modification of chromosomal target loci in plant genomes is achieved with the invention. To exemplify application of the invention in plants, modification of a native chromosomal copy of the alcohol dehydrogenase gene in A. *thaliana* was employed. In other embodiments, any gene or genomic sequence in plant or animal genomes may be manipulated using the invention. In one embodiment, the *A. thaliana* alcohol dehydrogenase (i.e. AtADH) gene is altered by insertion of a sequence within the coding region of the gene. This insertion may cause inactivation of the gene by, for example, inhibiting formation of functional mRNA transcripts from the modified allele. Alternatively, translation of the mRNA transcripts from the modified allele may result in a truncated or non-functional protein which is no longer able to perform the normal reaction of the protein encoded by the target locus (e.g. alcohol dehydrogenase). Inactive or null alleles of the AtADH gene (i.e. Atadh) enable the plant to grow in the presence of allyl alcohol [308] (i.e. the plants may be considered resistant to allyl alcohol). This is because a functional alcohol dehydrogenase enzyme normally oxidizes allyl alcohol to a toxic aldehyde, acrolein [308]. Thus Arabidopsis plants with a functional allele of AtADH will die when cultured in the presence of allyl alcohol (i.e. the plants are susceptible to allyl alcohol). This phenotype of allyl alcohol susceptibility and resistance can thus be used as a marker to score gene targeting events where the AtADH gene is inactivated. In summary, the assay involves generating gene targeting substrate designed to inactivate a chromosomal copy of the wild type AtADH gene in Arabidopsis. Since this plant line is initially wild type for AtADH, progeny from the line can be assayed for the frequency of allyl alcohol resistant plants (i.e. Atadh) to gauge the occurrence of gene targeting events.

To engineer the gene targeting substrate for this exampleassay, the AtADH allele must be cloned and modified to create the null allele. In one embodiment the AtADH allele was cloned and modified using the recombinogenic cloning method [281]. In alternative embodiments, conventional approaches using combinations of restriction enzymes are used to clone desired DNA fragments in required combinations and assemblies. BAC's (bacterial artificial chromosomes) #F1B15, #F8B23, and #F26N21 encoding AtADH from the Columbia ecotype of *A. thaliana,* were obtained from the Arabidopsis Biological Resource Centre (Ohio State University, 1060 Carmack Road, Columbus, OH, 432101002). The presence of AtADH gene in these BAC's was confirmed by PCR using the oligonucleotides ADH-Test-S(-400) and ADH-Test-AS(+400) and scoring for the amplification of a ∼0.8 kb DNA fragment. The BAC's #F1B15, #F8B23, and #F26N21 were then isolated and transformed into E. coli DY380 [281].

DY380 is a specialised E. coli strain that enables tight regulation of an efficient homologous recombination system within the strain. The tight regulation of homologous recombination helps ensure stability of complex DNA sequences such as those encoded by BAC's. The high efficiency of homologous recombination in this E. coli strain enables efficient gene targeting and manipulation of BAC or other DNA sequences in E. coli [281]. In brief, a cassette encoding an antibiotic resistance gene is amplified by PCR using oligonucleotide primers which incorporate, for example, ∼50 bp of flanking homology to a target gene carried, for example, by a BAC. This cassette is then transformed into DY380 whose homologous recombination functions are induced. The cassette is thus integrated into the BAC at the position specified by the ∼50 bp of flanking homology and these events are selected for using the antibiotic resistance encoded by the cassette. The desired gene interrupted by this cassette, plus surrounding sequences of desired extent, can then be subcloned using a similar approach. The desired vector is amplified by PCR using oligonucleotide primers which incorporate, for example, ∼50 bp of flanking homology corresponding to sequences encoded by a BAC which are desired to be subcloned. This amplified vector is then transformed into E. coli DY380 carrying the BAC with the desired gene disrupted by the antibiotic resistance cassette and whose homologous recombination functions are induced. Homologous recombination events transferring the disrupted gene, plus desired extents of flanking sequence, into the cloning vector are selected for using the antibiotic resistance markers on the gene disruption cassette and the cloning vector. The cassette disrupting the cloned gene can, if desired, then be excised by transforming the construct into E. coli EL250 strain which encodes the FLP recombinase [281]. This can leave a 'scar' sequence [282] which inhibits functional translation of the target gene. The modified target gene which is disrupted by the antibiotic cassette or the 'scar' sequence is then transferred to the gene targeting system described in the invention for application in plants or animals.

To modify the sequence of the AtADH gene to create a null allele using the recombinogenic cloning approach [281], the chloramphenicol resistance (i.e. Cm^{R}) cassette of pKD3 [282] is first amplified by PCR using oligonucleotides P1-ADH-1 and P2-ADH-1. These oligonucleotides incorporate into the Cm^{R} cassette ∼50 bp of flanking homology corresponding to 26 bp upstream and 22 bp downstream of the AtADH ATG start codon for P1-ADH-1 and from 46 bp to 95 bp downstream of the ATG start codon for P2-ADH-1. The resultant ∼1.1 kb DNA fragment is then used to transform E. coli DY380 possessing BAC F1B15. The DY380 recombination functions facilitate a homologous recombination event between the ends of the amplified Cm^{R} cassette and the sequences surrounding the ATG start codon of AtADH gene encoded by BAC F1B15. Clones with stable integration of the Cm^{R} cassette are identified by selection on TYS medium containing kanamycin (50 µg/ml), the selectable marker on the BAC, and chloramphenicol (20 µg/ml). The presence of the Cm^{R} cassette in the correct position of the BAC can then be assayed by a PCR reaction using the oligonucleotide primers C1 combined with ADH-Test-S(-400) and C2 combined with ADH-Test-AS(+400). The C1 and C2 primers anneal to sequences within the Cm^{R} cassette and the ADH-Test-S(-400) and ADH-Test-AS(+400) primers anneal to ∼400 bp upstream and downstream of the AtADH ATG start codon. Thus amplification of a ∼550 bp fragment with the C1 and ADH-Test-S(-400) combination of primers, and amplification of a ∼500 bp fragment with the C2 and ADH-Test-AS(+400) combination of primers is diagnostic for the Cm^{R} cassette to be integrated in the desired location of the AtADH gene. The resultant AtADH allele was denoted Atadh::Cm^{R}. The Atadh::Cm^{R} allele can be further evaluated and its arrangement confirmed by digesting the modified BAC containing the insertion at the AtADH gene with a series of restriction enzymes and then performing a Southern blot as per standard procedures [256].

### G1. Application of TYLCV-derived components to gene targeting in plants

To link the Atadh::Cm^{R} allele with the TYLCV initiator and terminator sequences, pNML5 is first amplified by PCR using oligonucleotides ADH-5'-2kb-TY-X-INIT and ADH-3'-2kb-TY-X-TERM. These oligonucleotides incorporate onto the ends of the amplified vector ∼50 bp of flanking homology corresponding to ∼2 kb upstream and ~3.7 kb downstream of the AtADH ATG start codon. The resultant ~6.4 kb fragment is then used to transform E. coli DY380 possessing BAC F1B15 encoding Atadh::Cm^{R}. The DY380 recombination functions facilitate a homologous recombination event between the ends of the amplified pNML5 and the sequences ~2 kb upstream and ~3.7 kb downstream of the Cm^{R} cassette integrated into the AtADH gene encoded by BAC F1B15. Clones where the homologous recombination event has occurred can be selected for using TYS medium containing chloramphenicol and ampicillin to select for combination of the Atadh::Cm^{R} allele and pNML5, respectively. The presence of Atadh::Cm^{R} allele and adjoining sequences linked to the TYLCV initiator and terminator sequences in pNML5 can be assayed for by a PCR reaction using the oligonucleotide primers C1 combined with Universal Primer (UP; Gibco BRL) and C2 combined with Reverse Primer (RP; Gibco BRL). The C1 and C2 primers anneal to sequences within the Cm^{R} cassette and the UP and RP primers anneal to sequences adjoining the multiple cloning site of pNML5. Thus amplification of a ~2 kb fragment with the C 1 and UP combination of primers, and amplification of a ~4 kb fragment with the C2 and UP combination of primers is diagnostic for the Atadh::Cm^{R} allele and adjoining sequences to be linked to the TYLCV initiator and terminator sequences in pNML5. The resultant clone is denoted pTY-Init-Term::Atadh::Cm^{R}. In some embodiments the Cm^{R} cassette is excised from Atadh by the action of FLP recombinase via introducing the construct into E. coli EL250 as described [281]. The loss of the cassette is assayed for by using a standard PCR reaction, as described above, with the oligonucleotide primers ADH-Test-S(-400) and ADH-Test-AS(+400). Amplification of a ~800 bp fragment is diagnostic for the loss of the Cm^{R} cassette. The 'scar' sequence that is left encodes translation stop codons that will impair translation of a functional ADH protein. The resultant clone is denoted pTY-Init-Term::Atadh::Scar.

A plant transformation construct is assembled to enable expression of the TYLCV RepC1 gene in a plant line encoding the TYLCV initiator and terminator sequences linked to the Atadh::Cm^{R} allele. In some embodiments the expression of TYLCV RepC1 is regulated by the AtH4 histone promoter cloned in pNML11. In some embodiments the expression of TYLCV RepC 1 is regulated by the AtCycD3 promoter cloned in pTK159. In some embodiments the expression of TYLCV RepC1 is regulated by the EntCUP2 promoter [302] cloned in p79-632 (AAFC Saskatoon). In some embodiments expression of TYLCV RepC1 is regulated by the AtDMC1 promoter cloned in pTK111. In some embodiments the expression of TYLCV RepC 1 is regulated by the AtSP011 promoter cloned in pJD1. In some embodiments the expression of TYLCV RepC1 is regulated by the AtMSH4 promoter cloned in pTK65. In some embodiments the expression of TYLCV RepC1 is regulated by the AtRAD51 promoter cloned in pTK1 14.

The RepC1 gene is first cloned behind these various promoters. For example, to link RepC1 gene to the AtH4 promoter pNML2 is first digested with NotI, treated with Klenow polymerase to make the ends blunt, and then digested with BamHI. pNML11 is digested with XbaI, treated with Klenow polymerase to make the ends blunt, and then digested with BamHI. DNA fragments of interest corresponding to RepC1 (~1.1 kb) and the pNML1 (~4.2 kb) are purified by agarose gel electrophoresis, recovered from the agarose, ligated together and transformed into E. coli, as described above. The resultant clone of RepC1 linked to the AtH4 promoter is denoted pH4::RepC1. In a similar fashion the RepC1 gene is linked to the cloned ~1.1 kb DNA fragment encoding AtCycD3 promoter, resulting in the clone pCycD3::RepC1. To link RepC1 to a constitutive promoter such as EntCUP2, p79-632 (AAFC Saskatoon) is digested with AatII and FseI, then treated with T4 polymerase to make the ends blunt. pH4::RepC1 is digested with SacI and XhoI, to remove the AtH4 promoter, and treated with T4 polymerase to make the ends blunt. DNA fragments of interest corresponding to EntCUP2 (~0.5 kb) and the vector (~4.4 kb) are purified by agarose gel electrophoresis, recovered from the agarose, ligated together and transformed into E. coli, as described above. The resultant clone of RepC1 linked to the EntCUP2 promoter is denoted pCUP::RepC1.

To link the promoter::RepC1 assemblies to TYLCV initiator and terminator sequences, the promoter::RepC1 assemblies are first isolated by digesting the respective plasmids with KpnI and PstI. pNML5 is digested with KpnI and Xbal to release a fragment encoding the TYLCV initiator and terminator sequences. pLITMUS28 (New England BioLabs) is digested with XbaI and NsiI which produces a cohesive end compatible with the cohesive end produced by PstI digestion of the promoter::RepC1 fragment. DNA fragments of interest corresponding to promoter::RepC1 assemblies (i.e.~2.3 kb for AtH4::RepC1, ~2.5 kb for AtCycD3::RepC1, ~1.9 kb for EntCUP2::RepC1), the TYLCV initiator and terminator sequences (~0.6 kb) and the vector (~2.8 kb) are purified by agarose gel electrophoresis, recovered from the agarose, ligated together and transformed into E. coli, as described above. The resultant clone of AtH4::RepC1 linked to the TYLCV initiator and terminator sequences is denoted pH4::RepC1::Init-Term. The resultant clone of AtCycD3::RepC1 linked to the TYLCV initiator and terminator sequences is denoted pCycD3::RepC1 ::Init-Term. The resultant clone of EntCUP2::RepC1 linked to the TYLCV initiator and terminator sequences is denoted pCUP::RepC1 ::Init-Term.

To transfer the promoter::RepC1 plus TYLCV initiator and terminator sequence assemblies to a plant transformation vector, pH4::RepC1::Init-Term, pCycD3::RepC1::Init-Term, and pCUP::RepC1::Init-Term are each digested with AvrII and Spel and the respective fragments encoding the assemblies are isolated (i.e. ~2.9 kb, ~3.1 kb, and ~2.5 kb, respectively). The plant transformation vector pCB302 [296] is digested with SpeI and AvrII which produces a cohesive end compatible with the cohesive end produced by XbaI. The resultant assemblies produced by ligation of these fragments are denoted pCB-H4::RepCl::Init-Term, pCB-CycD3::RepCl::Init-Term, and pCB-CUP::RepC1::Init-Term.

To transfer the Atadh::Cm^{R} allele into the plant transformation vector encoding the promoter::RepC1 plus TYLCV initiator and terminator sequence assemblies, pTY-Init-Term::Atadh::Cm^{R} is digested with AscI and PmeI and the resultant ~7.3 kb DNA fragment encoding the TYLCV initiator sequence plus the Atadh::Cm^{R} allele is purified. The plasmids pCB-H4::RepC1::Init-Term, pCB-CycD3::RepC1::Init-Term, and pCB-CUP::RepC1 ::Init-Term are digested with AscI and SmaI and the DNA fragment encoding the vector and functional components purified. These fragments are ligated together in independent reactions and transformed into E. coli. The desired recombinants are selected for by plating the cells on TYS medium containing chloramphenicol and kanamycin to select for the Atadh::Cm^{R} allele and the pCB302 vector backbone, respectively. The resultant assemblies produced by ligation of these fragments are denoted pCB-H4::RepC1::Init-Term-Atadh::Cm^{R}, pCB-CycD3::RepC1::Init-Term-Atadh::Cm^{R}, and pCB-CUP::RepC1::Init-Term-Atadh::Cm^{R}. In some embodiments the Cm^{R}cassette may be excised from Atadh by the action of FLP recombinase via introducing the construct into E. coli EL250 as described [281]. The loss of the cassette is assayed for by using a standard PCR reaction, as described above, with the oligonucleotide primers ADH-Test-S(-400) and ADH-Test-AS(+400). Amplification of a ~800 bp fragment is diagnostic for the loss of the Cm^{R} cassette. The 'scar' sequence that is left encodes translation stop codons that will impair translation of a functional ADH protein. The resultant clones are denoted pCB-H4::RepC1::Init-Term-Atadh-Scar, pCB-CycD3::RepC1::Init-Term-Atadh-Scar, andpCB-CUP::RepC1::Init-Term-Atadh-Scar.

In some embodiments expression of TYLCV RepC1 is regulated by the AtDMC1 promoter such as cloned in pTK.111. In some embodiments the expression of TYLCV RepC1 is regulated by the AtSPO11 promoter such as cloned in pJD1. In some embodiments the expression of TYLCV RepC1 is regulated by the AtMSH4 promoter such as cloned in pTK65. In some embodiments the expression of TYLCV RepC1 is regulated by the AtRAD51 promoter such as cloned in pTK114.

### Test gene targeting in plants using TYLCV-derived components

The plant transformation constructs encoding the gene targeting system employing the TYLCV- derived components are used to transform *A. thaliana* as a representative plant species where the invention may be applied. The constructs are first introduced into *Agrobacterium tumefaciens* C58C1(pMP90) [309] following standard microbiological procedures [256]. Arabidopsis plants are then transformed with the gene targeting constructs using the 'floral-dip' method [310]. Seed is collected from these plants treated with *A. tumefaciens.* T₀ plants are selected by sowing the seed on soil and, after 7-14 days of development, spraying the plants with a glufosinate ammonium herbicide (0.75-1mg/ml; Aventis; PCP#14817); herbicide resistance is indicative of the gene targeting construct being integrated into the plant chromosome since the construct encodes the Bar gene of pCB302 [296]. The To plants are allowed to self-cross and T₁ seed is collected from individual lines. Samples of T₁ seed from each herbicide resistant line is then plated on medium containing allyl alcohol as described [308]. Plants that are homozygous for an inactive Atadh allele will be able to grow in the presence of allyl alcohol and will reflect the incidence of gene targeting occurring.

To summarise the assay of gene targeting concerning modification of the AtADH gene as an example, the plants are transformed with the gene targeting constructs encoding RepC1 and the Atadh::Cm^{R} or the Atadh-Scar allele associated with the TYLCV initiator and terminator sequences. As a control, other plants may be transformed with the gene targeting constructs encoding the TYLCV initiator and terminator sequences without an intervening sequence (i.e. no Atadh allele). In the case of where promoters which are functional in vegetative cells are used to control expression of RepC 1, gene targeting events may occur as the seeds from the *A*. *tumefaciens* treated plants germinate and develop into the To plants. With each cell division, the targeting substrate may be produced by the action of RepC 1 on the TYLCV initiator and terminator sequences in conjunction with host DNA replication machinery. Thus numerous opportunities occur during plant development for the chromosomal allele of AtADH to be converted to a new sequence (i.e. Atadh) by the targeting substrate. With the possibility of gene conversion to occur very early in development (i.e. from time of germination), there is a high probability that the converted allele may be held by a cell lineage which leads to gamete formation. If the converted allele is carried into the germ line in a heterozygous state, meiosis in the particular flower or flowers derived from the converted cell lineage may be expected to produce gametes at a 1:1 ratio regarding the wild-type (AtADH) and converted (Atadh) allele. In the case of the alcohol dehydrogenase locus, selfed progeny from such a flower may segregate in a Mendelian fashion as 1:2:1 with 25% of the progeny being homozygous for the converted allele and selected for by allyl alcohol. Efficiency of gene targeting may be gauged by the frequency of To plants producing progeny resistant to allyl alcohol. In other embodiments, further generations (i.e. T₁, T₂, Tₙ) may be evaluated for occurrence of gene targeting events. This frequency may also be compared to that obtained in control plants transformed with the same gene targeting construct except not having an intervening sequence (i.e. no Atadh allele) associated with the TYLCV initiator and terminator sequences. Because the gene targeting construct encoding RepC1 and TYLCV initiator and terminator sequences linked to the Atadh reproducible sequence may integrate into a site in the plant genome distal from the target allele (e.g. AtADH), then through the process of natural genetic segregation plants may be identified which encode the modified target locus (e.g. Atadh) but no longer encode the initial gene targeting construct. As a result this plant may contain no undesired foreign sequences (e.g. transformation construct sequences). In addition, this plant line may be transformed with a new gene targeting construct to modify a second target locus and the identification of these primary transformants may use the same selectable marker as used in the initial gene targeting construct.

In other embodiments where the promoters which are functional in meiotic cells are used to control expression of RepC1, gene targeting events may occur as the To plant undergoes meiosis. In this case, the AtADH gene in numerous male and female gametes may be converted to Atadh allele. If this plant is allowed to self-cross, seeds will result that are either heterozygous for the converted allele (i.e. AtADH/Atadh) or homozygous for the converted allele (i.e. Atadh/Atadh), as well as homozygous wild type. Efficiency of gene targeting may be gauged by frequency of To plants producing progeny resistant to allyl alcohol. In other embodiments, further generations (i.e. Ti, T₂, Tₙ) may evaluated for occurrence of gene targeting events. This frequency may also be compared to that obtained in control plants transformed with the same gene targeting construct except not having an intervening reproducible sequence (i.e. no Atadh allele) associated with the TYLCV initiator and terminator sequences.

In other embodiments of the methods of the invention alternative genes encoded in plant or animal genomes may be modified using the gene targeting system described here. One example of commercial importance in plants would be herbicide resistance such as, for example, that associated with the acetolactate synthase (i.e. ALS) gene. Modification of, for example, amino acid residue #653 of the ALS protein from Arabidopsis thaliana corresponding to a serine, or the corresponding amino acid from ALS proteins from other species, whereby it is converted to an asparagine can confer resistance to a imidazolinone-type herbicide [311]. An engineered allele of the ALS gene to create a gene targeting substrate, which can facilitate such an amino acid change to confer herbicide resistance, can be used with this system.

In some embodiments an altered form of RepC1 is employed which no longer affects the normal function of protein regulators of the cell cycle, such as 'pocket family' proteins like retinoblastoma-related protein (RBR), or GRAB proteins [312]. RBR, for example, is known to be an important regulator of the cell cycle in eukaryotic cells by controlling the expression of genes required for the G1-S transition and S-phase progression [312]. The RepC1-like protein from different plant viruses can interact with RBR and alter the function of RBR thereby changing the regulation of the cell cycle and promote entry into S-phase [312]. In some applications of the invention this may be undesirable. Therefore an altered form of RepC1 which maintains its normal enzymatic activity but no longer affects the function of RBR can be used. The action of RepC 1 on RBR may be due to physical interactions between the two proteins alone or in conjunction with other host or viral encoded proteins. In some types of RepC1-like proteins this interaction is due to an LxCxE motif and point mutations in this motif greatly reduce or abolish the interaction [312]. Therefore such mutated proteins may be employed in the invention. Such mutants may be generated by site-directed mutagenesis following standard techniques [256]. In other instances the amino acid residues responsible for the interaction between RepC1-like proteins and pocket proteins or GRAB proteins are undefined [312]. Therefore, as an example of a method to isolate mutant forms of RepC1-like proteins which no longer interact with proteins regulating the host cell cycle, a yeast two-hybrid reverse-interaction screen [313] can be performed. Many plant homologues of, for example, RBR have been identified [312]. and RBR homologues from other species may be identified using standard homology-based cloning procedures [256]. The cloned RBR gene may, for example, be placed in the 'Bait' vector. A library of mutagenised version of the RepC1 gene, for example from TYLCV, is cloned in the 'Prey' vector. Versions of RepC1 which no longer interact with Rb can be identified by, for example, selection for growth on specific media [313]. Physical interactions between RepC1 and Rb can further be evaluated by immunoprecipitation experiments [256]. The RepC1 alleles identified through this screen can then be evaluated to confirm the proteins still maintain nickase activity. An allele of RepC1 that maintains nickase activity but no longer affects regulation of host cell cycle *in vivo* can then be applied to the gene targeting system disclosed here.

### G2) Application of φfd-derived components to gene targeting in plants

To link the Atadh::Cm^{R} allele with the φfd initiator and terminator sequences, pTY-Init-Term::Atadh::Cm^{R} is digested with AscI and MscI. pRH21 is digested with SacI, treated with Klenow polymerase to make the DNA ends blunt, and then digested with AscI. The resulting ~6.7 kb DNA fragment from pTY-Init-Term::Atadh::Cm^{R} and ~5.1 kb DNA fragment from pRH21 are purified by agarose gel electrophoresis and recovered from the agarose as described above. The fragments are ligated together, transformed into *E*. *coli* and putative clones of the assembly identified as described above. The resultant clone of the Atadh::Cm^{R} allele linked with the φfd initiator and terminator sequences is denoted pfd-Init-Term::Atadh::Cm^{R}. In some embodiments the Cm^{R} cassette is excised from Atadh by the action of FLP recombinase via introducing the construct into E. coli EL250 as described [281]. The loss of the cassette is assayed for by using a standard PCR reaction, as described above, with the oligonucleotide primers ADH-Test-S(-400) and ADH-Test-AS(+400). Amplification of a ~800 bp fragment is diagnostic for the loss of the Cm^{R} cassette. The 'scar' sequence that is left encodes translation stop codons that will impair translation of a functional ADH protein. The resultant clone is denoted pfd-Init-Term::Atadh::Scar.

In some embodiments components from prokaryotic DNA replication systems, such as bacteriophage φfd, are used to facilitate gene targeting. In some embodiments the bacteriophage φfd initiator and terminator sequences are linked to an intervening sequence (i.e. the reproducible sequence) and assembled in a plant transformation construct which also facilitates expression of g2p, or derivative thereof, in a manner as described above for the TYLCV-derived components. In some embodiments the bacteriophage initiator and terminator sequences may be associated with a promoter that transcribes through the initiator. To link a promoter functional in plants to the φfd initiator and terminator sequences pRH21 is digested with HindIII and the resultant DNA ends made blunt by treatment with T4 polymerase. p79-632 (AAFC Saskatoon) is digested with AatII and FseI, then treated with T4 polymerase to make the ends blunt. A DNA fragment corresponding to EntCUP2 (~0.5 kb) from p79-632 is purified by agarose gel electrophoresis, recovered from the agarose, ligated together to the modified pRH21 and transformed into E. coli, as described above. The resultant clone of φfd initiator and terminator sequences linked to the EntCUP2 promoter is denoted pCUP::fd-Init-Term.

The g2p-NLS gene is then cloned behind various promoters. For example, to link g2p-NLS gene to the AtH4 promoter pAS4 is first digested with EcoRV and PstI, then treated with Klenow polymerase to make the ends blunt. pNML11 its digested with SnaBI and XbaI, then treated with Klenow polymerase to make the ends blunt. DNA fragments of interest corresponding to g2p-NLS (~1.2 kb) and the pNML11 (~4.2 kb) are purified by agarose gel electrophoresis, recovered from the agarose, ligated together and transformed into E. coli, as described above. The resultant clone of g2p-NLS linked to the AtH4 promoter is denoted pH4::g2p-NLS. In a similar fashion the g2p-NLS gene is linked to the cloned ~1.1 kb DNA fragment encoding AtCycD33 promoter, resulting in the clone pCycD3::g2p-NLS. To link g2p-NLS to a constitutive promoter such as EntCUP2, p79-632 (AAFC Saskatoon) is digested with AatII and FseI, then treated with T4 polymerase to make the ends blunt. pH4::g2p-NLS is digested with SacI and XhoI, to remove the AtH4 promoter, and treated with T4 polymerase to make the ends blunt. DNA fragments of interest corresponding to EntCUP2 (~0.5 kb) and the vector (~4.4 kb) are purified by agarose gel electrophoresis, recovered from the agarose, ligated together and transformed into E. coli, as described above. The resultant clone of RepC1 linked to the EntCUP2 promoter is denoted pCUP::g2p-NLS.

To link these promoter::g2p-NLS assemblies to φfd initiator and terminator sequences, the promoter::g2p-NLS assemblies are first isolated by digesting the respective plasmids with SacI, treating with T4 polymerase to make the DNA ends blunt, then digesting with PstI. pCUP::fd-Init-Term is digested with SnaBI and SpeI to release a fragment encoding the φfd initiator and terminator sequences. pLITMUS28 (New England BioLabs) is digested with XbaI, producing a cohesive end compatible with SpeI, and NsiI, producing a cohesive end compatible with the cohesive end produced by PstI digestion. DNA fragments of interest corresponding to promoter::g2p-NLS assemblies (i.e. ~2.4 kb for AtH4::g2p-NLS, ~2.6 kb for AtCycD3::g2p-NLS, ~2 kb for EntCUP2::g2p-NLS), the φfd initiator and terminator sequences (~1.3 kb) and the vector (~2.8 kb) are purified by agarose gel electrophoresis, recovered from the agarose, ligated together and transformed into E. coli, as described above. The resultant clone of AtH4::g2p-NLS linked to the φfd initiator and terminator sequences is denoted pH4::g2p-NLS::Init-Term. The resultant clone of AtCycD3::g2p-NLS linked to the φfd initiator and terminator sequences is denoted pCycD3::g2p-NLS::Init-Term. The resultant clone of EntCUP2::g2p-NLS linked to the φfd initiator and terminator sequences is denoted pCUP::g2p-NLS::Init-Term.

To transfer the promoter::g2p-NLS plus φfd initiator and terminator sequence assemblies to a plant transformation vector, pH4::g2p-NLS::Init-Term, pCycD3::g2p-NLS::Init-Term, and pCUP::g2p-NLS::Init-Term are each digested with AvrII and SpeI and the respective fragments encoding the assemblies are isolated (i.e. ~3.7 kb, ~3.9 kb, and ~3.3 kb, respectively). The plant transformation vector pCB302 [296] is digested with SpeI and AvrII which produces a cohesive end compatible with the cohesive end produced by XbaI. The resultant assemblies produced by ligation of these fragments are denoted pCB-H4::g2p-NLS::Init-Term, pCB-CycD3::g2p-NLS::Init-Term, and pCB-CUP::g2p-NLS::Init-Term.

To transfer the Atadh::Cm^{R} allele into the plant transformation vector encoding the promoter::g2p-NLS plus φfd initiator and terminator sequence assemblies, first pTY-Init-Term::Atadh::Cm^{R} is digested with AscI and MscI releasing a ~6.7 kb DNA fragment encoding the Atadh::Cm^{R} allele which is purified. pRH21 encoding the φfd initiator and terminator sequences is digested with SacI, treated with T4 polymerase to make the DNA ends blunt, and then digested with AscI. The resulting ~6.7 kb DNA fragment from pTY-Init-Term::Atadh::Cm^{R} and ~5.1 kb DNA fragment from pRH21 are purified by agarose gel electrophoresis and recovered from the agarose as described above. The fragments are ligated together, transformed into *E*. *coli* and putative clones of the assembly identified as described above. The resultant clone of the Atadh::Cm^{R} allele linked with the φfd initiator and terminator sequences is denoted pfd-Init-Term::Atadh::Cm^{R}. In some embodiments the Cm^{R} cassette is excised from Atadh by the action of FLP recombinase via introducing the construct into E. coli EL250 as described [281]. The loss of the cassette may be assayed for by using a standard PCR reaction, as described above, with the oligonucleotide primers ADH-Test-S(-400) and ADH-Test-AS(+400). Amplification of a ~800 bp fragment is diagnostic for the loss of the Cm^{R} cassette. The 'scar' sequence that is left encodes translation stop codons that will impair translation of a functional ADH protein. The resultant clone is denoted pfd-Init-Term::Atadh::Scar.

To transfer the Atadh::Cm^{R} allele into the plant transformation vector encoding the promoter::g2p-NLS plus φfd initiator and terminator sequence assemblies, pfd-Init-Term::Atadh::Cm^{R} is digested with PmeI and AscI and the resultant ~7.1 kb DNA fragment purified. The plasmids pCB-H4::g2p-NLS::Init-Term, pCB-CycD3::g2p-NLS::Init-Term, and pCB-CUP::g2p-NLS::Init-Term are also digested with AscI and PmeI and the DNA fragment encoding the vector and functional components are purified. These fragments are ligated together in independent reactions and transformed into E. coli. The desired recombinants are selected for by plating the cells on TYS medium containing chloramphenicol and kanamycin to select for the Atadh::Cm^{R} allele and the pCB302 vector backbone, respectively. The resultant assemblies produced by ligation of these fragments are denoted pCB-H4::g2p-NLS::Init-Term-Atadh::Cm^{R}, pCB-CycD3::g2p-NLS::Init-Term-Atadh::Cm^{R}, and pCB-CUP::g2p-NLS::Init-Term-Atadh::Cm^{R}. In some embodiments the Cm^{R} cassette may be excised from Atadh by the action of FLP recombinase via introducing the construct into E. coli EL250 as described [281]. The loss of the cassette may be assayed for by using a standard PCR reaction, as described above, with the oligonucleotide primers ADH-Test-S(-400) and ADH-Test-AS(+400). Amplification of a ~800 bp fragment is diagnostic for the loss of the Cm^{R} cassette. The 'scar' sequence that is left encodes translation stop codons that will impair translation of a functional ADH protein. The resultant clones are denoted pCB-H4::g2p-NLS::Init-Term-Atadh-Scar, pCB-CycD3::g2p-NLS::Init-Term-Atadh-Scar, and pCB-CUP::g2p-NLS::Init-Term-Atadh-Scar.

In some embodiments expression of g2p-NLS is regulated by the AtDMC1 promoter such as cloned in pTK111. In some embodiments the expression of g2p-NLS is regulated by the AtSPO11 promoter such as cloned in pJD1. In some embodiments the expression of g2p-NLS is regulated by the AtMSH4 promoter such as cloned in pTK65. In some embodiments the expression of g2p-NLS is regulated by the AtRAD51 promoter such as cloned in pTK1 14.

The plant transformation constructs encoding the gene targeting system employing the φfd- derived components are used to transform *A. thaliana* as a representative plant species where the invention may be applied, as described above for the gene targeting system employing the TYLCV- derived components. The constructs are first introduced into *A. tumefaciens* and transformed into the Arabidopsis genome. Seed is collected from these plants treated with *A. tumefaciens.* To plants are selected by sowing the seed on soil and, after 7-14 days of development, spraying the plants with a glufosinate ammonium herbicide (0.75-1mg/ml; Aventis; PCP#14817); herbicide resistance is indicative of the gene targeting construct being integrated into the plant chromosome since the construct encodes the Bar gene of pCB302 [296]. The To plants are allowed to self-cross and T₁ seed is collected from individual lines. Samples of T₁ seed from each herbicide resistant line is then plated on medium containing allyl alcohol as described [308]. Plants that are homozygous for an inactive Atadh allele will be able to grow in the presence of allyl alcohol and will reflect the incidence of gene targeting occurring.

To summarise the assay of gene targeting concerning modification of the AtADH gene as an example, the plants are transformed with the gene targeting constructs encoding, for example, g2p-NLS and the Atadh::Cm^{R} or the Atadh-Scar allele associated with the φfd initiator and terminator sequences. As a control, other plants may be transformed with the gene targeting constructs encoding the φfd initiator and terminator sequences without an intervening sequence (i.e. no Atadh allele). In the case of promoters which are functional in vegetative cells are used to control expression of g2p-NLS, gene targeting events may occur as the seeds from the *A*. *tumefaciens* treated plants germinate and develops into the To plants. With each cell division, the targeting substrate may be produced by the action of g2p-NLS on the φfd initiator and terminator sequences in conjunction with host DNA replication machinery. Thus numerous opportunities occur during plant development for the chromosomal allele of AtADH to be converted to a new sequence (i.e. Atadh) by the targeting substrate. With the possibility of gene conversion to occur very early in development (i.e. from time of germination), there is a high probability that the converted allele may be held by a cell lineage which leads to gamete formation. If the converted allele is carried into the germ line in a heterozygous state, meiosis in the particular flower or flowers derived from the converted cell lineage may be expected to produce gametes at a 1:1 ratio regarding the wild-type (AtADH) and converted (Atadh) allele. In the case of the alcohol dehydrogenase locus, selfed progeny from such a flower may segregate in a Mendelian fashion as 1:2:1 with 25% of the progeny being homozygous for the converted allele and selected for by allyl alcohol. Efficiency of gene targeting may be gauged by the frequency of To plants producing progeny resistant to allyl alcohol. In other embodiments, further generations (i.e. T₁, T₂, Tₙ) may be evaluated for occurrence of gene targeting events. This frequency may also be compared to that obtained in control plants transformed with the same gene targeting construct except not having an intervening sequence (i.e. no Atadh allele) associated with the φfd initiator and terminator sequences. Because the gene targeting construct encoding g2p-NLS and φfd initiator and terminator sequences linked to the Atadh reproducible sequence may integrate into a site in the plant genome distal from the target allele (e.g. AtADH), then through the process of natural genetic segregation plants may be identified which encode the modified target locus (e.g. Atadh) but no longer encode the initial gene targeting construct. As a result this plant may contain no undesired foreign sequences (e.g. transformation construct sequences). In addition, this plant line may be transformed with a new gene targeting construct to modify a second target locus and the identification of these primary transformants may use the same selectable marker as used in the initial gene targeting construct

In other embodiments where the promoters which are functional in meiotic cells are used to control expression of g2p-NLS, gene targeting events may occur as the To plant undergoes meiosis. In this case, the AtADH gene in numerous male and female gametes may be converted to Atadh allele. If this plant is allowed to self-cross, seeds will result that are either heterozygous for the converted allele (i.e. AtADH/Atadh) or homozygous for the converted allele (i.e. Atadh/Atadh), as well as homozygous wild type. Efficiency of gene targeting may be gauged by frequency of To plants producing progeny resistant to allyl alcohol. In other embodiments, further generations (i.e. T₁, T₂, Tₙ) may be evaluated for occurrence of gene targeting events. This frequency may also be compared to that obtained in control plants transformed with the same gene targeting construct except not having an intervening sequence (i.e. no Atadh allele) associated with the φfd initiator and terminator sequences.

In other embodiments of the method of the present invention any gene encoded in plant or animal genomes may be modified using the gene targeting system described here. One example of commercial importance in plants would be herbicide resistance such as, for example, that associated with the acetolactate synthase (i.e. ALS) gene. Modification of amino acid residue #653 of the ALS protein from Arabidopsis thaliana corresponding to a serine, or the corresponding amino acid from ALS proteins from other species, whereby it is converted to an asparagine can confer resistance to an imidazolinone-like herbicide [311]. An engineered allele of the ALS gene to create a gene targeting substrate, which can facilitate such an amino acid change to confer herbicide resistance, can be used with this system.

In some embodiments of the methods of the present invention where gene targeting systems employing the φfd-derived components are used the cells may also be engineered to express a helicase to promote the activity of the nickase in initiating DNA replication. An example of a helicase which may be used is the REP helicase from E. coli as represented by the clone pNML10. In addition, the action of REP helicase in eukaryotic cells may be enhanced by engineering it to encode a nuclear localisation sequence, as represented by the clone pNML24. Expression of the REP helicase may be coordinated with that of the nickase by using similar promoters for each gene, examples of which include S-phase linked promoters like that from CycD3 or H4 histone genes, constitutive promoters, or meiosis-linked promoters, like that from DMC1, SPO11 or MSH4 genes, or promoters linked to DNA homologous recombination, like that from RAD51. Alternatively, the helicase and nickase genes may be expressed by unique promoters which may or may not confer overlapping expression patterns. In some embodiments the helicase is encoded on the same construct as the nickase so that they are introduced into the host nucleus on one DNA molecule and may be integrated into the host genome at one locus. Alternatively, the helicase and nickase genes may be introduced into the host nucleus or host genome at different times through separate transformation procedures. For example, a plant line expressing the helicase may be used as a host for transformation experiments to introduce a gene targeting construct which also bears the nickase. Alternatively, a plant line encoding the helicase and nickase may be transformed with a construct that encodes the gene targeting cassette flanked by one or more recognition sequences for the nickase.

### H. Functionality of cloned elements

The function of nickases of prokaryotic origin which are engineered for enhanced activity in eukaryotic cells through addition of a nuclear localization sequence (NLS) was evaluated. This was done by testing the engineered nickase for its ability to initiate rolling-circle replication. This activity is detectable by observing production of novel DNA molecules in an E. coli strain expressing the nikcase and possessing the corresponding initiator and terminator sequences with an intervening reproducible sequence. The types of DNA molecules observed in such a strain is compared to that observed in strains possessing only the initiator-terminator plus intervening sequence construct, or expressing the nickase in the absence of the initiator-terminator plus intervening sequence construct.

To evaluate the function of the cloned and engineered rolling-circle replication components, E. coli DH5α (Gibco BRL) was transformed with the plasmids capable of expressing g2p (pRH27) or g2p-NLS (pAS 17). E. coli DH5α was also transformed with plasmids encoding the φfd initiator and terminator sequence plus an intervening sequence which will be referred to as 'template' plasmids. The template plasmids included pRH24, pMW113, and pMW114. pMW114 has the same intervening sequence as pMW113 but does not encode functional φfd initiator and terminator sequences. E. coli DH5α was also transformed with various combinations of the nickase-expressing plasmids and template plasmids. The strains were then cultured overnight at 37C with shaking (225 RPM) in 3 ml TYS medium containing the antibiotics ampicillin and/or chloramphenicol, as appropriate for the plasmid combinations. Inoculum (-60 µl) from the overnight cultures was transferred to 3 ml TYS medium containing the appropriate antibiotics and incubated at 37 C with shaking (225 RPM for ~3 h. Isopropylthio-β-galactosidasc (IPTG; Gibco BRL) was then added to 0.1 mM and the cultures were incubated for a further ~4 h. DNA was isolated by the alkaline lysis method [256] and the concentration of the DNA samples estimated by spectrophotometry [256]. Approximately 1 µg samples of DNA were digested with SacII, which has a single recognition sequence in pAS 17, pMW113 and pMW114, or digested with PstI, which has a single recognition sequence in pRH24 and pRH27. The DNA was then resolved by agarose gel electrophoresis and detected using ethidium bromide as per standard procedures [256].

As illustrated in **Figure 1**, the combination of a cloned nickase with the cloned initiator-terminator sequences (i.e. pAS17 combined with pMW113; pRH27 combined with pRH24) results in amplification of the intervening reproducible sequence, as indicated by the production of a novel type of DNA molecule. This amplification occurs by rolling-circle replication *in vivo.* This confirms the functionality of the cloned initiator-terminator sequences embodied here and applied to achieving gene targeting in eukaryotic cells. **Figure 1** also illustrates the functionality of a prokaryotic nickase engineered to encode a NLS, as demonstrated by the novel type of DNA molecule observable when the initiator-terminator sequences plus intervening reproducible sequence are combined with the expressed g2p-NLS (i.e. pAS17 and pMW113). The level of activity of g2p-NLS is very similar to that of the unmodified g2p, as demonstrated by the levels of amplified DNA product produced when these enzymes are combined with a template plasmid (i.e. pAS17 combined with pMW113 vs. pRH27 combined with pRH24). This also confirms the functionality of the cloned and engineered g2p-NLS gene embodied here and applied to achieving gene targeting in eukaryotic cells. The amplification of the intervening reproducible sequence linked to the initiator-terminator sequences was also found to be dependent upon the presence of functional nickase recognition sequences, as shown by the absence of a novel type of DNA molecule when the nickase is combined with pMW 114.

### I. Application of rolling-circle replication components to gene targeting in eukaryotic cells

To demonstrate application of the invention for genetic modification of a chromosomal target locus, yeast was used as a model eukaryote. The processes of DNA replication, recombination and repair are highly conserved from yeast to animals, including humans, and plants [314-318].

The genetic assay to demonstrate the invention in yeast as a representative eukaryotic cell involves modification of the chromosomal URA3 locus. This locus in Saccharomyces cerevisiae encodes the orotidine-5'-phosphate decarboxylase enzyme [319] which is required for the conversion of orotidine-5'-monophosphate to uridine 5'monophosphate [320], leading to biosynthesis of uracil. Uracil is a component of RNA molecules and, therefore, is an essential requirement of the cell. Cells that are defective for uracil biosynthesis cannot grow. Yeast strains with defective URA3 alleles (i.e. ura3) cannot grow on minimal medium unless the medium is supplemented with uracil. 5-fluoroorotic acid (FOA; Diagnostic Chemicals Ltd.) can be catabolysed by orotidine-5'-phosphate decarboxylase to form 5-fluorouracil, a toxic substance that inhibits cell growth. Thus a yeast strain with a functional URA3 allele will not be able to grow when FOA is present in the medium.. However, a yeast strain with a defective ura3 allele will be able to grow in the presence of FOA because it does not catablolyse FOA to the toxin. If these culture steps employing FOA are done on minimal medium then supplementation with uracil is required to meet the metabolic needs of the ura3 strain.

Using this selection strategy to identify if the URA3 locus in test cells is functional or defective, the assay for gene targeting may be done in two exemplary fashions. Firstly, the chromosomal allele may be non-functional and the gene targeting cassette may encode a sequence capable of converting the chromosomal allele into a functional allele. Such events could be identified by selecting for uracil prototrophs by plating cells on minimal medium lacking uracil. Secondly, the chromosomal allele may be functional and the gene targeting cassette may encode a sequence capable of converting the chromosomal allele into a non-functional allele. Such events could be identified by selecting for FOA-resistant cells on minimal medium containing FOA and uracil. In both instances the number of cells growing on the selective medium and the total number of viable cells, as determined by culturing on non-selective medium, would be determined for each treatment to estimate the frequency of modification of the target locus that occurs. The frequency of cells identified on the selective medium would also be determined for control strains. One control would be a strain expressing the Rep factor(s), in the absence of the gene targeting cassette, to determine if the Rep factor(s) had any inherent ability to promote modification of the target locus. This control would also help estimate the frequency of natural spontaneous alterations of the target locus. Another control would be a strain possessing the gene targeting cassette without the Rep factor(s) present. This could account for background levels of modification of the target locus resulting from interactions between the gene targeting cassette and the target locus. Another treatment would be a strain possessing both the gene targeting cassette and expressing the Rep factor(s). By comparing the frequency of cells occurring on the selective medium using this latter strain to the two controls described above, one can determine the effect the action of Rep factor(s) on the gene targeting cassette has on promoting modification of the target locus. This is representative of the gene targeting frequency.

The genetic assays in yeast employed the S. cerevisiae RK2575 strain [321] with a genotype as follows: *Mata ura3-52 his3 trp1-289 leu2-3,112 lys2ΔBgl hom3-10.* RK2575 has defective alleles at the URA3, HIS3, LEU2 and LYS2 loci. The strain is thus termed auxotrophic for uracil, histidine, leucine and lysine because it is unable to grow in the absence of these compounds being provided in the growth medium. The defective alleles can be complemented by functional alleles carried on plasmids which can be used to enable selective maintenance of the plasmids in the strain, as per standard procedures [256]. Conversion of such alleles to a functional form which can confer prototrophy to a cell can also be used to assay for gene targeting events.

The ura3-52 allele in RK2575 is non-functional because it is interrupted by a transposable element [322]. To use this allele to assay the gene targeting system RK2575 was transformed with various plasmids encoding the system components derived from bacteriophage φfd. Yeast transformations were done as per Geitz et al. (1995) [323]. pRH33 encodes φfd initiator-terminator sequences flanking the ura3ΔtuI-SmaI allele as a reproducible sequence. This allele is defective in that it does not encode a functional orotidine-5'-phosphate decarboxylase enzyme. However the ura3AStuI-SmaI allele has ~1.1 kb homologous to the region upstream of the transposon in ura3-52 and ~0.3 kb homologous to the region downstream of the transposon insertion. Thus a homologous recombination event between a gene targeting substrate encoded by pRH32 (i.e. ura3ΔStuI-SmaI allele) and the chromosomal ura3-52 allele could result in a functional URA3 locus. Such events would be identifiable by selecting cells on minimal medium. pRH37 expresses the NLS-g2p gene via the Tet7x promoter. Strains containing plasmids with this promoter were cultured in the presence of doxycycline (10 µg/ml for solid media; 5 µg/ml for liquid media; Sigma) to suppress promoter activity until time of assay. Strains of RK2575 possessing pRH32 or pRH37, alone or in combination, were prepared. Single colonies from each test strain were used to first inoculate 4 ml of medium in a 50 ml tube (Falcon) which was then incubated at 30 C with shaking (225 RPM) for 2 days. For the growth media [324], SC-LEU was used for the strain possessing pRH32, SC-TRP was used for the strain possessing pRH37, and SC-LEU-TRP was used for the strain possessing both pRH32 and pRH37. After incubation, aliquots of cells from each culture were collected to assay for conversion of the chromosomal *ura3-52* allele to a functional allele. Dilutions of these cells were made using sterile distilled water (SDW) and plated on YPD medium (per litre: 10g Bacto-yeast extract, 20 g Bacto-peptone, 20 g glucose, 20 g Bacto-agar; [325]) to determine viable cell number, or plated on minimal media lacking uracil (i.e. SC-URA; [324]) to determine the number of uracil prototrophs. The plates were incubated at 30 C for 2-5 days and then colonies were counted. Frequency of recombinants for each culture was determined by dividing the number of prototrophs conferred by restoration of function of the *ura3-52* test locus by the viable cell number, taking into consideration the dilution factors.

In this experiment, the frequency of uracil prototrophs in a culture of RK2575 possessing just the gene targeting cassette (i.e. pRH32) was 3.2x10⁻⁷. No prototrophs were detected in a culture of the strain expressing NLS-g2p (i.e. pRH37). However, a culture of the strain possessing both the gene targeting cassette and expressing NLS-g2p (i.e. pRH32 and pRH37) had a uracil prototroph frequency of 1.6x10-5. This represents a 50-fold increase over the control. Statistical significance of the differences between these values was confirmed by evaluation using the t-test [326]. This demonstrates that φfd components like the g2p nickase and the initiator and terminator sequences can be used to facilitate modification of specific chromosomal target loci in eukaryotes. In this case a non-functional allele on the chromosome was converted into a functional allele.

A second genetic assay was performed to evaluate the gene targeting system whereby a chromosomal locus is converted to a non-functional allele. To do this a derivative of S. cerevisiae RK2575 was first created whereby the defective ura3-52 allele was changed to a functional URA3 allele. A gene targeting cassette encoding a non-functional ura3 allele could then be introduced to this strain and the efficiency of gene targeting estimated by measuring conversion of the chromosomal allele to be non-functional.

To first create the uracil prototrophic derivative of RK2575, the URA3 containing DNA fragment of pMW41 was isolated by digestion of the plasmid with XhoI and SmaI. Approximately 1µg of the ~1.85 kb fragment encoding URA3 was used to transform RK2575 by the method of Geitz et al. (1995) [323]. The treated cells ere plated on SC-URA [324] to identify prototrophs. A uracil prototrophic isolate identified from this experiment was denoted RK2575-URA. Its genotype is identical to the RK2575 parent except for being prototrophic for uracil.

RK2575-URA was used to evaluate gene targeting systems comprising components from bacteriophage φfd and φX174, and the eukaryotic virus TYLCV. The gene targeting cassette used here encodes the ura3ΔPstI-EcoRV allele which does not encode a functional allele as ~20 bp of the promoter region and ~190 bp of the open reading frame is deleted. Transfer of this deletion mutation to the chromosomal URA3 locus will convert it to a non-functional allele. As a result, such events can be detected by screening for cells resistant to FOA and an estimation of gene targeting frequency can be determined.

To evaluate gene targeting systems comprising components of bacteriophage φfd, RK2575-URA was transformed with pAS27 (expressing g2p-NLS) or pNML18 (encoding φfd initiator-terminator linked to ura3ΔPstI-EcoRV), alone or in combination. To evaluate gene targeting systems comprising components of gemini virus TYLCV, RK2575-URA was transformed with pNML23 (expressing RepC1) or pNML17 (encoding TYLCV initiator-terminator linked to ura3ΔPstI-EcoRV), alone or in combination. The plasmids pAS27 and pNML3 use the TRP1 gene as a selectable marker in yeast whereas pNML18 and pNML17 use the LEU2 gene as a selectable marker. The respective double transformants of pAS27 plus pNML18 and pNML3 plus pNML17 thus require culture in SC-LEU-TRP [324]. Therefore, to keep media composition uniform for all treatments in the experiment, the strains transformed with the single experimental constructs (e.g. pAS27 and pNML18 into separate strains instead of in combination) were also transformed with an empty vector (e.g. YEplac181Tet2x; YEPlac112Tet7x) solely for the purpose of supplying the complementary selectable marker as present in the experimental double-transformants. In this manner all strains could be cultured in the same SC-LEU-TRP medium.

RK2575-URA cells were transformed with the above mentioned plasmid combinations as per Geitz et al. (1995) [323] and the cells were plated on SC-LEU-TRP. The plates were incubated at 30 C until colony diameter was 3-4 mm. Nine to eleven colonies from each treatment were individually collected and disbursed in 1 ml sterile distilled water (SDW). An aliquot of these cells was used to prepare serial dilutions in SDW and plated on YPD medium to determine viable cell number. Additional aliquots were plated on FOA selection medium [324]. The plates were incubated 2-5 days and the colonies were then counted. The data of viable cell number and number of FOA-resistant cells was compiled, taking into consideration the dilution factors, and analysed by the method of the median [327] with statistical analysis as described by Dixon and Massey (1969) [328]. The FOA-resistant cells represent genetic events where the chromosomal URA3 locus is converted to a mutant null allele as encoded by the gene targeting cassette of pNML18 or pNML17.

As shown in **Table 2**, the exemplified embodiments demonstrate modification of a specific target locus in a eukaryotic chromosome can be achieved by employing components involved in the DNA replication of prokaryotic or eukaryotic viruses as part of a gene targeting system as embodied here. The genetic evidence demonstrates that conversion of a target locus in a eukaryotic chromosome to an alternate allele can be promoted by employing a nickase to act on its recognition sequence and initiate replication and amplification of a linked reproducible sequence to produce gene targeting substrate which can interact with and alter the sequence of a chromosomal target locus.

**Table 2: Analysis of gene targeting systems employing φfd- and TYLCV-derived components**

| System Components | Gene Constructs | Gene Targeting Events/ Cell Division (x10⁷)^{a} | Gene Targeting Ratio^{b} |
|---|---|---|---|
| g2p-NLS | pAS27 | 0 0 | |
| φfd initiator-terminator:: ura3ΔPstI-EcoRV | pNML18 | 1.50 1.75 (1.6) | |
| g2p-NLS + φfd initiator-terminator:: ura3ΔPstI-EcoRV | pAS27 pNML18 | 30.80 25.20 (28) | 18 |
| RepC1 | pNML3 | 0 0 | |
| TYLCV initiator-terminator:: ura3ΔPstI-EcoRV | pNML17 | 3.00 1.89 (2.4) | |
| RepC1 + TYLCV initiator-terminator:: ura3APstI-EcoRV | pNML3 pNML17 | 9.74 4.98 (7.4) | 3 |

| | | | |
|---|---|---|---|
| ^{a}Represents conversion of the chromosomal URA3 locus to ura3 as detected by FOA-resistance. Numbers in parenthesis represents the average of the data from two independent experiments. ^{b}Represents the fold difference of the average number of gene targeting events observed when the nickase was combined with the gene targeting cassette vs. that observed with the gene targeting cassette alone. | | | |

The data in Table 2 indicates the chromosomal URA3 locus is very genetically stable in RK2575-URA. This is demonstrated by the fact that the rate of URA3 mutating to ura3, as indicated by the frequency of FOA-resistant cells, was zero in a strain expressing the nickase alone (i.e. RK2575-URA3/pAS27; RK2575-URA3/pNML3). This result further indicates that such nickase enzymes have no inherent tendency to alter the genetic composition of a eukaryotic host cell. The rate of converting the chromosomal URA3 locus to a null allele is increased by a very small amount when the gene targeting cassette encoding the ura3ΔPstI-EcoRV allele is present in the cell. This is demonstrated by the rate (~10⁻⁷) of occurrence of FOA-resistant cells in a strain encoding the gene targeting cassette alone (i.e. RK2575-URA3/pNML18; RK2575-URA3/pNML17). This reflects the background of homologous recombination events which occur between homologous sequences carried in the same cell (i.e. the gene targeting cassette encoding ura3ΔPstI-EcoRV and the chromosomal URSA3 locus) under the growth conditions used. However, the rate of converting the chromosomal URSA3 locus to a null allele is greatly increased over the background level when the nickase is expressed in a cell also possessing the gene targeting cassette. This is demonstrated by the 3-20-fold increase in the occurrence of FOA-resistant cells in a strain encoding the gene targeting cassette and expressing a nickase (i.e. RK2575-URA3/pAS27/pNMLl8; RK2575-URA3/pNML3/pNML17). Thus the gene targeting systems embodied here can be applied to efficiently alter eukaryotic chromosomal loci.

The data therefore demonstrates that the gene targeting systems embodied here can be used to facilitate modification of a eukaryotic chromosomal target locus at high frequency. The data further demonstrates that gene targeting systems can be developed using components of prokaryotic and eukaryotic origin involved in DNA replication. These components may be derived from a prokaryotic virus or a eukaryotic virus as embodied here with φfd- and TYLCV-derived components. The data further demonstrates that an engineered nickase of prokaryotic origin can function in eukaryotes to facilitate gene targeting. Thus g2p, and derivatives thereof (e.g. g2p-NLS), and its cognate DNA recognition sequences can be applied to facilitate gene targeting in all eukaryotic species. The data also demonstrates that a nickase of eukaryotic origin can function in heterologous eukaryotic species to facilitate gene targeting. Thus RepC1, and derivatives thereof, and its cognate DNA recognition sequences can be applied to facilitate gene targeting in all eukaryotic species.

### REFERENCES

The following documents are hereby incorporated by reference (there is no admission thereby made with respect to whether any of the documents constitute prior art with respect to any of the claims):
1. Bertling,W: Gene Targeting. In: Vega, MA (ed), Gene Targeting, pp. 1-44. CRC Press, Boca Raton (1995).
2. Lanzov,VA: Gene targeting for gene therapy: prospects. Mol.Genet.Metab 68: 276-282 (1999).
3. Roth,DB, Wilson,JH: Illegitimate recombination in mammalian cells. In: Kucherlapati, R. and Smith, G (eds), Genetic Rcombination, p. 621. American Society for Microbiology, Washington, D.C. (1988).
4. Gheysen,G, Villarroel,R, Van Montagu,M: Illegitimate recombination in plants: a model for T-DNA integration. Genes Dev. 5: 287-297 (1991).
5. Peach,C, Velten,J: Transgene expression variability (position effect) of CAT and GUS reporter genes driven by linked divergent T-DNA promoters. Plant Mol Biol 17: 49-60 (1991).
6. Mlynarova, L, Keizer,LCP, Stiekema,WJ, Nap,JP. Approaching the lower limits of transgene variability. Plant Cell 8:1589-1599. (1996).
7. Lai,LW, Lien,YH: Homologous recombination based gene therapy. Exp Nephrol. 7: 11-14 (1999).
8. Meyer,P, Saedler,H. Homology-dependent gene silencing in plants. Annu.Rev.Plant Physiol.Plant Mol.Biol. 47: 23-48. 1996.
9. Mol,JN, van der Krol,AR, van Tunen,AJ, van Blokland,R, de Lange,P, Stuitje,AR: Regulation of plant gene expression by antisense RNA. FEBS Lett 268: 427-430 (1990).
10. Rothstein,R: Targeting, disruption, replacement, and allele rescue: integrative DNA transformation in yeast. Methods Enzymol. 194: 281-301 (1991).
11. Simon,JR, Moore,PD. Homologous recombination between single-stranded DNA and chromosomal genes in Saccharomyces cerevisiae. Mol Cell Biochem 7, pp. 2329-2334. 1987.
12. Winzeler,EA, Shoemaker,DD, Astromoff,A, Liang,H, Anderson,K, Andre,B, Bangham,R, Benito,R, Boeke,JD, Bussey,H, Chu,AM, Connelly,C, Davis,K, Dietrich,F, Dow,SW, E1 Bakkoury,M, Foury,F, Friend,SH, Gentalen,E, Giaever,G, Hegemann,JH, Jones,T, Laub,M, Liao,H, Davis,RW: Functional characterization of the S. cerevisiae genome by gene deletion and parallel analysis. Science 285: 901-906 (1999).
13. Broverman,S, MacMorris,M, Blumenthal,T: Alteration of Caenorhabditis elegans gene expression by targeted transformation. Proc.Natl.Acad.Sci.U.S.A 90: 4359-4363 (1993).
14. Rong,YS, Golic,KG: Gene targeting by homologous recombination in drosophila. Science 288: 2013-2018 (2000).
15. Thomas,KR, Capecchi,MR: Site-directed mutagenesis by gene targeting in mouse embryo-derived stem cells. Cell 51: 503-512 (1987).
16. Thomas,KR, Folger,KR, Capecchi,MR: High frequency targeting of genes to specific sites in the mammalian genome. Cell 44: 419-428 (1986).
17. Thompson,S, Clarke,AR, Pow,AM, Hooper,ML, Melton,DW: Germ line transmission and expression of a corrected HPRT gene produced by gene targeting in embryonic stem cells. Cell 56: 313-321 (1989).
18. Shcherbakova,OG, Lanzov,VA, Ogawa,H, Filatov,MV: Overexpression of bacterial RecA protein stimulates homologous recombination in somatic mammalian cells. Mutat.Res. 459: 65-71 (2000).
19. Yanez,RJ, Porter,AC: Gene targeting is enhanced in human cells overexpressing hRAD51. Gene Ther. 6:1282-1290 (1999).
20. Schaefer,DG, Zryd,JP: Efficient gene targeting in the moss Physcomitrella patens. Plant J. 11: 1195-1206 (1997).
21. Zhu,T, Mettenburg,K, Peterson,DJ, Tagliani,L, Baszczynski,CL: Engineering herbicide-resistant maize using chimeric RNA/DNA oligonucleotides. Nat.Biotechnol. 18: 555-558 (2000).
22. Zhu,T, Peterson,DJ, Tagliani,L, St Clair,G, Baszczynski,CL, Bowen,B: Targeted manipulation of maize genes in vivo using chimeric RNA/DNA oligonucleotides. Proc.Natl.Acad.Sci.U.S.A 96: 8768-8773 (1999).
23. Beetham,PR, Kipp,PB, Sawycky,XL, Arntzen,CJ, May,GD: A tool for functional plant genomics: chimeric RNA/DNA oligonucleotides cause in vivo gene-specific mutations. Proc.Natl.Acad.Sci.U.S.A 96: 8774-8778 (1999).
24. Offringa,R, Franke-van Dijk,ME, De Groot,MJ, van den Elzen,PJ, Hooykaas,PJ: Nonreciprocal homologous recombination between Agrobacterium transferred DNA and a plant chromosomal locus. Proc.Natl.Acad.Sci.U.S.A 90: 7346-7350 (1993).
25. Miao,ZH, Lam,E: Targeted disruption of the TGA3 locus in Arabidopsis thaliana. Plant J. 7: 359-365 (1995).
26. Rauth,S, Song,KY, Ayares,D, Wallace,L, Moore,PD, Kucherlapati,R: Transfection and homologous recombination involving single-stranded DNA substrates in mammalian cells and nuclear extracts. Proc Natl Acad Sci U S A 83: 5587-5591 (1986).
27. De Groot,MJ, Offringa,R, Does,MP, Hooykaas,PJ, van den Elzen,PJ: Mechanisms of intermolecular homologous recombination in plants as studied with si. Nucleic Acids Res. 20: 2785-2794 (1992).
28. Alexeev,V, Igoucheva,0, Domashenko,A, Cotsarelis,G, Yoon,K: Localized in vivo genotypic and phenotypic correction of the albino mutation in skin by RNA-DNA oligonucleotide. Nat.Biotechnol. 18: 43-47 (2000).
29. Yoon,K, Cole-Strauss,A, Kmiec,EB: Targeted gene correction of episomal DNA in mammalian cells mediated by a chimeric RNA.DNA oligonucleotide. Proc.Natl.Acad.Sci.U.S.A 93: 2071-2076 (1996).
30. Cole-Strauss,A, Yoon,K, Xiang,Y, Byrne,BC, Rice,MC, Gryn,J, Holloman,WK, Kmiec,EB: Correction of the mutation responsible for sickle cell anemia by an RNA-DNA oligonucleotide. Science 273: 1386-1389 (1996).
31. Yang,XW, Model,P, Heintz,N. Homologous recombination based modification in Escherichia coli and germline transmission in transgenic mice of a bacterial artificial chromosome. Nat.Biotechnol. 15, pp. 859-865. 1997.
32. Gamper,HB, Jr., Cole-Strauss,A, Metz,R, Parekh,H, Kumar,R, Kmiec,EB: A plausible mechanism for gene correction by chimeric oligonucleotides. Biochemistry 39: 5808-5816 (2000).
33. Cole-Strauss,A, Gamper,H, Holloman,WK, Munoz,M, Cheng,N, Kmiec,EB: Targeted gene repair directed by the chimeric RNA/DNA oligonucleotide in a mammalian cell-free extract. Nucleic Acids Res 27: 1323-1330 (1999).
34. Kaeppler,SM, Kaeppler,HF, Rhee,Y: Epigenetic aspects of somaclonal variation in plants. Plant Mol Biol 43: 179-188 (2000).
35. Gallego,ME, Sirand-Pugnet,P, White,CI: Positive-negative selection and T-DNA stability in Arabidopsis transformation. Plant Mol Biol 39: 83-93 (1999).
36. Lin,FL, Sperle,K, Stemberg,N: Recombination in mouse L cells between DNA introduced into cells and homologous chromosomal sequences. Proc Natl Acad Sci U S A 82: 1391-1395 (1985).
37. Kresn,FA, Molendijk,L, Wullems,GJ, Schilperoort,RA. In vitro transformation of plant protoplasts with Ti-plasmid DNA. Nature 296:. 72. 1982.
38. Deshayes,A, Herrera-Estrella,L, Caboche,M: Liposome-mediated transformation of tobacco mesophyll protoplasts by an Escherichia coli plasmid. EMBO J 4: 2731-2737 (1985).
39. Brinster,RL, Braun,RE, Lo,D, Avarbock,MR, Oram,F, Palmiter,RD: Targeted correction of a major histocompatibility class II E alpha gene by DNA microinjected into mouse eggs. Proc Natl Acad Sci U S A 86: 7087-7091 (1989).
40. Shillito,RD, Saul,MW, Paszkowski,J, Muller,M, Potrykus,I. High efficiency direct gene transfer to plants. Bio/technology 3:. 1099. (1985).
41. D'Halluin,K, Bonne,E, Bossut,M, De Beuckeleer,M, Leemans,J: Transgenic maize plants by tissue electroporation. Plant Cell 4: 1495-1505 (1992).
42. Crossway,A, Oakes,JV, Irvine,JM, Ward,B, Knauf,VC, Shewmaker,CK. Integration of foreign DNA following microinjection of tobacco mesophyll protoplasts. Mol Gen Genet 202: 179. (1986).
43. Yoshida,K, Takegami,T, Katoh,A, Nishikawa,M, Nishida,T: Construction of a novel conjugative plasmid harboring a GFP reporter gene and its introduction into animal cells by transfection and trans-kingdom conjugation. Nucleic Acids Symp Ser. 157-158 (1997).
44. Negritto,MT, Wu,X, Kuo,T, Chu,S, Bailis,AM: Influence of DNA sequence identity on efficiency of targeted gene replacement. Mol Cell Biol 17: 278-286 (1997).
45. Bennett,CB, Lewis,AL, Baldwin,KK, Resnick,MA: Lethality induced by a single site-specific double-strand break in a dispensable yeast plasmid. Proc Natl Acad Sci U S A 90: 5613-5617 (1993).
46. Cummings,WJ, Zolan,ME: Functions of DNA repair genes during meiosis. Curr.Top.Dev.Biol. 37: 117-140 (1998).
47. Galli,A, Schiestl,RH: Effects of DNA double-strand and single-strand breaks on intrachromosomal recombination events in cell-cycle-arrested yeast cells. Genetics 149: 1235-1250 (1998).
48. Lebkowski,JS, DuBridge,RB, Antell,EA, Greisen,KS, Calos,MP: Transfected DNA is mutated in monkey, mouse, and human cells. Mol Cell Biol 4: 1951-1960 (1984).
49. Wake,CT, Gudewicz,T, Porter,T, White,A, Wilson,JH: How damaged is the biologically active subpopulation of transfected DNA? Mol Cell Biol 4: 387-398 (1984).
50. Perucho,M, Hanahan,D, Wigler,M: Genetic and physical linkage of exogenous sequences in transformed cells. Cell 22: 309-317 (1980).
51. Deng,C, Capecchi,MR: Reexamination of gene targeting frequency as a function of the extent of homology between the targeting vector and the target locus. Mol Cell Biol 12: 3365-3371 (1992).
52. Orr-Weaver,TL, Szostak,JW, Rothstein,RJ: Yeast transformation: a model system for the study of recombination. Proc Natl Acad Sci U S A 78: 6354-6358 (1981).
53. Jasin,M, Berg,P: Homologous integration in mammalian cells without target gene selection. Genes Dev. 2: 1353-1363 (1988).
54. Puchta,H, Dujon,B, Hohn,B: Homologous recombination in plant cells is enhanced by in vivo induction of double strand breaks into DNA by a site-specific endonuclease. Nucleic Acids Res 21: 5034-5040 (1993).
55. Ilyina,TV, Koonin,EV: Conserved sequence motifs in the initiator proteins for rolling circle DNA replication encoded by diverse replicons from eubacteria, eucaryotes and archaebacteria. Nucleic Acids Res 20: 3279-3285 (1992).
56. Dujon,B: Group I introns as mobile genetic elements: facts and mechanistic speculations--a review. Gene 82: 91-114 (1989).
57. Colleaux,L, D'Auriol,L, Galibert,F, Dujon,B: Recognition and cleavage site of the intron-encoded omega transposase. Proc Natl Acad Sci U S A 85: 6022-6026 (1988).
58. Jin,Y, Binkowski,G, Simon,LD, Norris,D: Ho endonuclease cleaves MAT DNA in vitro by an inefficient stoichiometric reaction mechanism. J Biol Chem 272: 7352-7359 (1997).
59. Nicolas,AL, Munz,PL, Falck-Pedersen,E, Young,CS: Creation and repair of specific DNA double-strand breaks in vivo following infection with adenovirus vectors expressing Saccharomyces cerevisiae HO endonuclease. Virology 266: 211-224 (2000).
60. Gasser,CS, Fraley,RT. Genetically engineering plants for crop improvement. Science 244: 1293. (1989).
61. Klein,TM, Harper,EC, Svab,Z, Sanford,JC, Fromm,ME, Maliga,P. Stable genetic transformation of intact Nicotiana cells by the particle bombardment process. Proc Natl Acad Sci U S A 85: 8502. (1988).
62. Wong,EA, Capecchi,MR: Homologous recombination between coinjected DNA sequences peaks in early to mid-S phase. Mol Cell Biol 7: 2294-2295 (1987).
63. Merrill,GF: Cell synchronization. Methods Cell Biol 57: 229-249 (1998).
64. Reichheld,JP, Gigot,C, Chaubet-Gigot,N: Multilevel regulation of histone gene expression during the cell cycle in tobacco cells. Nucleic Acids Res 26: 3255-3262 (1998).
65. Osley,MA: The regulation of histone synthesis in the cell cycle. Annu.Rev Biochem 60: 827-861 (1991).
66. Huntley,RP, Murray,JA: The plant cell cycle. Curr.Opin.Plant Biol 2: 440-446 (1999).
67. Roeder,GS: Meiotic chromosomes: it takes two to tango. Genes Dev. 11: 2600-2621 (1997).
68. Klimyuk,VI, Jones,JD: AtDMC1, the Arabidopsis homologue of the yeast DMC1 gene: characterization, transposon-induced allelic variation and meiosis-associated expression. Plant J. 11: 1-14 (1997).
69. Ross-Macdonald,P, Roeder,GS: Mutation of a meiosis-specific MutS homolog decreases crossing over but not mismatch correction. Cell 79: 1069-1080 (1994).
70. Kobayashi,T, Kobayashi,E, Sato,S, Hotta,Y, Miyajima,N, Tanaka,A, Tabata,S: Characterization of cDNAs induced in meiotic prophase in lily microsporocytes. DNA Res. 1: 15-26 (1994).
71. Chu,S, DeRisi,J, Eisen,M, Mulholland,J, Botstein,D, Brown,PO, Herskowitz,I: The transcriptional program of sporulation in budging yeast. Science 282: 699-705 (1998).
72. Tsuzuki,T, Fujii,Y, Sakumi,K, Tominaga,Y, Nakao,K, Sekiguchi,M, Matsushiro,A, Yoshimura,Y, MoritaT: Targeted disruption of the Rad51 gene leads to lethality in embryonic mice. Proc.Natl.Acad.Sci.U.S.A 93: 6236-6240 (1996).
73. Coventry,J, Kott,L, Beversdorf,W: Manual for microspore culture technique for Brassica napus. University of Guelph, Guelph (1988).
74. Offringa,R, De Groot,MJ, Haagsman,HJ, Does,MP, van den Elzen,PJ, Hooykaas,PJ: Extrachromosomal homologous recombination and gene targeting in plant cells after Agrobacterium mediated transformation. EMBO J. 9: 3077-3084 (1990).
75. Friedberg,EC, Walker,GC, Siede,W: DNA Repair and Mutagenesis. American Society for Microbiology, Washington, D.C. (1995).
76. Hoffmann,GR: Induction of genetic recombination: consequences and model systems. Environ.Mol Mutagen. 23 Suppl 24: 59-66 (1994).
77. Schiestl,RH: Nonmutagenic carcinogens induce intrachromosomal recombination in yeast. Nature 337: 285-288 (1989).
78. Basile,G, Aker,M, Mortimer,RK: Nucleotide sequence and transcriptional regulation of the yeast recombinational repair gene RAD51. Mol.Cell Biol. 12: 3235-3246 (1992).
79. Rozwadowski,K, Kreiser,T, Hasnadka,R, Lydiate,D. AtMRE11: a component of meiotic recombination and DNA repair in plants. 10th International Conference on Arabidopsis Research, Melbourne, Australia, July 4-8, 1999. 1999.
80. Ainley,WM, Key,JL: Development of a heat shock inducible expression cassette for plants: characterization of parameters for its use in transient expression assays. Plant Mol.Biol. 14: 949-967 (1990).
81. Martinez,A, Sparks,C, Hart,CA, Thompson,J, Jepson,I: Ecdysone agonist inducible transcription in transgenic tobacco plants. Plant J. 19: 97-106 (1999).
82. Bohner,S, Lenk,I, Rieping,M, Herold,M, Gatz,C: Technical advance: transcriptional activator TGV mediates dexamethasone-inducible and tetracycline-inactivatable gene expression. Plant J. 19: 87-95 (1999).
83. Gatz,C, Kaiser,A, Wendenburg,R: Regulation of a modified CaMV 35S promoter by the Tn10-encoded Tet repressor in transgenic tobacco. Mol.Gen.Genet. 227: 229-237 (1991).
84. Weinmann,P, Gossen,M, Hillen,W, Bujard,H, Gatz,C: A chimeric transactivator allows tetracycline-responsive gene expression in whole plants. Plant J. 5: 559-569 (1994).
85. Mett,VL, Podivinsky,E, Tennant,AM, Lochhead,LP, Jones,WT, Reynolds,PH: A system for tissue-specific copper-controllable gene expression in transgenic plants: nodule-specific antisense of aspartate aminotransferase-P2. Transgenic Res. 5: 105-113 (1996).
86. Mett,VL, Lochhead,LP, Reynolds,PH: Copper-controllable gene expression system for whole plants. Proc.Natl.Acad.Sci.U.S.A 90: 4567-4571 (1993).
87. Guyer,D, Tuttle,A, Rouse,S, Volrath,S, Johnson,M, Potter,S, Gorlach,J, Goff,S, Crossland,L, Ward,E: Activation of latent transgenes in Arabidopsis using a hybrid transcription factor. Genetics 149: 633-639 (1998).
88. Moore,I, Galweiler,L, Grosskopf,D, Schell,J, Palme,K: A transcription activation system for regulated gene expression in transgenic plants. Proc.Natl.Acad.Sci.U.S.A 95: 376-381 (1998).
89. Labow,MsA, Baim,SB, Shenk,T, Levine,AJ: Conversion of the lac repressor into an allosterically regulated transcriptional activator for mammalian cells. Mol.Cell Biol. 10: 3343-3356 (1990).
90. Benton,BM, Eng,WK, Dunn,JJ, Studier,FW, Sternglanz,R, Fisher,PA: Signal-mediated import of bacteriophage T7 RNA polymerase into the Saccharomyces cerevisiae nucleus and specific transcription of target genes. Mol.Cell Biol. 10: 353-360 (1990).
91. Bechtold,N, Pelletier,G: In planta Agrobacterium-mediated transformation of adult Arabidopsis thaliana plants by vacuum infiltration. Methods Mol Biol 82: 259-266 (1998).
92. Clough,SJ, Bent,AF: Floral dip: a simplified method for Agrobacterium-mediated transformation of Arabidopsis thaliana. Plant J 16: 735-743 (1998).
93. Scholz,S, Scholthof,K-BG: Plant virus gene vectors for transient expression of foreign proteins in plants. Annu.Rev.of Phytopathol. 34: 299-323 (1996).
94. Wilmut,I, Schnieke,AE, McWhir,J, Kind,AJ, Campbell,KH: Viable offspring derived from fetal and adult mammalian cells. Nature 385: 810-813 (1997).
95. Model,P, Russel,M: Filamentous Bacteriophage. In: Calendar, R. (ed), The Bacteriophages, pp. 375-456. Plenum Press, New York (1988).
96. Hayashi,M, Aoyama,A, Richardson Jr.,Dl, Hayashi,MN: Biology of the bacteriophage phiX174. In: Calendar, R (ed), The Bacteriophages, pp. 1-71. Plenum Press, New York (1988).
97. Chang,TL, Kramer,MG, Ansari,RA, Khan,SA: Role of individual monomers of a dimeric initiator protein in the initiation and termination of plasmid rolling circle replication. J Biol Chem 275: 13529-13534 (2000).
98. Novick,RP: Contrasting lifestyles of rolling-circle phages and plasmids. Trends Biochem Sci 23: 434-438 (1998).
99. Castellano,MM, Sanz-Burgos,AP, Gutierrez,C: Initiation of DNA replication in a eukaryotic rolling-circle replicon: identification of multiple DNA-protein complexes at the geminivirus origin. J Mol Biol 290: 639-652 (1999).
100. Meehan,BM, Creelan,JL, McNulty,MS, Todd,D: Sequence of porcine circovirus DNA: affinities with plant circoviruses. J Gen Virol 78: 221-227 (1997).
101. Pansegrau,W, Lanka,E. Enzymology of DNA transfer by conjugative mechanisms. Progress in Nucleic Acid Research and Molecular Biology 54: 197-251. (1996).
102. Cotmore,SF, Tattersall,P: High-mobility group 1/2 proteins are essential for initiating rolling-circle-type DNA replication at a parvovirus hairpin origin. J Virol 72: 8477-8484 (1998).
103. Im,DS, Muzyczka,N: The AAV origin binding protein Rep68 is an ATP-dependent site-specific endonuclease with DNA helicase activity. Cell 61: 447-457 (1990).
104. Laufs,J, Jupin,I, David,C, Schumacher,S, Heyraud-Nitschke,F, Gronenborn,B: Geminivirus replication: genetic and biochemical characterization of Rep protein function, a review. Biochimie 77: 765-773 (1995).
105. Sims,J, Capon,D, Dressler,D: dnaG (primase)-dependent origins of DNA replication. Nucleotide sequences of the negative strand initiation sites of bacteriophages St-1, phi K, and alpha 3. J Biol Chem 254: 12615-12628 (1979).
106. Heidekamp,F, Baas,PD, Jansz,HS: Nucleotide sequences at the phi X gene A protein cleavage site in replicative form I DNAs of bacteriophages U3, G14, and alpha 3. J Virol 42: 91-99 (1982).
107. Godson,GN, Barrell,BG, Staden,R, Fiddes,JC: Nucleotide sequence of bacteriophage G4 DNA. Nature 276: 236-247 (1978).
108. Gielow,A, Diederich,L, Messer,W: Characterization of a phage-plasmid hybrid (phasyl) with two independent origins of replication isolated from Escherichia coli. J Bacteriol 173: 73-79 (1991).
109. Harding,RM, Burns,TM, Hafner,G, Dietzgen,RG, Dale,JL: Nucleotide sequence of one component of the banana bunchy top virus genome contains a putative replicase gene. J Gen Virol 74 : 323-328 (1993).
110. Hafner,GJ, Stafford,MR, Wolter,LC, Harding,RM, Dale,JL: Nicking and joining activity of banana bunchy top virus replication protein in vitro. J Gen Virol 78: 1795-1799 (1997).
111. Chu,PW, Keese,P, Qiu,BS, Waterhouse,PM, Gerlach,WL: Putative full-length clones of the genomic DNA segments of subterranean clover stunt virus and identification of the segment coding for the viral coat protein. Virus Res 27: 161-171 (1993).
112. Rohde,W, Randles,JW, Langridge,P, Hanold,D: Nucleotide sequence of a circular single-stranded DNA associated with coconut foliar decay virus. Virology 176: 648-651 (1990).
113. Todd,D, Creelan,JL, Mackie,DP, Rixon,F, McNulty,MS: Purification and biochemical characterization of chicken anaemia agent. J Gen Virol 71: 819-823 (1990).
114. Ritchie,BW, Niagro,FD, Lukert,PD, Steffens,WL, III, Latimer,KS: Characterization of a new virus from cockatoos with psittacine beak and feather disease. Virology 171: 83-88 (1989).
115. Snyder,RO, Im,DS, Ni,T, Xiao,X, Samulski,RJ, Muzyczka,N: Features of the adeno-associated virus origin involved in substrate recognition by the viral Rep protein. J Virol 67: 6096-6104 (1993).
116. Brister,JR, Muzyczka,N: Mechanism of Rep-mediated adeno-associated virus origin nicking. J Virol 74: 7762-7771 (2000). 117. Nuesch,JP, Cotmore,SF, Tattersall,P: Sequence motifs in the replicator protein of parvovirus MVM essential for nicking and covalent attachment to the viral origin: identification of the linking tyrosine. Virology 209:122-135.
118. Noirot-Gros,MF, Bidnenko,V, Ehrlich,SD: Active site of the replication protein of the rolling circle plasmid pC194. EMBO J 13: 4412-4420 (1994).
119. Gros,MF, te,RH, Ehrlich,SD: Replication origin of a single-stranded DNA plasmid pC194. EMBO J 8: 2711-2716 (1989).
120. Koepsel,RR, Murray,RW, Rosenblum,WD, Khan,SA: The replication initiator protein of plasmid pT181 has sequence-specific endonuclease and topoisomerase-like activities. Proc Natl Acad Sci U S A 82: 6845-6849 (1985).
121. Murray,RW, Koepsel,RR, Khan,SA: Synthesis of single-stranded plasmid pT181 DNA in vitro. Initiation and termination of DNA replication. J Biol Chem 264: 1051-1057 (1989).
122. Boe,L, Gros,MF, te,RH, Ehrlich,SD, Gruss,A: Replication origins of single-stranded-DNA plasmid pUB110. J Bacteriol 171: 3366-3372 (1989).
123. Yang,X, McFadden,BA: A small plasmid, pCA2.4, from the cyanobacterium Synechocystis sp. strain PCC 6803 encodes a rep protein and replicates by a rolling circle mechanism. J Bacteriol 175: 3981-3991 (1993).
124. Sozhamannan,S, Dabert,P, Moretto,V, Ehrlich,SD, Gruss,A: Plus-origin mapping of single-stranded DNA plasmid pE194 and nick site homologies with other plasmids. J Bacteriol 172: 4543-4548 (1990).
125. Yasukawa,H, Hase,T, Sakai,A, Masamune,Y: Rolling-circle replication of the plasmid pKYM isolated from a gram-negative bacterium. Proc Natl Acad Sci U S A 88: 10282-10286 (1991).
126. Yasukawa,H, Masamune,Y: Rolling-circle plasmid pKYM re-initiates DNA replication. DNA Res 4: 193-197 (1997).
127. Gruss,A, Ehrlich,SD: The family of highly interrelated single-stranded deoxyribonucleic acid plasmids. Microbiol Rev 53: 231-241 (1989).
128. Espinosa,M, del Solar,G, Rojo,F, Alonso,JC: Plasmid rolling circle replication and its control. FEMS Microbiol Lett 130: 111-120 (1995).
129. del Solar,G, Giraldo,R, Ruiz-Echevarria,MJ, Espinosa,M, Diaz-Orejas,R: Replication and control of circular bacterial plasmids. Microbiol Mol Biol Rev 62: 434-464 (1998).
130. Matson,SW, Nelson,WC, Morton,BS: Characterization of the reaction product of the oriT nicking reaction catalyzed by Escherichia coli DNA helicase I. J Bacteriol 175: 2599-2606 (1993).
131. Llosa,M, Bolland,S, de la,CF: Structural and functional analysis of the origin of conjugal transfer of the broad-host-range IncW plasmid R388 and comparison with the related IncN plasmid R46. Mol Gen Genet 226: 473-483 (1991).
132. Pansegrau,W, Lanka,E: Mechanisms of initiation and termination reactions in conjugative DNA processing. Independence of tight substrate binding and catalytic activity of relaxase (TraI) of IncPalpha plasmid RP4. J Biol Chem 271: 13068-13076 (1996).
133. Furste,JP, Pansegrau,W, Ziegelin,G, Kroger,M, Lanka,E: Conjugative transfer of promiscuous IncP plasmids: interaction of plasmid-encoded products with the transfer origin. Proc Natl Acad Sci U S A 86: 1771-1775 (1989).
134. Scherzinger,E, Ziegelin,G, Barcena,M, Carazo,JM, Lurz,R, Lanka,E: The RepA protein of plasmid RSF1010 is a replicative DNA helicase. J Biol Chem 272: 30228-30236 (1997).
135. Coupland,GM, Brown,AM, Willetts,NS: The origin of transfer (oriT) of the conjugative plasmid R46: characterization by deletion analysis and DNA sequencing. Mol Gen Genet 208: 219-225 (1987).
136. Finlay,BB, Frost,LS, Paranchych,W: Origin of transfer of IncF plasmids and nucleotide sequences of the type II oriT, traM, and traY alleles from ColB4-K98 and the type IV traY allele from R100-1. J Bacteriol 168: 132-139 (1986).
137. Furuya,N, Nisioka,T, Komano,T: Nucleotide sequence and functions of the oriT operon in IncI1 plasmid R64. J Bacteriol 173: 2231-2237 (1991).
138. Murphy,CG, Malamy,MH: Requirements for strand- and sitespecific cleavage within oriT region of Tn4399, a mobilizing transposon from Bacteroides fragilis. J Bacteriol 177: 3158-3165 (1995).
139. Murphy,CG, Malamy,MH: Characterization of a "mobilization cassette" in transposon Tn4399 from Bacteroides fragilis. J Bacteriol 175: 5814-5823 (1993).
140. Bastia,D: Determination of restriction sites and the nucleotide sequence surrounding the relaxation site of ColE1. J Mol Biol 124: 601-639 (1978).
141. Roessler,E, Fenwick,RG, Jr., Chinault,AC: Analysis of mobilization elements in plasmids from Shigella flexneri. J Bacteriol 161: 1233-1235 (1985).
142. Snijders,A, van Putten,AJ, Veltkamp,E, Nijkamp,HJ: Localization and nucleotide sequence of the bom region of Clo DF13. Mol Gen Genet 192: 444-451 (1983).
143. Bernardi,A, Bernardi,F: Complete sequence of pSC101. Nucleic Acids Res 12: 9415-9426 (1984).
144. Beck,E, Zink,B: Nucleotide sequence and genome organisation of filamentous bacteriophages fl and fd. Gene 16: 35-58 (1981).
145. Sanger,F, Air,GM, Barrell,BG, Brown,NL, Coulson,AR, Fiddes,CA, Hutchison,CA, Slocombe,PM, Smith,M: Nucliotide sequence of bacteriophage phi X174 DNA. Nature 265: 687-695 (1977).
146. Meyer,TF, Geider,K: Enzymatic synthesis of bacteriophage fd viral DNA. Nature 296: 828-832 (1982).
147. Harth,G, Baumel,I, Meyer,TF, Geider,K: Bacteriophage fd gene-2 protein. Processing of phage fd viral strands replicated by phage T7 enzymes. Eur J Biochem 119: 663-668 (1981).
148. Shavitt,O, Livneh,Z: Rolling-circle replication of UV-irradiated duplex DNA in the phi X174 replicative-form----single-strand replication system in vitro. J Bacteriol 171: 3530-3538 (1989).
149. Lin,NS, Pratt,D: Role of bacteriophage M13 gene 2 in viral DNA replication. J Mol Biol 72: 37-49 (1972).
150. Goetz,GS, Hurwitz,J: Studies on the role of the phi X174 gene A protein in phi X viral strand synthesis. I. Replication of DNA containing an alteration in position 1 of the 30-nucleotide icosahedral bacteriophage origin. J Biol Chem 263: 16421-16432 (1988).
151. Hanai,R, Wang,JC: The mechanism of sequence-specific DNA cleavage and strand transfer by phi X174 gene A* protein. J Biol Chem 268: 23830-23836 (1993).
152. Higashitani,A, Greenstein,D, Hirokawa,H, Asano,S, Horiuchi,K: Multiple DNA conformational changes induced by an initiator protein precede the nicking reaction in a rolling circle replication origin. J Mol Biol 237: 388-400 (1994).
153. Asano,S, Higashitani,A, Horiuchi,K: Filamentous phage replication initiator protein gpII forms a covalent complex with the 5' end of the nick it introduced. Nucleic Acids Res 27: 1882-1889 (1999).
154. Higashitani,A, Greenstein,D, Horiuchi,K: A single amino acid substitution reduces the superhelicity requirement of a replication initiator protein. Nucleic Acids Res 20: 2685-2691 (1992).
155. Greenstein,D, Horiuchi,K: Double-strand cleavage and strand joining by the replication initiator protein of filamentous phage fl. J Biol Chem 264: 12627-12632 (1989).
156. Fluit,AC, Baas,PD, Van Boom,JH, Veeneman,GH, Jansz,HS: Gene A protein cleavage of recombinant plasmids containing the phi X174 replication origin. Nucleic Acids Res 12: 6443-6454 (1984).
157. van Mansfeld,AD, van Teeffelen,HA, Baas,PD, Jansz,HS: Two juxtaposed tyrosyl-OH groups participate in phi X174 gene A protein catalysed cleavage and ligation of DNA. Nucleic Acids Res 14: 4229-4238 (1986).
158. van Mansfeld,AD, van Teeffelen,HA, Baas,PD, Veeneman,GH, Van Boom,JH, Jansz,HS: The bond in the bacteriophage phi X174 gene A protein--DNA complex is a tyrosyl-5'-phosphate ester. FEBS Lett 173: 351-356 (1984).
159. van Mansfeld,AD, Baas,PD, Jansz.HS: Gene A protein of bacteriophage phi X174 is a highly specific single-strand nuclease and binds via a tyrosyl residue to DNA after cleavage. Adv Exp Med Biol 179: 221-230 (1984).
160. Dente,L, Cesareni,G, Cortese,R: pEMBL: a new family of single stranded plasmids. Nucleic Acids Res 11: 1645-1655 (1983).
161. Dotto,GP, Enea,V, Zinder,ND: Functional analysis of bacteriophage fl intergenic region. Virology 114: 463-473 (1981).
162. Fluit,AC, Baas,PD, Jansz,HS: The complete 30-base pair origin region of bacteriophage phi X174 in a plasmid is both required and sufficient for in vivo rolling-circle DNA replication and packaging. Eur J Biochem 149: 579-584 (1985).
163. van der,EA, Teertstra,R, Weisbeek,PJ: Initiation and termination of the bacteriophage phi X174 rolling circle DNA replication in vivo: packaging of plasmid single-stranded DNA into bacteriophage phi X174 coats. Nucleic Acids Res 10: 6849-6863 (1982).
164. Dotto,GP, Zinder,ND: Increased intracellular concentration of an initiator protein markedly reduces the minimal sequence required for initiation of DNA synthesis. Proc Natl Acad Sci U S A 81: 1336-1340 (1984).
165. Goetz,GS, Hurwitz,J: Studies on the role of the phi X174 gene A protein in phi X174 viral strand synthesis. III. Replication of DNA containing two viral replication origins. J Biol Chem 263: 16443-16451 (1988).
166. Goetz,GS, Schmidt-Glenewinkel,T, Hu,MH, Belgado,N, Hurwitz,J: Studies on the role of the phi X174 gene A protein in phi X viral strand synthesis. II. Effects of DNA replication of mutations in the 30-nucleotide icosahedral bacteriophage origin. J Biol Chem 263: 16433-16442 (1988).
167. Reinberg,D, Zipursky,SL, Weisbeek,P, Brown,D, Hurwitz,J: Studies on the phi X174 gene A protein-mediated termination of leading strand DNA synthesis. J Biol Chem 258: 529-537 (1983).
168. Dotto,GP, Horiuchi,K, Zinder,ND: Initiation and termination of phage fl plus-strand synthesis. Proc Natl Acad Sci U S A 79: 7122-7126 (1982).
169. Short,JM, Fernandez,JM, Sorge,JA, Huse,WD: Lambda ZAP: a bacteriophage lambda expression vector with in vivo excision properties. Nucleic Acids Res 16: 7583-7600 (1988).
170. Dotto,GP, Horiuchi,K: Replication of a plasmid containing two origins of bacteriophage. J Mol Biol 153: 169-176 (1981).
171. Dotto,GP, Horiuchi,K, Zinder,ND: The functional origin of bacteriophage fl DNA replication. Its signals and domains. J Mol Biol 172: 507-521 (1984).
172. Meyer,TF, Geider,K: Cloning of bacteriophage fd gene 2 and construction of a plasmid dependent on fd gene 2 protein. Proc Natl Acad Sci U S A 78: 5416-5420 (1981).
173. Strathern,JN, Weinstock,KG, Higgins,DR, McGiII,CB: A novel recombinator in yeast based on gene II protein from bacteriophage fl. Genetics 127: 61-73 (1991).
174. Heyraud-Nitschke,F, Schumacher,S, Laufs,J, Schaefer,S, Schell,J, Gronenborn,B: Determination of the origin cleavage and joining domain of geminivirus Rep proteins. Nucleic Acids Res 23: 910-916 (1995).
175. Choi,IR, Stenger,DC: Strain-specific determinants of beet curly top geminivirus DNA replication. Virology 206: 904-912 (1995).
176. Laufs,J, Traut,W, Heyraud,F, Matzeit,V, Rogers,SG, Schell,J, Gronenborn,B: In vitro cleavage and joining at the viral origin of replication by the replication initiator protein of tomato yellow leaf curl virus. Proc Natl Acad Sci USA 92: 3879-3883 (1995).
177. Desbiez,C, David,C, Mettouchi,A, Laufs,J, Gronenborn,B: Rep protein of tomato yellow leaf curl geminivirus has an ATPase activity required for viral DNA replication. Proc Natl Acad Sci U S A 92: 5640-5644 (1995).
178. Laufs,J, Schumacher,S, Geisler,N, Jupin,I, Gronenborn,B: Identification of the nicking tyrosine of geminivirus Rep protein. FEBS Lett 377: 258-262 (1995).
179. Orozco,BM, Hanley-Bowdoin,L: Conserved sequence and structural motifs contribute to the DNA binding and cleavage activities of a geminivirus replication protein. J Biol Chem 273: 24448-24456 (1998).
180. Orozco,BM, Kong,LJ, Batts,LA, Elledge,S, Hanley-Bowdoin,L: The multifunctional character of a geminivirus replication protein is reflected by its complex oligomerization properties. J Biol Chem 275: 6114-6122 (2000).
181. Orozco,BM, Miller,AB, Settlage,SB, Hanley-Bowdoin,L: Functional domains of a geminivirus replication protein. J Biol Chem 272: 9840-9846 (1997).
182. Lazarowitz,SG, Wu,LC, Rogers,SG, Elmer,JS: Sequence-specific interaction with the viral AL1 protein identifies a geminivirus DNA replication origin. Plant Cell 4: 799-809 (1992).
183. Jupin,I, Hericourt,F, Benz,B, Gronenborn,B: DNA replication specificity of TYLCV geminivirus is mediated by the amino-terminal 116 amino acids of the Rep protein. FEBS Lett 362: 116-120 (1995).
184. Rigden,JE, Dry,IB, Krake,LR, Rezaian,MA: Plant virus DNA replication processes in Agrobacterium: insight into the origins of geminiviruses? Proc Natl Acad Sci U S A 93: 10280-10284 (1996).
185. Akbar Behjatnia,SA, Dry,IB, Ali,RM: Identification of the replication-associated protein binding domain within the intergenic region of tomato leaf curl geminivirus. Nucleic Acids Res 26: 925-931 (1998).
186. Fontes,EP, Eagle,PA, Sipe,PS, Luckow,VA, Hanley-Bowdoin,L: Interaction between a geminivirus replication protein and origin DNA is essential for viral replication. J Biol Chem 269: 8459-8465 (1994).
187. Sanz-Burgos,AP, Gutierrez,C: Organization of the cis-acting element required for wheat dwarf geminivirus DNA replication and visualization of a rep protein-DNA complex. Virology 243: 119-129 (1998).
188. Woolston,CJ, Barker,R, Gunn,H, Boulton,MI, Mullineaux,PM. Agroinfection and nucleotide sequence of cloned wheat dwarf virus DNA. Plant Mol.Biol. 11:. 35-43. 1988.
189. Navot,N, Pichersky,E, Zeidan,M, Zamir,D, Czosnek,H: Tomato yellow leaf curl virus: a whitefly-transmitted geminivirus with a single genomic component. Virology 185: 151-161 (1991).
190. Dry,IB, Rigden,JE, Krake,LR, Mullineaux,PM, Rezaian,MA: Nucleotide sequence and genome organization of tomato leaf curl geminivirus. J Gen Virol 74: 147-151 (1993).
191. Mankertz,A, Mankertz,J, Wolf,K, Buhk,HJ: Identification of a protein essential for replication of porcine circovirus. J Gen Virol 79: 381-384 (1998).
192. Mankertz,A, Persson,F, Mankertz,J, Blaess,G, Buhk,HJ: Mapping and characterization of the origin of DNA replication of porcine circovirus. J Virol 71: 2562-2566 (1997).
193. Backert,S, Dorfel,P, Lurz,R, Borner,T: Rolling-circle replication of mitochondrial DNA in the higher plant Chenopodium album (L.). Mol Cell Biol 16: 6285-6294 (1996).
194. Gros,MF, te,RH, Ehrlich,SD: Rolling circle replication of single-stranded DNA plasmid pC194. EMBO J 6: 3863-3869 (1987).
195. Firth,N, Ippen-Ihler,K, Skurray,RA: Structure and function of the F factor and mechanism of conjugation. In: Neidhardt, F (ed), Escherichia coli and Salmonella, pp. 2377-2401. American Society for Microbiology, (1995).
196. Lessl,M, Lanka,E: Common mechanisms in bacterial conjugation and Ti-mediated T-DNA transfer to plant cells. Cell 77: 321-324 (1994).
197. Nishikawa,M, Suzuki,K, Yoshida,K: Structural and functional stability of IncP plasmids during stepwise transmission by trans-kingdom mating: promiscuous conjugation of Escherichia coli and Saccharomyces cerevisiae. Jpn.J Genet 65: 323-334 (1990).
198. Byrd,DR, Matson,SW: Nicking by transesterification: the reaction catalysed by a relaxase. Mol Microbiol 25: 1011-1022 (1997).
199. Liosa,M, Grandoso,G, Hernando,MA, de la,CF: Functional domains in protein TrwC of plasmid R388: dissected DNA strand transferase and DNA helicase activities reconstitute protein function. J Mol Biol 264: 56-67 (1996).
200. Grandoso,G, Avila,P, Cayon,A, Hernando,MA, Llosa,M, de la,CF: Two active-site tyrosyl residues of protein TrwC act sequentially at the origin of transfer during plasmid R388 conjugation. J Mol Biol 295: 1163-1172 (2000).
201. Grandoso,G, Llosa,M, Zabala,JC, de la,CF: Purification and biochemical characterization of TrwC, the helicase involved in plasmid R388 conjugal DNA transfer. Eur J Biochem 226: 403-412 (1994).
202. Llosa,M, Grandoso,G, de la,CF: Nicking activity of TrwC directed against the origin of transfer of the IncW plasmid R388. J Mol Biol 246: 54-62 (1995).
203. Pansegrau,W, Ziegelin,G, Lanka,E: Covalent association of the traI gene , product of plasmid RP4 with the 5'-terminal nucleotide at the relaxation nick site. J Biol Chem 265: 10637-10644 (1990).
204. Scherzinger,E, Kruft,V, Otto,S: Purification of the large mobilization protein of plasmid RSF1010 and characterization of its site-specific DNA-cleaving/DNA-joining activity. Eur J Biochem 217: 929-938 (1993).
205. Scherzinger,E, Lurz,R, Otto,S, Dobrinski,B: In vitro cleavage of do. Nucleic Acids Res 20:41-48 (1992).
206. Sherman,JA, Matson,SW: Escherichia coli DNA helicase I catalyzes a sequence-specific cleavage/ligation reaction at the F plasmid origin of transfer. J Biol Chem 269: 26220-26226 (1994).
207. Matson,SW, Morton,BS: Escherichia coli DNA helicase I catalyzes a. J Biol Chem 266: 16232-16237 (1991).
208. Moncalian,G, Grandoso,G, Llosa,M, de la,CF: oriT-processing and regulatory roles of TrwA protein in plasmid R388 conjugation. J Mol Biol 270: 188-200 (1997).
209. Moncalian,G, Cabezon,E, Alkorta,I, Valle,M, Moro,F, Valpuesta,JM, Goni,FM, de la,CF: Characterization of ATP and DNA binding activities of TrwB, the coupling protein essential in plasmid R388 conjugation. J Biol Chem 274: 36117-36124 (1999).
210. Ziegelin,G, Pansegrau,W, Lurz,R, Lanka,E: TraK protein of conjugative plasmid RP4 forms a specialized nucleoprotein complex with the transfer origin. J Biol Chem 267: 17279-17286 (1992).
211. Fekete,RA, Frost,LS: Mobilization of chimeric oriT plasmids by F and R100-1: role of relaxosome formation in defining plasmid specificity. J Bacteriol 182: 4022-4027 (2000).
212. Bravo-Angel,AM, Gloeckler,V, Hohn,B, Tinland,B: Bacterial conjugation protein MobA mediates integration of complex DNA structures into plant cells. J Bacteriol 181: 5758-5765 (1999).
213. Turlan,C, Chandler,M: Playing second fiddle: second-strand processing and liberation of transposable elements from donor DNA. Trends Microbiol 8: 268-274 (2000).
214. Stellwagen,AE, Craig,NL: Mobile DNA elements: controlling transposition with ATP-dependent molecular switches. Trends Biochem Sci 23: 486-490 (1998).
215. Haren,L, Ton-Hoang,B, Chandler,M: Integrating DNA: transposases and retroviral integrases. Annu.Rev Microbiol 53: 245-281 (1999).
216. Whiteley,M, Kassis,JA: Rescue of Drosophila engrailed mutants with a highly divergent mosquito engrailed cDNA using a homing, enhancer-trapping transposon. Development 124: 1531-1541 (1997).
217. Maes,T, De Keukeleire,P, Gerats,T: Plant tagnology. Trends Plant Sci 4: 90-96 (1999).
218. New England Biolabs: Cleavage of single-stranded DNA. New England Biolabs 1988/99 Catalogue. Page 262.
219. Ziegelin, G, Lanka, E.: Bacteriophage P4 DNA replication. FEMS Microbiol. Rev. 17: 99-107 (1995).
220. Salas, M.: Protein-priming of DNA replication. Annu. Rev. Biochem. 60:39-71 (1991).
221. Gene Targeting Protocols. Kmiec,EB ed. [133]. 2000. Totowa, NJ., Humana Press. Methods in Molecular Biology.
222. Smith,AE: Viral vectors in gene therapy. Annu.Rev Microbiol 49: 807-838 (1995).
223. Scott,JR, Churchward,GG: Conjugative transposition. Annu.Rev Microbiol 49: 367-397 (1995).
224. Mahillon,J, Chandler,M: Insertion sequences. Microbiol Mol Biol Rev 62: 725-774 (1998).
225. Tavakoli,N, Comanducci,A, Dodd,HM, Lett,MC, Albiger,B, Bennett,P: IS1294, a DNA element that transposes by RC transposition. Plasmid 44: 66-84 (2000).
226. Furukawa,K, Hayashida,S, Taira,K: Gene-specific transposon mutagenesis of the biphenyl/polychlorinated biphenyl-degradation-controlling bph operon in soil bacteria. Gene 98: 21-28 (1991).
227. Norgren,M, Caparon,MG, Scott,JR: A method for allelic replacement that uses the conjugative transposon Tn916: deletion of the emm6.1 allele in Streptococcus pyogenes JRS4. Infect.Immun. 57: 3846-3850 (1989).
228. Biswas,I, Gruss,A, Ehrlich,SD, Maguin,E: High-efficiency gene inactivation and replacement system for gram-positive bacteria. J Bacteriol 175: 3628-3635 (1993).
229. Alonso,JC, Ayora,S, Canosa,I, Weise,F, Rojo,F: Site-specific recombination in gram-positive theta-replicating plasmids. FEMS Microbiol Lett 142: 1-10 (1996).
230. Morel-Deville,F, Ehrlich,SD: Theta-type DNA replication stimulates homologous recombination in the Bacillus subtilis chromosome. Mol Microbiol 19: 587-598 (1996).
231.Heslip,TR, Hodgetts,RB: Targeted transposition at the vestigial locus of Drosophila melanogaster. Genetics 138: 1127-1135 (1994).
232. Current Protocols in Molecular Biology. Ausubel,FM, Brent,R, Kingston,RE, Moore,DD, Seidman,JG, Smith,JA, Struhl,K eds. 1987. John Wiley and Sons, Inc.
233. Arezi,B, Kuchta,RD: Eukaryotic DNA primase. Trends Biochem Sci 25: 572-576 (2000).
234. Boulikas,T: Common structural features of replication origins in all life forms. J Cell Biochem 60: 297-316 (1996).
235. Masai,H, Arai,K: Mechanisms of primer RNA synthesis and D-loop/R-loop-dependent DNA replication in Escherichia coli. Biochimie 78: 1109-1117 (1996).
236. Sandler,SJ, Marians,KJ: Role of PriA in replication fork reactivation in Escherichia coli. J Bacteriol 182: 9-13 (2000).
237. Haren,L, Ton-Hoang,B, Chandler,M: Integrating DNA: transposases and retroviral integrases. Annu.Rev Microbiol 53: 245-281 (1999).
238. Carles-Kinch,K, Kreuzer,KN: RNA-DNA hybrid formation at a bacteriophage T4 replication origin. J Mol Biol 266: 915-926 (1997).
239. Castellano,MM, Sanz-Burgos,AP, Gutierrez,C: Initiation of DNA replication in a eukaryotic rolling-circle replicon: identification of multiple DNA-protein complexes at the geminivirus origin. J Mol Biol 290: 639-652 (1999).
240. Concepts in Eukaryotic DNA Replication. Cold Spring Harbor Press, Cold Spring Harbor, NY (1999).
241. Biological Responses to DNA Damage. Cold Spring Harbor Press, Cold Spring Harbor, NY (2000).
242. Scully,R, Puget,N, Vlasakova,K: DNA polymerase stalling, sister chromatid recombination and the BRCA genes. Oncogene 19: 6176-6183 (2000).
243. Michel,B: Replication fork arrest and DNA recombination. Trends Biochem Sci 25: 173-178 (2000).
244. Haber,JE: DNA recombination: the replication connection. Trends Biochem.Sci. 24: 271-275 (1999).
245. Huntley,R, Healy,S, Freeman,D, Lavender,P, de Jager,S, Greenwood,J, Makker,J, Walker,E, Jackman,M, Xie,Q, Bannister,AJ, Kouzarides,T, Gutierrez,C, Doonan,JH, Murray,JA: The maize retinoblastoma protein homologue ZmRb-1 is regulated during leaf development and displays conserved interactions with G1/S regulators and plant cyclin D (CycD) proteins. Plant Mol Biol 37: 155-169 (1998).
246. Ludlow,JW: Interactions between SV40 large-tumor antigen and the growth suppressor proteins pRB and p53. FASEB J 7: 866-871 (1993).
247. Moran,E: Mammalian cell growth controls reflected through protein interactions with the adenovirus E1A gene products. Semin. Virol. 5: 327-340 (1994).
248. Vousden,K: Interactions of human papillomavirus transforming proteins with the products of tumor suppressor genes. FASEB J 7: 872-879 (1993).
249. Horvath,GV, Pettko-Szandtner,A, Nikovics,K, Bilgin,M, Boulton,M, Davies,JW, Gutierrez,C, Dudits,D: Prediction of functional regions of the maize streak virus replication-associated proteins by protein-protein interaction analysis. Plant Mol Biol 38: 699-712 (1998).
250. Liu,L, Saunders,K, Thomas,CL, Davies,JW, Stanley,J: Bean yellow dwarf virus RepA, but not rep, binds to maize retinoblastoma protein, and the virus tolerates mutations in the consensus binding motif. Virology 256: 270-279 (1999).
251. Ach,RA, Durfee,T, Miller,AB, Taranto,P, Hanley-Bowdoin,L, Zambryski,PC, Gruissem,W: RRB1 and RRB2 encode maize retinoblastoma-related proteins that interact with a plant D-type cyclin and geminivirus replication protein. Mol Cell Biol 17: 5077-5086 (1997).
252. Kowalski,D, Eddy,MJ: The DNA unwinding element: a novel, cis-acting component that facilitates opening of the Escherichia coli replication origin. EMBO J 8: 4335-4344 (1989).
253. Natale,DA, Schubert,AE, Kowalski,D: DNA helical stability accounts for mutational defects in a yeast replication origin. Proc Natl Acad Sci U S A 89: 2654-2658 (1992).
254. Lin,S, Kowalski,D: DNA helical instability facilitates initiation at the SV40 replication origin. J Mol Biol 235: 496-507 (1994).
255. Natale,DA, Umek,RM, Kowalski,D: Ease of DNA unwinding is a conserved property of yeast replication origins. Nucleic Acids Res 21: 555-560 (1993).
256. Current Protocols in Molecular Biology. Ausubel,FM, Brent,R, Kingston,RE, Moore,DD, Seidman,JG, Smith,JA, Struhl,K eds. 1987-2000. John Wiley and Sons, Inc.
257. Kalderon,D, Roberts,BL, Richardson,WD, Smith,AE: A short amino acid sequence able to specify nuclear location. Cell 39: 499-509 (1984).
258. Tinland,B, Koukolikova-Nicola,Z, Hall,MN, Hohn,B: The T-DNA-linked VirD2 protein contains two distinct functional nuclear localization signals. Proc Natl Acad Sci U S A 89: 7442-7446 (1992).
259. Relic,B, Andjelkovic,M, Rossi,L, Nagamine,Y, Hohn,B: Interaction of the DNA modifying proteins VirD1 and VirD2 of Agrobacterium tumefaciens: analysis by subcellular localization in mammalian cells. Proc Natl Acad Sci U S A 95: 9105-9110 (1998).
260. Hopp,T, Prickett,S, Price,V, Libby,R, March,C, Cerretti,D, Urdal,D, Conlon,P. A short polypeptide marker sequence useful for recombinant protein identification and purification. Bio/technology 6: 1204-1210. (1988).
261. Russell,D, Bennet,G. Construction and analysis of in vivo activity of E. coli promoter hybrids and promoter mutants that alter the -35 to -10 spacing. Gene 20:. 231-243. (1982).
262. Kleiner,D, Paul,W, Merrick,MJ: Construction of multicopy expression vectors for regulated over-production of proteins in Klebsiella pneumoniae and other enteric bacteria. J Gen Microbiol 134 : 1779-1784 (1988).
263. Chang,AC, Cohen,SN: Construction and characterization of amplifiable multicopy DNA cloning vehicles derived from the P15A cryptic miniplasmid. J Bacteriol 134:1141-1156 (1978).
264. Brosius,J, Holy,A: Regulation of ribosomal RNA promoters with a synthetic lac operator. Proc Natl Acad Sci U S A 81: 6929-6933 (1984).
265. Gari,E, Piedrafita,L, Aldea,M, Herrero,E: A set of vectors with a tetracycline-regulatable promoter system for modulated gene expression in Saccharomyces cerevisiae. Yeast 13: 837-848 (1997).
266. Schneider,JC, Guarente,L: Vectors for expression of cloned genes in yeast: regulation, overproduction, and underproduction. Methods Enzymol. 194: 373-388 (1991).
267. Voth,WP, Richards,JD, Shaw,JM, Stillman,DJ: Yeast vectors for integration at the HO locus. Nucleic Acids Res 29: E59-E59 (2001).
268. Gietz,RD, Sugino,A: New yeast-Escherichia coli shuttle vectors constructed with in vitro mutagenized yeast genes lacking six-base pair restriction sites. Gene 74: 527-534 (1988).
269. Mead,DA, Szczesna-Skorupa,E, Kemper,B: Single-stranded DNA 'blue' T7 promoter plasmids: a versatile tandem promoter system for cloning and protein engineering. Protein Eng 1: 67-74 (1986).
270. Dente,L, Cortese,R: pEMBL: a new family of single-stranded plasmids for sequencing DNA. Methods Enzymol. 155: 111-119 (1987).
271. Hanai,R, Wang,JC: The mechanism of sequence-specific DNA cleavage and strand transfer by phi X174 gene A* protein. J Biol Chem 268: 23830-23836 (1993).
272. Colasanti,J, Denhardt,DT: Expression of the cloned bacteriophage phi X174 A* gene in Escherichia coli inhibits DNA replication and cell division. J Virol 53: 807-813 (1985).
273. Yoshimatsu,T, Nagawa,F: Control of gene expression by artificial introns in Saccharomyces cerevisiae. Science 244: 1346-1348 (1989).
274. Yanisch-Perron,C, Vieira,J, Messing,J: Improved M13 phage cloning vectors and host strains: nucleotide sequences of the M13mp18 and pUC19 vectors. Gene 33: 103-119 (1985).
275. van der,EA, Teertstra,R, Weisbeek,PJ: Initiation and termination of the bacteriophage phi X174 rolling circle DNA replication in vivo: packaging of plasmid single-stranded DNA into bacteriophage phi X174 coats. Nucleic Acids Res 10: 6849-6863 (1982).
276. Woolston,CJ, Barker,R, Gunn,H, Boulton,MI, Mullineaux,PM. Agroinfection and nucleotide sequence of cloned wheat dwarf virus DNA. Plant Mol.Biol. 11: 35-43. 1988.
277. Schalk,HJ, Matzeit,V, Schiller,B, Schell,J, Gronenborn,B: Wheat dwarf virus, a geminivirus of graminaceous plants needs splicing for replication. EMBO J 8: 359-364 (1989).
278. Arai,N, Kornberg,A: Rep protein as a helicase in an active, isolatable replication fork of duplex phi X174 DNA. J Biol Chem 256: 5294-5298 (1981).
279. Bialkowska-Hobrzanska,H, Denhardt,DT: The rep mutation. VII. Cloning and analysis of the functional rep gene of Escherichia coli K-12. Gene 28: 93-102 (1984).
280. Messing,J, Crea,R, Seeburg,PH: A system for shotgun DNA sequencing. Nucleic Acids Res 9: 309-321 (1981).
281. Lee,EC, Yu,D, Mactinez,d, V, Tessarollo,L, Swing,DA, Court,DL, Jenkins,NA, Copeland,NG: A highly efficient Escherichia coli-based chromosome engineering system adapted for recombinogenic targeting and subcloning of BAC DNA. Genomics 73: 56-65 (2001).
282. Datsenko,KA, Wanner,BL: One-step inactivation of chromosomal genes in Escherichia coli K-12 using PCR products. Proc Natl Acad Sci U S A 97: 6640-6645 (2000).
283. Kowalczykowski,SC, Dixon,DA, Eggleston,AK, Lauder,SD, Rehrauer,WM: Biochemistry of homologous recombination in Escherichia coli. Microbiol.Rev. 58: 401-465 (1994).
284. Paques,F, Haber,JE: Multiple pathways of recombination induced by double-strand breaks in Saccharomyces cerevisiae. Microbiol.Mol.Biol.Rev. 63: 349-404 (1999).
285. Habu,T, Taki,T, West,A, Nishimune,Y, Morita,T: The mouse and human homologs of DMC1, the yeast meiosis-specific homologous recombination gene, have a common unique form of exon-skipped transcript in meiosis. Nucleic Acids Res. 24: 470-477 (1996).
286. Doutriaux,MP, Couteau,F, Bergounioux,C, White,C: Isolation and characterisation of the RAD51 and DMC1 homologs from Arabidopsis thaliana. Mol.Gen.Genet. 257: 283-291 (1998).
287. Shinohara.,A, Ogawa,H, Matsuda,Y, Ushio,N, Ikeo,K, Ogawa,T: Cloning of human, mouse and fission yeast recombination genes homologous to RAD51 and recA [published erratum appears in Nat Genet 1993 Nov;5(3):312]. Nat.Genet. 4: 239-243 (1993).
288. Muris,DF, Bezzubova,O, Buerstedde,JM, Vreeken,K, Balajee,AS, Osgood,CJ, Troelstra,C, Hoeijmakers,JH, Ostermann,K, Schmidt,H: Cloning of human and mouse genes homologous to RAD52, a yeast gene involved in DNA repair and recombination. Mutat.Res. 315: 295-305 (1994).
289. Milne,GT, Weaver,DT: Dominant negative alleles of RAD52 reveal a DNA repair/recombination complex including Rad51 and Rad52. Genes Dev. 7: 1755-1765 (1993).
290. Muyrers,JP, Zhang,Y, Buchholz,F, Stewart,AF: RecE/RecT and Redalpha/Redbeta initiate double-stranded break repair by specifically interacting with their respective partners. Genes Dev. 14: 1971-1982 (2000).
291. Link,AJ, Olson,MV: Physical map of the Saccharomyces cerevisiae genome at 110-kilobase resolution. Genetics 127: 681-698 (1991).
292. Atanassova,R, Chaubet,N, Gigot,C: A 126 bp fragment of a plant histone gene promoter confers preferential expression in meristems of transgenic Arabidopsis. Plant J 2: 291-300 (1992).
293. Soni,R, Carmichael,JP, Shah,ZH; Murray,JA: A family of cyclin D homologs from plants differentially controlled by growth regulators and containing the conserved retinoblastoma protein interaction motif. Plant Cell 7: 85-103 (1995).
294. Riou-Khamlichi,C, Menges,M, Healy,JM, Murray,JA: Sugar control of the plant cell cycle: differential regulation of Arabidopsis D-type cyclin gene expression. Mol Cell Biol 20: 4513-4521 (2000).
295. von Arnim,AG, Deng,XW, Stacey,MG: Cloning vectors for the expression of green fluorescent protein fusion proteins in transgenic plants. Gene 221: 35-43 (1998).
296. Xiang,C, Han,P, Lutziger,I, Wang,K, Oliver,DJ: A mini binary vector series for plant transformation. Plant Mol Biol 40: 711-717 (1999).
297. Rozwadowski,K, Kreiser,T, Hasnadka,R, Lydiate,D. AtMRE11 : a component of meiotic recombination and DNA repair in plants. 10th International Conference on Arabidopsis Research, Melbourne, Australia, July 4-8, 1999.
298. Friedberg,EC, Walker,GC, Siede,W: DNA Repair and Mutagenesis. American Society for Microbiology, Washington, D.C. (1995).
299. Keeney,S, Giroux,CN, Kleckner,N: Meiosis-specific DNA double-strand breaks are catalyzed by Spo11, a member of a widely conserved protein family. Cell 88: 375-384 (1997).
300. Keeney,S, Baudat,F, Angeles,M, Zhou,ZH, Copeland,NG, Jenkins,NA, Manova,K, Jasin,M: A mouse homolog of the Saccharomyces cerevisiae meiotic recombination DNA transesterase Spo11p. Genomics 61: 170-182 (1999).
301. Hartung,F, Puchta,H: Molecular characterisation of two paralogous SPO11 homologues in Arabidopsis thaliana. Nucleic Acids Res. 28: 1548-1554 (2000).
302. Wu,K, Malik,K, Tian,L, Hu,M, Martin,T, Foster,E, Brown,D, Miki,B: Enhancers and core promoter elements are essential for the activity of a cryptic gene activation sequence from tobacco, tCUP. Mol Genet Genomics 265: 763-770 (2001).
303. Odell,JT, Nagy,F, Chua,NH: Identification of DNA sequences required for activity of the cauliflower mosaic virus 35S promoter. Nature 313: 810-812 (1985).
304. Bevan,MW, Flavell,RB, Chilton,MD: A chimaeric antibiotic resistance gene as a selectable marker for plant cell transformation. 1983. Biotechnology 24: 367-370 (1992).
305. Callis,J, Raasch,JA, Vierstra,RD: Ubiquitin extension proteins of Arabidopsis thaliana. Structure, localization, and expression of their promoters in transgenic tobacco. J Biol Chem 265: 12486-12493 (1990).
306. Mandel,T, Fleming,AJ, Krahenbuhl,R, Kuhlemeier,C: Definition of constitutive gene expression in plants: the translation initiation factor 4A gene as a model. Plant Mol Biol 29: 995-1004 (1995).
307. Zhang,W, McElroy,D, Wu,R: Analysis of rice Act1 5'region activity in transgenic rice plants. Plant Cell 3:1155-1165 (1991).
308. Jacobs,M, Dolferus,R, Van den,BD: Isolation and biochemical analysis of ethyl methanesulfonate-induced alcohol dehydrogenase null mutants of arabidopsis thaliana (L.) Heynh. Biochem Genet 26: 105-122 (1988).
309. Koncz,C, Schell,J. The promoter of TL-DNA gene 5 controls the tissue-specific expression of chimaeric genes carried by a novel type of Agrobacterium binary vector. Mol.Gen.Genet. 204, pp. 383-396. 1986.
310. Bechtold,N, Pelletier,G: In planta Agrobacterium-mediated transformation of adult Arabidopsis thaliana plants by vacuum infiltration. Methods Mol Biol 82: 259-266 (1998).
311. Sathasivan,K, Haughn,GW, Murai,N: Nucleotide sequence of a mutant acetolactate synthase gene from an imidazolinone-resistant Arabidopsis thaliana var. Columbia. Nucleic Acids Res 18: 2188 (1990).
312. Castellano,MM, Sanz-Burgos,AP, Gutierrez,C: Initiation of DNA replication in a eukaryotic rolling-circle replicon: identification of multiple DNA-protein complexes at the geminivirus origin. J Mol Biol 290: 639-652 (1999).
313. Leanna,CA, Hannink,M: The reverse two-hybrid system: a genetic scheme for selection against specific protein/protein interactions. Nucleic Acids Res 24: 3341-3347 (1996).
314. Concepts in Eukaryotic DNA Replication. Cold Spring Harbor Press, Cold Spring Harbor, NY (1999).
315. Biological Responses to DNA Damage. Cold Spring Harbor Press, Cold Spring Harbor, NY (2000).
316. Scully,R, Puget,N, Vlasakova,K: DNA polymerase stalling, sister chromatid recombination and the BRCA genes. Oncogene 19: 6176-6183 (2000).
317. Michel,B: Replication fork arrest and DNA recombination. Trends Biochem Sci 25: 173-178 (2000).
318. Haber,JE: DNA recombination: the replication connection. Trends Biochem.Sci. 24: 271-275 (1999).
319. Bell,JB, Jones,ME: Purification and characterization of yeast orotidine 5'-monophosphate decarboxylase overexpressed from plasmid PGU2. J Biol Chem 266: 12662-12667 (1991).
320. Harris,P, Navarro Poulsen,JC, Jensen,KF, Larsen,S: Structural basis for the catalytic mechanism of a proficient enzyme: orotidine 5'-monophosphate decarboxylase. Biochemistry 39: 4217-4224 (2000).
321. Marsischky,GT, Filosi,N, Kane,MF, Kolodner,R: Redundancy of Saccharomyces cerevisiae MSH3 and MSH6 in MSH2-dependent mismatch repair. Genes Dev. 10: 407-420 (1996).
322. Rose,M, Winston,F: Identification of a Ty insertion within the coding sequence of the S. cerevisiae URA3 gene. 193: 557-560 (1984).
323. Gietz,RD, Schiestl,RH, Willems,AR, Woods,RA: Studies on the transformation of intact yeast cells by the LiAc/SS-DNA/PEG procedure. Yeast 11: 355-360 (1995).
324. Adams,A, Gottschling,DE, Kaiser,CA, Steams,T: Methods in Yeast Genetics. Cold Spring Harbor Laboratory Press, (1997).
325. Kobayashi,T, Hotta,Y, Tabata,S: Isolation and characterization of a yeast gene that is homologous with a meiosis-specific cDNA from a plant. Mol.Gen.Genet. 237: 225-232 (1993).
326. Devore,JL: Probability and Statistics. Duxbury Press, (1995).
327. Lea,D, Coulson,C. The distribution of the numbers of mutants in bacterial populations. J.Genet. 49: 264-285. 1948.
328. Dixon,W, Massey F.: Introduction to statistical analysis. McGraw-Hill, Inc., New York (1969).

Numeric ranges are inclusive of the numbers defining the range. Polynucleotides encoding desired proteins may be modified to optimize codon usage or enhance stability of expressed products, for example to adapt sequences for expression in alternative cell types or organisms. In the specification, the word "comprising" is used as an openended term, substantially equivalent to the phrase "including, but not limited to", and the word "comprises" has a corresponding meaning. Citation of references herein shall not be construed as an admission that such references are prior art to the present invention. The invention includes all embodiments and variations substantially as hereinbefore described and with reference to the examples.

## Claims

1. A method for modifying a target sequence in a genome of a host comprising:
introducing and integrating into the genome of the host or a progenitor of the host a gene targeting cassette comprised of recombinant nucleic acid sequences, wherein the gene targeting cassette comprises:
a) a replication initiator sequence recognized in the host by at least one replication factor comprised in the host to mediate DNA replication in the host initiated at the replication initiator sequence, wherein the at least one replication factor includes a nickase activity, and wherein the replication initiator sequence is a DNA sequence encoding a rolling circle replication protein recognition and nicking site where DNA replication is initiated;
b) a reproducible sequence operably linked to the replication initiator sequence so that DNA replication initiated at the replication initiator sequence replicates the reproducible sequence, to release a copy of the reproducible sequence, wherein the reproducible sequence is homologous to the target sequence in the genome of the host;
whereby DNA replication initiated at the replication initiator sequence results in the regeneration of the gene targeting cassette for subsequent rounds of DNA replication to produce multiple copies of the reproducible sequence; and whereby at least a portion of one of the copies of the reproducible sequence mediates a heritable genetic change in the sequence of the homologous target sequence;
with the proviso that the method is not for modifying the germ line genetic identity of human beings; and
with the proviso that the method is not for treatment of the human or animal body by surgery or therapy.

2. An in vitro method for modifying a target sequence in a genome of a host comprising:
introducing and integrating into the genome of the host or a progenitor of the host a gene targeting cassette comprised of recombinant nucleic acid sequences, wherein the gene targeting cassette comprises:
a) a replication initiator sequence recognized in the host by at least one replication factor comprised in the host to mediate DNA replication in the host initiated at the replication initiator sequence, wherein the at least one replication factor includes a nickase activity, and wherein the replication initiator sequence is a DNA sequence encoding a rolling circle replication protein recognition and nicking site where DNA replication is initiated;
b) a reproducible sequence operably linked to the replication initiator sequence so that DNA replication initiated at the replication initiator sequence replicates the reproducible sequence, to release a copy of the reproducible sequence, wherein the reproducible sequence is homologous to the target sequence in the genome of the host;
whereby DNA replication initiated at the replication initiator sequence results in the regeneration of the gene targeting cassette for subsequent rounds of DNA replication to produce multiple copies of the reproducible sequence; and whereby at least a portion of one of the copies of the reproducible sequence mediates a heritable genetic change in the sequence of the homologous target sequence.

3. A method for gene targeting for modifying a target sequence in a genome of a eukaryotic host comprising:
transforming the host with a gene targeting cassette comprised of recombinant nucleic acid sequences on an extrachromosomal element, wherein the gene targeting cassette comprises:
a) a replication initiator sequence recognized in the host by at least one replication factor comprised in the host to mediate DNA replication in the host initiated at the replication initiator sequence, wherein the at least one replication factor includes a nickase activity, and wherein the replication initiator sequence is a DNA sequence encoding a rolling circle replication protein recognition and nicking site where DNA replication is initiated;
b) a reproducible sequence operably linked to the replication initiator sequence so that DNA replication initiated at the replication initiator sequence replicates the reproducible sequence, to release a copy of the reproducible sequence, wherein the reproducible sequence is homologous to the target sequence in the genome of the host; and,
whereby DNA replication initiated at the replication initiator sequence results in regeneration of the gene targeting cassette for subsequent rounds of DNA replication to produce multiple copies of the reproducible sequence, wherein the replication of the reproducible sequence initiated at the replication initiator sequence replicates only a portion of the extrachromosomal element; and whereby at least a portion of one of the copies of the reproducible sequence mediates a heritable genetic change in the sequence of the homologous target sequence in the genome of the host;
with the proviso that the method is not for modifying the germ line genetic identity of human beings; and
with the proviso that the method is not for treatment of the human or animal body by surgery or therapy.

4. An in vitro method for gene targeting for modifying a target sequence in a genome of a eukaryotic host comprising:
transforming the host with a gene targeting cassette comprised of recombinant nucleic acid sequences on an extrachromosomal element, wherein the gene targeting cassette comprises:
a) a replication initiator sequence recognized in the host by at least one replication factor comprised in the host to mediate DNA replication in the host initiated at the replication initiator sequence, wherein the at least one replication factor includes a nickase activity, and wherein the replication initiator sequence is a DNA sequence encoding a rolling circle replication protein recognition and nicking site where DNA replication is initiated;
b) a reproducible sequence operably linked to the replication initiator sequence so that DNA replication initiated at the replication initiator sequence replicates the reproducible sequence, to release a copy of the reproducible sequence, wherein the reproducible sequence is homologous to the target sequence in the genome of the host; and,
whereby DNA replication initiated at the replication initiator sequence results in regeneration of the gene targeting cassette for subsequent rounds of DNA replication to produce multiple copies of the reproducible sequence, wherein the replication of the reproducible sequence initiated at the replication initiator sequence replicates only a portion of the extrachromosomal element; and whereby at least a portion of one of the copies of the reproducible sequence mediates a heritable genetic change in the sequence of the homologous target sequence in the genome of the host.

5. The method of any of claims 1 to 4, wherein the portion of one of the copies of the reproducible sequence has at least 90% sequence identity to a portion of the target sequence, when optimally aligned.

6. The method of any of claims 1 to 5, wherein the gene targeting cassette further comprises a replication terminator sequence operably linked to the reproducible sequence to terminate DNA replication initiated at the replication initiator sequence, wherein DNA replication initiated at the replication initiator sequence is terminated at the replication terminator sequence.

7. The method of any of claims 3 to 5, wherein the reproducible sequence is operably linked to a replication terminator sequence to terminate DNA replication initiated at the replication initiator sequence, to release the copy of the reproducible sequence; and wherein the replication of the reproducible sequence initiated at the replication initiator sequence and terminated at the replication terminator sequence replicates only a portion of the extrachromosomal element.

8. The method of claim 5, wherein the portion of one of the copies of the reproducible sequence differs from the portion of the target sequence by having at least one nucleic acid deletion, substitution or addition.

9. The method of claim 8, wherein the portion of one of the copies of the reproducible sequence is at least 15 nucleotides in length.

10. The method of any one of claims 1 to 4, wherein the host, or a lineal relative of the host, is transformed with a nucleotide sequence encoding the replication factor.

11. The method of claim 10, wherein the nucleotide sequence encoding the replication factor is expressed under the control of a promoter selected from the group consisting of cell-cycle-specific promoters, G1 phase specific promoters, S phase specific promoters, G1/S boundary promoters, G2 phase specific promoters, S/G2 boundary promoters, tissue specific promoters, developmental stage specific promoters, environmental stimuli responsive promoters, constitutive promoters, bipartite promoters, or promoters regulatable by induction or repression.

12. The method of any one of claims 1 to 4, wherein the host is eukaryotic and a replication factor comprises a nuclear localization sequence.

13. The method of any one of claims 1 to 4, wherein the host is a plant cell or a plant.

14. The method of any one of claims 1 to 4, wherein the host cell is an animal cell or an animal.

15. The method of claim 1 or 2 further comprising the step of excising the gene targeting cassette from the genome by site specific recombination.

16. The method of claim 1 or 2 further comprising the step of removing the gene targeting cassette from the genome.

17. The method of claim 16, wherein the gene targeting cassette is removed from the genome by genetic segregation and host identification after meiosis.

18. The method of claim 3 or 4, further comprising the step of removing the gene targeting cassette from the host.

19. The method of claim 1 or 2, wherein the host is a cell, and the cell cycle of the cell is modulated by a cell cycle regulator so that the multiple copies of the gene targeting substrate are present in the cell at a particular cell cycle phase of the cell.

20. The method of claim 19, wherein the particular cell cycle phase is S phase.

21. The method of claim 19, wherein the cell cycle regulator is selected from the group consisting of pocket family of proteins, retinoblastoma tumour suppressor proteins, E2F transcription factors, cyclins and cyclin dependent kinases.

22. The method of claim 3 or 4, further comprising selecting for the heritable genetic change in the homologous target sequence in the genome of the host.

## Patentansprüche

1. Verfahren zum Modifizieren einer Zielsequenz in einem Genom eines Wirts, umfassend:
Einführen und Integrieren einer gezielten Gen-Modifikationskassette, die aus rekombinanten Nukleinsäuresequenzen besteht, in das Genom des Wirts oder eines Vorläufers des Wirts, wobei die gezielte Gen-Modifikationskassette umfasst:
a) eine Replikationseinleitungsssequenz, die im Wirt von mindestens einem Replikationsfaktor erkannt wird, der im Wirt enthalten ist, um eine DNA-Replikation im Wirt zu vermitteln, die an der Replikationseinleitungssequenz eingeleitet wird, wobei der mindestens eine Replikationsfaktor eine Nickase-Aktivität enthält und wobei die Replikationseinleitungssequenz eine DNA-Sequenz ist, die für eine Rolling-Circle-Replikations-Proteinerkennungs- und Nicking-Site codiert, wo eine DNA-Replikation eingeleitet wird;
b) eine reproduzierbare Sequenz, die funktionsfähig an die Replikationseinleitungssequenz gebunden ist, so dass eine DNA-Replikation, die an der Replikationseinleitungssequenz eingeleitet wird, die reproduzierbare Sequenz repliziert, um eine Kopie der reproduzierbaren Sequenz freizusetzen, wobei die reproduzierbare Sequenz zur Zielsequenz im Genom des Wirts homolog ist;
wobei eine DNA-Replikation, die an der Replikationseinleitungssequenz eingeleitet wird, zur Regeneration der gezielten Gen-Modifikationskassette für anschließende Runden einer DNA-Replikation führt, um mehrere Kopien der reproduzierbaren Sequenz zu erzeugen; und wobei mindestens ein Abschnitt einer der Kopien der reproduzierbaren Sequenz eine vererbbare genetische Veränderung in der Sequenz der homologen Zielsequenz vermittelt;
unter der Voraussetzung, dass das Verfahren nicht zur Modifizierung der genetischen Keimlinienidentität von Menschen dient; und
unter der Voraussetzung, dass das Verfahren nicht zur Behandlung des menschlichen oder tierischen Körpers durch Chirurgie oder Therapie dient.

2. In-vitro-Verfahren zum Modifizieren einer Zielsequenz in einem Genom eines Wirts, umfassend:
Einführen und Integrieren einer gezielten Gen-Modifikationskassette, die aus rekombinanten Nukleinsäuresequenzen besteht, in das Genom des Wirts oder eines Vorläufers des Wirts, wobei die gezielte Gen-Modifikationskassette umfasst:
a) eine Replikationseinleitungsssequenz, die im Wirt von mindestens einem Replikationsfaktor erkannt wird, der im Wirt enthalten ist, um eine DNA-Replikation im Wirt zu vermitteln, die an der Replikationseinleitungssequenz eingeleitet wird, wobei der mindestens eine Replikationsfaktor eine Nickase-Aktivität enthält und wobei die Replikationseinleitungssequenz eine DNA-Sequenz ist, die für eine Rolling-Circle-Replikations-Proteinerkennungs- und Nicking-Site codiert, wo eine DNA-Replikation eingeleitet wird;
b) eine reproduzierbare Sequenz, die funktionsfähig an die Replikationseinleitungssequenz gebunden ist, so dass eine DNA-Replikation, die an der Replikationseinleitungssequenz eingeleitet wird, die reproduzierbare Sequenz repliziert, um eine Kopie der reproduzierbaren Sequenz freizusetzen, wobei die reproduzierbare Sequenz zur Zielsequenz im Genom des Wirts homolog ist;
wobei eine DNA-Replikation, die an der Replikationseinleitungssequenz eingeleitet wird, zur Regeneration der gezielten Gen-Modifikationskassette für anschließende Runden einer DNA-Replikation führt, um mehrere Kopien der reproduzierbaren Sequenz zu erzeugen; und wobei mindestens ein Abschnitt einer der Kopien der reproduzierbaren Sequenz eine vererbbare genetische Veränderung in der Sequenz der homologen Zielsequenz vermittelt.

3. Verfahren für eine gezielte Gen-Modifizierung zum Modifizieren einer Zielsequenz in einem Genom eines eukaryotischen Wirts, umfassend:
transformieren des Wirts mit einer gezielten Gen-Modifikationskassette, die aus rekombinanten Nukleinsäuresequenzen auf einem extrachromosomalen Element besteht, wobei die gezielte Gen-Modifikationskassette umfasst:
a) eine Replikationseinleitungsssequenz, die im Wirt von mindestens einem Replikationsfaktor erkannt wird, der im Wirt enthalten ist, um eine DNA-Replikation im Wirt zu vermitteln, die an der Replikationseinleitungssequenz eingeleitet wird, wobei der mindestens eine Replikationsfaktor eine Nickase-Aktivität enthält und wobei die Replikationseinleitungssequenz eine DNA-Sequenz ist, die für eine Rolling-Circle-Replikations-Proteinerkennungs- und Nicking-Site codiert, wo eine DNA-Replikation eingeleitet wird;
b) eine reproduzierbare Sequenz, die funktionsfähig an die Replikationseinleitungssequenz gebunden ist, so dass eine DNA-Replikation, die an der Replikationseinleitungssequenz eingeleitet wird, die reproduzierbare Sequenz repliziert, um eine Kopie der reproduzierbaren Sequenz freizusetzen, wobei die reproduzierbare Sequenz zur Zielsequenz im Genom des Wirts homolog ist; und
wobei eine DNA-Replikation, die an der Replikationseinleitungssequenz eingeleitet wird, zur Regeneration der gezielten Gen-Modifikationskassette für anschließende Runden einer DNA-Replikation führt,
um mehrere Kopien der reproduzierbaren Sequenz zu erzeugen, wobei die Replikation der reproduzierbaren Sequenz, die an der Replikationseinleitungssequenz eingeleitet wird, nur einen Abschnitt des extrachromosomalen Elements repliziert; und wobei mindestens ein Abschnitt einer der Kopien der reproduzierbaren Sequenz eine vererbbare genetische Veränderung in der Sequenz der homologen Zielsequenz im Genom des Wirts vermittelt;
unter der Voraussetzung, dass das Verfahren nicht zur Modifizierung der genetischen Keimlinienidentität von Menschen dient; und
unter der Voraussetzung, dass das Verfahren nicht zur Behandlung des menschlichen oder tierischen Körpers durch Chirurgie oder Therapie dient.

4. In-vitro-Verfahren für eine gezielte Gen-Modifizierung zum Modifizieren einer Zielsequenz in einem Genom eines eukaryotischen Wirts, umfassend:
transformieren des Wirts mit einer gezielten Gen-Modifikationskassette, die aus rekombinanten Nukleinsäuresequenzen auf einem extrachromosomalen Element besteht, wobei die gezielte Gen-Modifikationskassette umfasst:
a) eine Replikationseinleitungsssequenz, die im Wirt von mindestens einem Replikationsfaktor erkannt wird, der im Wirt enthalten ist, um eine DNA-Replikation im Wirt zu vermitteln, die an der Replikationseinleitungssequenz eingeleitet wird, wobei der mindestens eine Replikationsfaktor eine Nickase-Aktivität enthält und wobei die Replikationseinleitungssequenz eine DNA-Sequenz ist, die für eine Rolling-Circle-Replikations-Proteinerkennungs- und Nicking-Site codiert, wo eine DNA-Replikation eingeleitet wird;
b) eine reproduzierbare Sequenz, die funktionsfähig an die Replikationseinleitungssequenz gebunden ist, so dass eine DNA-Replikation, die an der Replikationseinleitungssequenz eingeleitet wird, die reproduzierbare Sequenz repliziert, um eine Kopie der reproduzierbaren Sequenz freizusetzen, wobei die reproduzierbare Sequenz zur Zielsequenz im Genom des Wirts homolog ist; und
wobei eine DNA-Replikation, die an der Replikationseinleitungssequenz eingeleitet wird, zur Regeneration der gezielten Gen-Modifikationskassette für anschließende Runden einer DNA-Replikation führt,
um mehrere Kopien der reproduzierbaren Sequenz zu erzeugen, wobei die Replikation der reproduzierbaren Sequenz, die an der Replikationseinleitungssequenz eingeleitet wird, nur einen Abschnitt des extrachromosomalen Elements repliziert; und wobei mindestens ein Abschnitt einer der Kopien der reproduzierbaren Sequenz eine vererbbare genetische Veränderung in der Sequenz der homologen Zielsequenz im Genom des Wirts vermittelt.

5. Verfahren nach einem der Ansprüche 1 bis 4, wobei der Abschnitt einer der Kopien der reproduzierbaren Sequenz mindestens 90% Sequenzidentität mit einem Abschnitt der Zielsequenz bei optimaler Ausrichtung hat.

6. Verfahren nach einem der Ansprüche 1 bis 5, wobei die gezielte Gen-Modifikationskassette des Weiteren eine Replikationsbeendigungssequenz umfasst, die funktionsfähig an die reproduzierbare Sequenz gebunden ist, um eine DNA-Replikation zu beenden, die an der Replikationseinleitungssequenz eingeleitet wird, wobei die DNA-Replikation, die an der Replikationseinleitungssequenz eingeleitet wird, an der Replikationsbeendigungssequenz beendet wird.

7. Verfahren nach einem der Ansprüche 3 bis 5, wobei die reproduzierbare Sequenz funktionsfähig an eine Replikationsbeendigungssequenz gebunden ist, um eine DNA-Replikation zu beenden, die an der Replikationseinleitungssequenz eingeleitet wird, um die Kopie der reproduzierbaren Sequenz freizusetzen; und wobei die Replikation der reproduzierbaren Sequenz, die an der Replikationseinleitungssequenz eingeleitet und an der Replikationsbeendigungssequenz beendet wird, nur einen Abschnitt des extrachromosomalen Elements repliziert.

8. Verfahren nach Anspruch 5, wobei der Abschnitt einer der Kopien der reproduzierbaren Sequenz sich vom Abschnitt der Zielsequenz unterscheidet, indem er mindestens eine Nukleinsäuredelektion, -substitution oder -addition aufweist.

9. Verfahren nach Anspruch 8, wobei der Abschnitt einer der Kopien der reproduzierbaren Sequenz mindestens 15 Nukleotide lang ist.

10. Verfahren nach einem der Ansprüche 1 bis 4, wobei der Wirt oder ein linearer Verwandter des Wirts mit einer Nukleotidsequenz transformiert ist, die für den Replikationsfaktor codiert.

11. Verfahren nach Anspruch 10, wobei die Nukleotidsequenz, die für den Replikationsfaktor codiert, unter der Kontrolle eines Promotors exprimiert wird, der ausgewählt ist aus der Gruppe bestehend aus Zellzyklus-spezifischen Promotoren, G1-phasenspezifischen Promotoren, S-phasenspezifischen Promotoren, G1/S-Grenzpromotoren, G2-phasenspezifischen Promotoren, S/GS-Grenzpromotoren, gewebespezifischen Promotoren, Entwicklungsstufenspezifischen Promotoren, auf Umweltreize ansprechenden Promotoren, konstitutiven Promotoren, zweiteiligen Promotoren oder Promotoren, die durch Induktion oder Repression regulierbar sind.

12. Verfahren nach einem der Ansprüche 1 bis 4, wobei der Wirt eukaryotisch ist und ein Replikationsfaktor eine nukleare Lokalisierungssequenz umfasst.

13. Verfahren nach einem der Ansprüche 1 bis 4, wobei der Wirt eine Pflanzenzelle oder eine Pflanze ist.

14. Verfahren nach einem der Ansprüche 1 bis 4, wobei die Wirtszelle eine Tierzelle oder ein Tier ist.

15. Verfahren nach Anspruch 1 oder 2, umfassend den Schritt des Ausschneidens der gezielten Gen-Modifizierungskassette aus dem Gen durch ortsspezifische Rekombination.

16. Verfahren nach Anspruch 1 oder 2, umfassend den Schritt des Entfernens der gezielten Gen-Modifizierungskassette aus dem Genom.

17. Verfahren nach Anspruch 16, wobei die gezielte Gen-Modifizierungskassette aus dem Genom durch genetische Segregation und Wirtsidentifizierung nach Meiose entfernt wird.

18. Verfahren nach Anspruch 3 oder 4, des Weiteren umfassend den Schritt zum Entfernen der gezielten Gen-Modifizierungskassette aus dem Wirt.

19. Verfahren nach Anspruch 1 oder 2, wobei der Wirt eine Zelle ist und der Zellzyklus der Zelle durch einen Zellzyklusregulator derart moduliert ist, dass die mehrfachen Kopien des gezielten Gen-Modifizierungssubstrats in der Zelle in einer bestimmten Zellzyklusphase der Zelle vorhanden sind.

20. Verfahren nach Anspruch 19, wobei die besondere Zellzyklusphase eine S-Phase ist.

21. Verfahren nach Anspruch 19, wobei der Zellzyklusregulator ausgewählt ist aus der Gruppe bestehend aus Proteinen der Pocket-Familie, Retinoblastom-Tumorsuppressorproteinen, E2F-Transkriptionsfaktoren, Cyclinen und clycinabhängigen Kinasen.

22. Verfahren nach Anspruch 3 oder 4, des Weiteren umfassend das Selektieren auf die vererbbare genetische Veränderung in der homologen Zielsequenz im Genom des Wirts.

## Revendications

1. Procédé de modification d'une séquence cible dans un génome hôte, qui comprend :
l'introduction et l'intégration dans le génome de l'hôte ou d'un progéniteur de l'hôte d'une cassette de ciblage génique composée de séquences d'acide nucléique recombinant, dans lequel la cassette de ciblage génique comprend :
a) une séquence d'initiation de la réplication reconnue dans l'hôte par au moins un facteur de réplication compris dans l'hôte pour médier la réplication de l'ADN dans l'hôte initiée au niveau de la séquence d'initiation de la réplication, dans lequel le ou les facteurs de réplication comprennent une activité nickase, et dans lequel la séquence d'initiation de la réplication est une séquence d'ADN codant pour un site de reconnaissance et de découpage de la protéine de réplication par cercle roulant où la réplication de l'ADN est initiée ;
b) une séquence reproductible liée de manière opérationnelle à la séquence d'initiation de la réplication de manière à ce que la réplication de l'ADN initiée au niveau de la séquence d'initiation de la réplication réplique la séquence reproductible, pour libérer une copie de la séquence reproductible, dans lequel la séquence reproductible est homologue à la séquence cible dans le génome de l'hôte ;
la réplication de l'ADN initiée au niveau de la séquence d'initiation de la réplication résultant ainsi en la régénération de la cassette de ciblage génique pour des cycles subséquents de réplication de l'ADN pour produire de multiples copies de la séquence reproductible ; et au moins une partie de l'une des copies de la séquence reproductible médiant ainsi un changement génétique héréditaire dans la séquence de la séquence cible homologue ;
à condition que le procédé ne soit pas destiné à modifier l'identité génétique de la lignée germinale des êtres humains ; et
à condition que le procédé ne soit pas destiné au traitement du corps humain ou animal par chirurgie ou thérapie.

2. Procédé in vitro de modification d'une séquence cible dans le génome d'un hôte, qui comprend :
l'introduction et l'intégration dans le génome de l'hôte ou d'un progéniteur de l'hôte d'une cassette de ciblage génique composée de séquences d'acide nucléique recombinant, dans lequel la cassette de ciblage génique comprend :
a) une séquence d'initiation de la réplication reconnue dans l'hôte par au moins un facteur de réplication compris dans l'hôte pour médier la réplication de l'ADN dans l'hôte initiée au niveau de la séquence d'initiation de la réplication, dans lequel le ou les facteurs de réplication comprennent une activité nickase, et dans lequel la séquence d'initiation de la réplication est une séquence d'ADN codant pour un site de reconnaissance et de découpage de la protéine de réplication par cercle roulant où la réplication de l'ADN est initiée ;
b) une séquence reproductible liée de manière opérationnelle à la séquence d'initiation de la réplication de manière à ce que la réplication de l'ADN initiée au niveau de la séquence d'initiation de la réplication réplique la séquence reproductible, pour libérer une copie de la séquence reproductible, dans lequel la séquence reproductible est homologue à la séquence cible dans le génome de l'hôte ;
la réplication de l'ADN initiée au niveau de la séquence d'initiation de la réplication résultant ainsi en la régénération de la cassette de ciblage génique pour des cycles subséquents de réplication de l'ADN pour produire de multiples copies de la séquence reproductible ; et au moins une partie de l'une des copies de la séquence reproductible médiant ainsi un changement génétique héréditaire dans la séquence de la séquence cible homologue.

3. Procédé de ciblage génique pour la modification d'une séquence cible dans le génome d'un hôte eucaryote, qui comprend :
la transformation de l'hôte avec une cassette de ciblage génique comprenant des séquences d'acide nucléique recombinant sur un élément extrachromosomique, dans lequel la cassette de ciblage génique comprend :
a) une séquence d'initiation de la réplication reconnue dans l'hôte par au moins un facteur de réplication compris dans l'hôte pour médier la réplication de l'ADN dans l'hôte initiée au niveau de la séquence d'initiation de la réplication, dans lequel le ou les facteurs de réplication comprennent une activité nickase, et dans lequel la séquence d'initiation de la réplication est une séquence d'ADN codant pour un site de reconnaissance et de découpage de la protéine de réplication par cercle roulant où la réplication de l'ADN est initiée ;
b) une séquence reproductible liée de manière opérationnelle à la séquence d'initiation de la réplication de manière à ce que la réplication de l'ADN initiée au niveau de la séquence d'initiation de la réplication réplique la séquence reproductible, pour libérer une copie de la séquence reproductible, dans lequel la séquence reproductible est homologue à la séquence cible dans le génome de l'hôte ; et
la réplication de l'ADN initiée au niveau de la séquence d'initiation de la réplication résultant ainsi en la régénération de la cassette de ciblage génique pour des cycles subséquents de réplication de l'ADN pour produire de multiples copies de la séquence reproductible, dans lequel la réplication de la séquence reproductible initiée au niveau de la séquence d'initiation de la réplication ne réplique qu'une partie de l'élément extrachromosomique ; et au moins une partie de l'une des copies de la séquence reproductible médiant ainsi un changement génétique héréditaire dans la séquence de la séquence cible homologue dans le génome de l'hôte ;
à condition que le procédé ne soit pas destiné à modifier l'identité génétique de la lignée germinale des êtres humains ; et
à condition que le procédé ne soit pas destiné au traitement du corps humain ou animal par chirurgie ou thérapie.

4. Procédé in vitro de ciblage génique pour la modification d'une séquence cible dans le génome d'un hôte eucaryote, qui comprend :
la transformation de l'hôte avec une cassette de ciblage génique comprenant des séquences d'acide nucléique recombinant sur un élément extrachromosomique, dans lequel la cassette de ciblage génique comprend :
a) une séquence d'initiation de la réplication reconnue dans l'hôte par au moins un facteur de réplication compris dans l'hôte pour médier la réplication de l'ADN dans l'hôte initiée au niveau de la séquence d'initiation de la réplication, dans lequel le ou les facteurs de réplication comprennent une activité nickase, et dans lequel la séquence d'initiation de la réplication est une séquence d'ADN codant pour un site de reconnaissance et de découpage de la protéine de réplication par cercle roulant où la réplication de l'ADN est initiée ;
b) une séquence reproductible liée de manière opérationnelle à la séquence d'initiation de la réplication de manière à ce que la réplication de l'ADN initiée au niveau de la séquence d'initiation de la réplication réplique la séquence reproductible, pour libérer une copie de la séquence reproductible, dans lequel la séquence reproductible est homologue à la séquence cible dans le génome de l'hôte ; et
la réplication de l'ADN initiée au niveau de la séquence d'initiation de la réplication résultant ainsi en la régénération de la cassette de ciblage génique pour des cycles subséquents de réplication de l'ADN pour produire de multiples copies de la séquence reproductible, dans lequel la réplication de la séquence reproductible initiée au niveau de la séquence d'initiation de la réplication ne réplique qu'une partie de l'élément extrachromosomique ; et au moins une partie de l'une des copies de la séquence reproductible médiant ainsi un changement génétique héréditaire dans la séquence de la séquence cible homologue dans le génome de l'hôte.

5. Procédé d'une quelconque des revendications 1 à 4, dans lequel la partie de l'une des copies de la séquence reproductible présente une identité de séquence d'au moins 90 % par rapport à une partie de la séquence cible, lors d'un alignement optimal.

6. Procédé de l'une quelconque des revendications 1 à 5, dans lequel la cassette de ciblage génique comprend en outre une séquence de terminaison de la réplication liée de manière opérationnelle à la séquence reproductible pour terminer la réplication de l'ADN initiée au niveau de la séquence d'initiation de la réplication, dans lequel la réplication de l'ADN initiée au niveau de la séquence d'initiation de la réplication se termine au niveau de la séquence de terminaison de la réplication.

7. Procédé d'une quelconque des revendications 3 à 5, dans lequel la séquence reproductible est liée de manière opérationnelle à une séquence de terminaison de la réplication pour terminer la réplication de l'ADN initiée au niveau de la séquence d'initiation de la réplication, pour libérer la copie de la séquence reproductible ; et dans lequel la réplication de la séquence reproductible initiée au niveau de la séquence d'initiation de réplication et terminée au niveau de la séquence de terminaison de la réplication ne réplique qu'une partie de l'élément extrachromosomique.

8. Procédé selon la revendication 5, dans lequel la partie de l'une des copies de la séquence reproductible diffère de la partie de la séquence cible en ce qu'elle a au moins une délétion, une substitution ou une addition d'acide nucléique.

9. Procédé selon la revendication 8, dans lequel la partie de l'une des copies de la séquence reproductible est d'une longueur d'au moins 15 nucléotides.

10. Procédé d'une quelconque des revendications 1 à 4, dans lequel l'hôte, ou un parent de lignée de l'hôte, est transformé avec une séquence de nucléotides codant pour le facteur de réplication.

11. Procédé selon la revendication 10, dans lequel la séquence de nucléotides codant pour le facteur de réplication est exprimée sous le contrôle d'un promoteur choisi dans le groupe constitué par des promoteurs spécifiques du cycle cellulaire, des promoteurs spécifiques de la phase G1, des promoteurs spécifiques de la phase S, des promoteurs de la limite G1/S, des promoteurs spécifiques de la phase G2, des promoteurs de la limite S/G2, des promoteurs spécifiques du tissu, des promoteurs spécifiques des stades développementaux, des promoteurs répondant aux stimuli environnementaux, des promoteurs constitutifs, des promoteurs bipartites, ou des promoteurs pouvant être régulés par l'induction ou la répression.

12. Procédé d'une quelconque des revendications 1 à 4, dans lequel l'hôte est eucaryote et un facteur de réplication comprend une séquence de localisation nucléaire.

13. Procédé d'une quelconque des revendications 1 à 4, dans lequel l'hôte est une cellule végétale ou une plante.

14. Procédé d'une quelconque des revendications 1 à 4, dans lequel la cellule hôte est une cellule d'animal ou un animal.

15. Procédé selon la revendication 1 ou 2, comprenant en outre l'étape consistant à exciser la cassette de ciblage génique du génome par recombinaison spécifique du site.

16. Procédé selon la revendication 1 ou 2, comprenant en outre l'étape consistant à éliminer la cassette de ciblage génique du génome.

17. Procédé selon la revendication 16, dans lequel la cassette de ciblage génique est retirée du génome par ségrégation génétique et identification de l'hôte après la méiose.

18. Procédé selon la revendication 3 ou 4, comprenant en outre l'étape consistant à éliminer la cassette de ciblage génique de l'hôte.

19. Procédé selon la revendication 1 ou 2, dans lequel l'hôte est une cellule, et le cycle cellulaire de la cellule est modulé par un régulateur du cycle cellulaire de manière à ce que de multiples copies du substrat de ciblage génique soient présentes dans la cellule pendant une phase particulière du cycle cellulaire de la cellule.

20. Procédé selon la revendication 19, dans lequel la phase particulière du cycle cellulaire est la phase S.

21. Procédé selon la revendication 19, dans lequel le régulateur du cycle cellulaire est choisi dans le groupe constitué par les protéines de la famille pocket, les protéines suppresseurs de tumeur de rétinoblastome, les facteurs de transcription E2F, les cyclines et les kinases dépendantes des cyclines.

22. Procédé selon la revendication 3 ou 4, comprenant en outre le choix de la modification génétique héréditaire dans la séquence cible homologue dans le génome de l'hôte.
